# EUROPEAN PATENT APPLICATION

(11) **EP 2 163 258 A1**
(43) Date of publication of application: **17.03.2010**
(21) Application number: 08765763.1
(22) Date of filing: 19.06.2008
(51) Int. Cl.: A61K 45/00, A61K 31/7105, A61K 31/713, A61K 39/395, A61K 48/00, A61P 35/00, A61P 35/02, A61P 43/00

(54) **PREVENTIVE/REMEDY FOR CANCER**

(30) Priority: 19.06.2007 JP 2007161769
(71) Applicant: Takeda Pharmaceutical Company Limited, Osaka 541-0045 (JP)
(72) Inventor: OHTA, Yoshikazu, Tsukuba-shi Ibaraki 300-4293 (JP); HAYASHI, Akira, Tsukuba-shi Ibaraki 300-4293 (JP)
(74) Representative: Dossmann, Gérard
(86) International application number: PCT/JP2008/061268
(87) International publication number: WO 2008/156153

(57) **Abstract**

The present invention provides an agent for preventing or treating a trastuzumab-resistant cancer, which contains one or more medicaments selected from a cofilin inhibitor, a PAK1 inhibitor, a LIMK inhibitor, a RHO inhibitor, a ROCK1 inhibitor and a ROCK2 inhibitor.

## Description

### Technical Field

The present invention relates to an agent for preventing or treating a trastuzumab-resistant cancer, which comprises one or more medicaments selected from a cofilin inhibitor, a PAK1 inhibitor, a LIMK inhibitor, a RHO inhibitor, a ROCK1 inhibitor and a ROCK2 inhibitor, and the like.

### Background Art

At present, trastuzumab (trade mark: herceptin), which is a conventional HER2 inhibitor, has been widely used for HER2-expressing cancer. However, it is known that certain cancers do not respond to trastuzumab even if it expresses HER2, and even HER2-dependent cancers, for which trastuzumab is effective, acquire resistance to trastuzumab during continuous treatment therewith (non-patent document 1). As the mechanism of acquiring resistance to HER2 inhibitor, overexpression of MUC4, compensatory signal transduction by other HER family, compensatory signal transduction by IGF-1 receptor, and altered downstream signaling pathway via PTEN/Akt are known (non-patent document 1, non-patent document 2). With these mechanisms, however, the aforementioned trastuzumab resistance cannot be explained.

As HER2 inhibitors, patent documents 1-3 disclose condensed pyrimidine derivatives. In addition, patent document 4 discloses a concomitant drug using such condensed pyrimidine derivative. However, a HER2 inhibitor still effective against the aforementioned trastuzumab-resistant cancer has not been reported heretofore.

Non-patent document 3 describes that EGF receptor signals are transmitted to Cofilin sequentially via Rho, ROCK, PAK and LIMK. Non-patent document 4 reports that the Rho/ROCK system is involved in the metastasis of cancer cells. Non-patent documents 5-7 describe anticancer action of a small-molecule Rho inhibitor (CCG-1423), anticancer action of a small-molecule Rho/ROCK inhibitor (Y-27632) in a liver cancer model and anticancer action of a small-molecule ROCK inhibitor (Wf-536) (suppression of metastasis of melanoma to lung cancer), respectively.
However, the connection between the trastuzumab-resistant cancer and the signal transduction pathway has not been known heretofore.
patent document 1: WO 2005/118588
patent document 2: WO 2007/073879
patent document 3: US 12/005,883
patent document 4: WO 2008/044782
non-patent document 1: Breast Cancer Research, 2006, 8: 215
non-patent document 2: Nature Clinical Practice Oncology, 2006, 3: 269
non-patent document 3: Nature Review 7, p431, 2007
non-patent document 4: FEBS Letters 580, p4252, 2006
non-patent document 5: Mol Cancer Ther 6, p2249, 2007
non-patent document 6: Hepatology Research 2008
non-patent document 7: Cancer Chemother Pharmacol 52, p319, 2003

### Disclosure of the Invention

### Problems to be Solved by the Invention

Administration of trastuzumab, which is a conventional HER2 inhibitor, to HER2 positive cancer cells causes resistance to the HER2 inhibitor in the cancer cells. The present invention aims to provide an agent for preventing or treating HER2 positive cancer, which is effective against such trastuzumab-resistant cancer.

### Means of Solving the Problems

The present inventors have conducted intensive studies and found that inhibition of cofilin or an enzyme (PAK1, LIMK, RHO, ROCK1, ROCK2) present at the upstream of cofilin in the cytoskeletal signaling is useful for the prophylaxis or treatment of trastuzumab-resistant cancer, and that a HER2 inhibitor including compound N-{2-[4-({3-chloro-4-[3-(trifluoromethyl)phenoxy]phenyl}amino)-5H-pyrrolo[3,2-dlpyrimidin-5-yl]ethyl}-3-hydroxy-3-methylbutanamide (compound A) alone is useful for the prophylaxis or treatment of trastuzumab-resistant cancer. They have further studied and completed the present invention.

Accordingly, the present invention relates to in the following.
[1] An agent for preventing or treating a trastuzumab-resistant cancer, comprising one or more medicaments selected from a cofilin inhibitor, a PAK1 inhibitor, a LIMK inhibitor, a RHO inhibitor, a ROCK1 inhibitor and a ROCK2 inhibitor.
[2] The agent of the above-mentioned [1], comprising a cofilin inhibitor.
[3] The agent of the above-mentioned [1], comprising a PAK1 inhibitor.
[4] The agent of the above-mentioned [1], comprising a LIMK inhibitor.
[5] The agent of the above-mentioned [1], comprising a RHO inhibitor.
[6] The agent of the above-mentioned [1], comprising a ROCK1 inhibitor.
[7] The agent of the above-mentioned [1], comprising a ROCK2 inhibitor.
[8] A combination drug comprising (1) a HER2 inhibitor and (2) one or more medicaments selected from a cofilin inhibitor, a PAK1 inhibitor, a LIMK inhibitor, a RHO inhibitor, a ROCK1 inhibitor and a ROCK2 inhibitor.
[9] The drug of the above-mentioned [8], wherein the HER2 inhibitor is trastuzumab or lapatinib.
[10] The drug of the above-mentioned [8], wherein the HER2 inhibitor is a compound represented by the formula

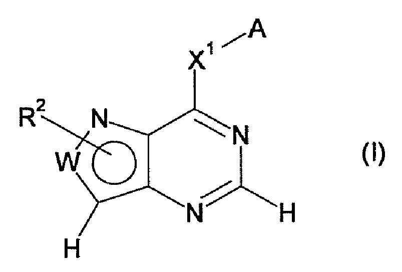

wherein W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or hetero atom on an aryl group or heteroaryl group for A to form an optionally substituted ring structure,
Y¹ is a single bond or C₁₋₄ alkylene or -O-(C₁₋₄ alkylene)-,
each of which is optionally substituted,
R¹ is a hydrogen atom, or an optionally substituted group bonded via a carbon atom, a nitrogen atom or an oxygen atom, and R² is a hydrogen atom, or an optionally substituted group bonded via a carbon atom or a sulfur atom, or
R¹ and R², or R² and R³ are optionally bonded to each other to form an optionally substituted ring structure, except a compound represented by the formula

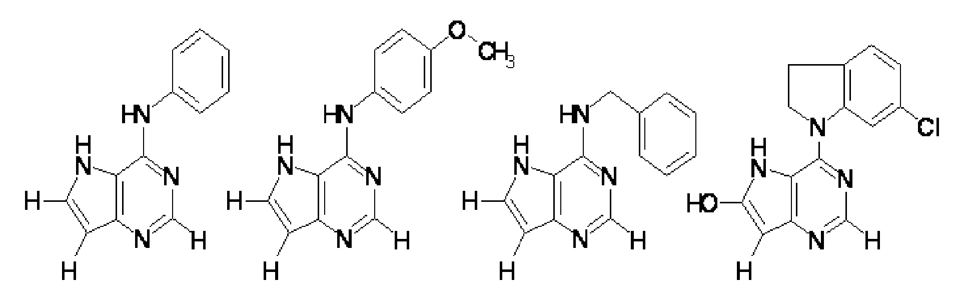

or a salt thereof.
[11] The drug of the above-mentioned [8], wherein the HER2 inhibitor is a compound represented by the formula

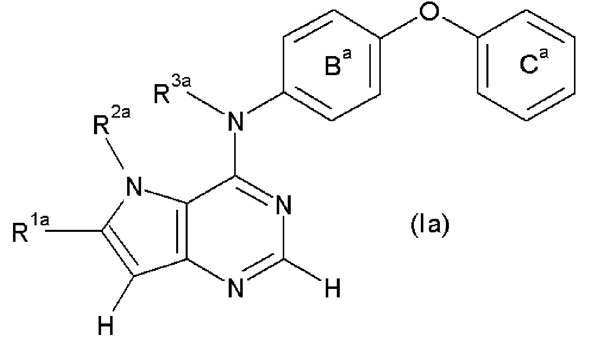

wherein
R^{1a} is a hydrogen atom, or an optionally substituted group bonded via a carbon atom, a nitrogen atom or an oxygen atom,
R^{2a} is an optionally substituted group bonded via a carbon atom or a sulfur atom, or
R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or R^{3a} is optionally bonded via a carbon atom of the adjacent phenyl group to form an optionally substituted ring structure,
B^{a} is an optionally substituted benzene ring, and C^{a} is an optionally substituted C₆₋₁₈ aryl group, or a salt thereof.
[12] The drug of the above-mentioned [8], wherein the HER2 inhibitor is a compound represented by the formula

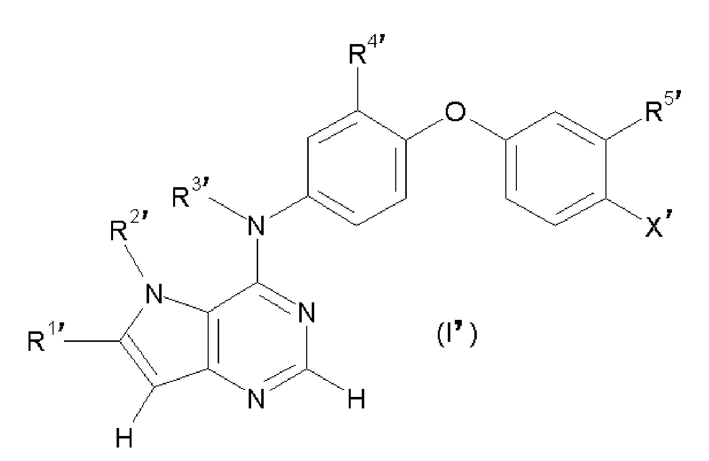

wherein
R^{1'} is a hydrogen atom,
R^{2'} is a C₁₋₆ alkyl group substituted by a group represented by -NR^{6'}-CO-(CH₂)ₙ-SO₂-(optionally halogenated C₁₋₄ alkyl) wherein n is an integer of 1 to 4, R^{6'} is a hydrogen atom or a C₁₋₄ alkyl group, wherein -(CH₂)ₙ- is optionally substituted by C₁₋₄ alkyl, R^{3'} is a hydrogen atom or a C₁₋₆ alkyl group,
R^{4'} is a halogen atom or a C₁₋₆ alkyl group,
R^{5'} is a halogen atom or a C₁₋₆ alkyl group, and
X' is a hydrogen atom or a halogen atom, except N-[2-(4-{[3-chloro-4-(3-chlorophenoxy)phenyl]amino}-5H-pyrrolo[3,2-d]pyrimidin-5-yl)ethyl]-2-(methylsulfonyl)acetamide, or a salt thereof.
[13] The drug of the above-mentioned [8], wherein the HER2 inhibitor is a compound represented by the formula

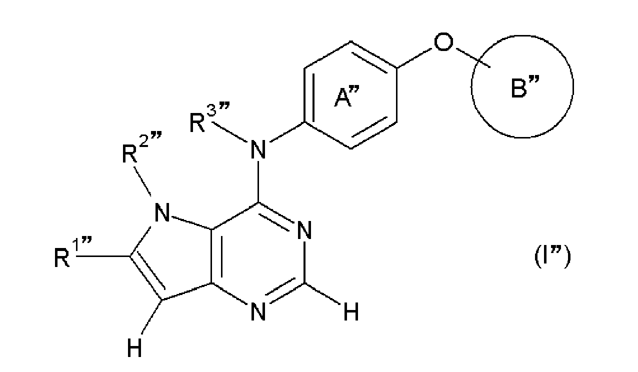

wherein
R^{1"} is a hydrogen atom, a halogen atom, or an optionally substituted group bonded via a carbon atom, a nitrogen atom or an oxygen atom,
R^{2"} is a hydrogen atom, or an optionally substituted group bonded via a carbon atom or a sulfur atom,
or R^{1"} and R^{2"}, or R^{2"} and R^{3"} are optionally bonded to each other to form an optionally substituted ring structure;
R^{3"} is a hydrogen atom or an optionally substituted aliphatic hydrocarbon group, or
R^{3"} is optionally bonded to a carbon atom of ring A" to form an optionally substituted ring structure;
ring A^{"} is an optionally substituted benzene ring;
ring B^{"} is
(i) an optionally substituted fused ring, or
(ii) a pyridine ring having optionally substituted carbamoyl wherein the pyridine ring is optionally further substituted, or a salt thereof.

[14] The drug of the above-mentioned [8], wherein the HER2 inhibitor is N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof.
[15] The drug of the above-mentioned [8], which is an agent for preventing or treating HER2-expressing cancer.
[16] A method of preventing or treating trastuzumab-resistant cancer, which comprises inhibiting one or more selected from cofilin, PAK1, LIMK, RHO, ROCK1 and ROCK2.
[17] The method of the above-mentioned [16], which comprises administering an effective amount of one or more medicaments selected from a cofilin inhibitor, a PAK1 inhibitor, a LIMK inhibitor, a RHO inhibitor, a ROCK1 inhibitor and a ROCK2 inhibitor to a mammal.
[18] Use of one or more medicaments selected from a cofilin inhibitor, a PAK1 inhibitor, a LIMK inhibitor, a RHO inhibitor, a ROCK1 inhibitor and a ROCK2 inhibitor for the production of an agent for preventing or treating a trastuzumab-resistant cancer.
[19] A method of examining the sensitivity of a HER2-expressing cancer to a HER2 inhibitor, comprising measuring the expression or activation state of one or more selected from cofilin, PAK1, LIMK, RHO, ROCK1 and ROCK2 in a sample collected from an animal having the cancer.
[20] A method of treating a cancer in an animal judged to be low sensitive to a HER2 inhibitor by the method of the above-mentioned [19], which comprises administering an effective amount of each of (1) a HER2 inhibitor, and (2) one or more medicaments selected from a cofilin inhibitor, a PAK1 inhibitor, a LIMK inhibitor, a RHO inhibitor, a ROCK1 inhibitor and a ROCK2 inhibitor to the animal.
[21] A method of screening for a drug for the prophylaxis or treatment of a trastuzumab-resistant cancer, comprising measuring and comparing the expression, activation state or activity of one or more selected from cofilin, PAK1, LIMK, RHO, ROCK1 and ROCK2 in a cell in the presence or absence of a test compound.
[22] An agent for preventing or treating a trastuzumab-resistant cancer, comprising N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof.
[23] A method of treating a trastuzumab-resistance cancer in a mammal, comprising administering an effective amount of N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof to the animal.

### Effect of the Invention

An agent for preventing or treating a trastuzumab-resistant cancer of the present invention, which comprises one or more medicaments selected from a cofilin inhibitor, a PAK1 inhibitor, a LIMK inhibitor, a Rho inhibitor, a ROCK1 inhibitor and a ROCK2 inhibitor, can be used effectively for the prophylaxis or treatment of a trastuzumab-resistant cancer not only by a single use but also in combination with a conventional HER2 inhibitor. In addition, even compound A alone can be used for the prophylaxis or treatment of a trastuzumab-resistant cancer.

### Brief Description of the Drawings

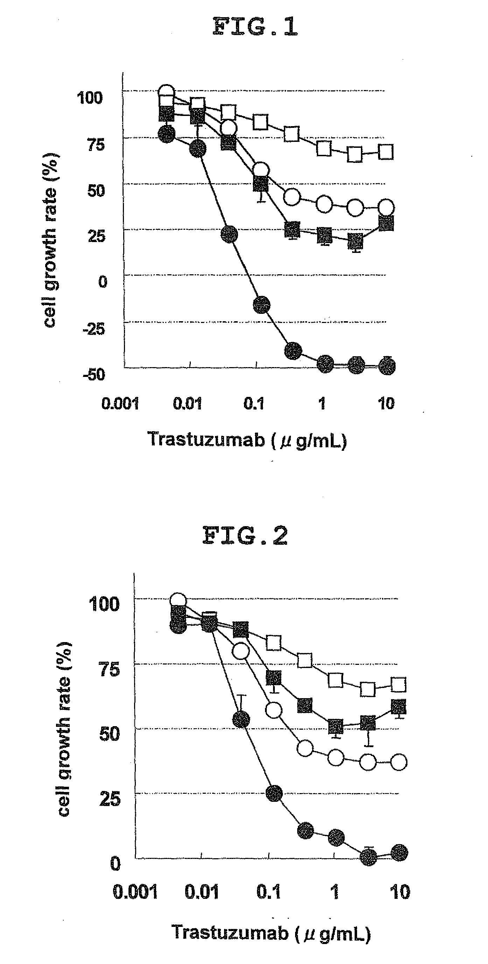

Fig. 1 shows that the sensitivity to a HER2 inhibitor is enhanced and a HER2 inhibitor becomes effective against low sensitive cell line by knockdown of LIMK1 with LIMK1 siRNA, wherein -•-_{:} high sensitive cell line, LIMK1 siRNA; -■-_{:} low sensitive cell line, LIMK1 siRNA; -○-_{:} high sensitive cell line, control siRNA; -□-: low sensitive cell line, control siRNA.
Fig. 2 shows that the sensitivity to a HER2 inhibitor is enhanced by knockdown of PAK1 with PAK1 siRNA, wherein -•-_{:} high sensitive cell line, PAK1 siRNA; -■-_{:} low sensitive cell line, PAK1 siRNA; -○-_{:} high sensitive cell line, control siRNA; -□-: low sensitive cell line, control siRNA.

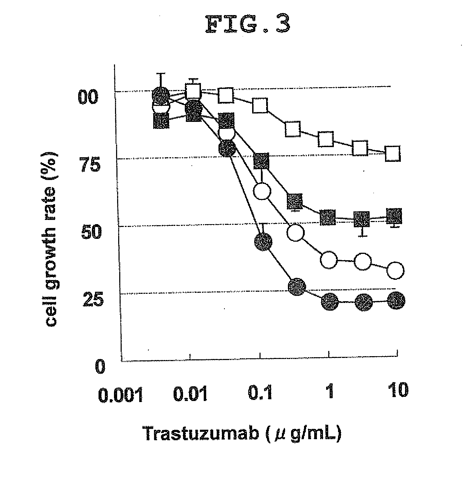

Fig. 3 shows that the sensitivity to a HER2 inhibitor is enhanced by knockdown of cofilin1 with cofilin1 siRNA, wherein -•-: high sensitive cell line, cofilin1 siRNA; -■-: low sensitive cell line, cofilin1 siRNA; -○-: high sensitive cell line, control siRNA; -□-: low sensitive cell line, control siRNA.

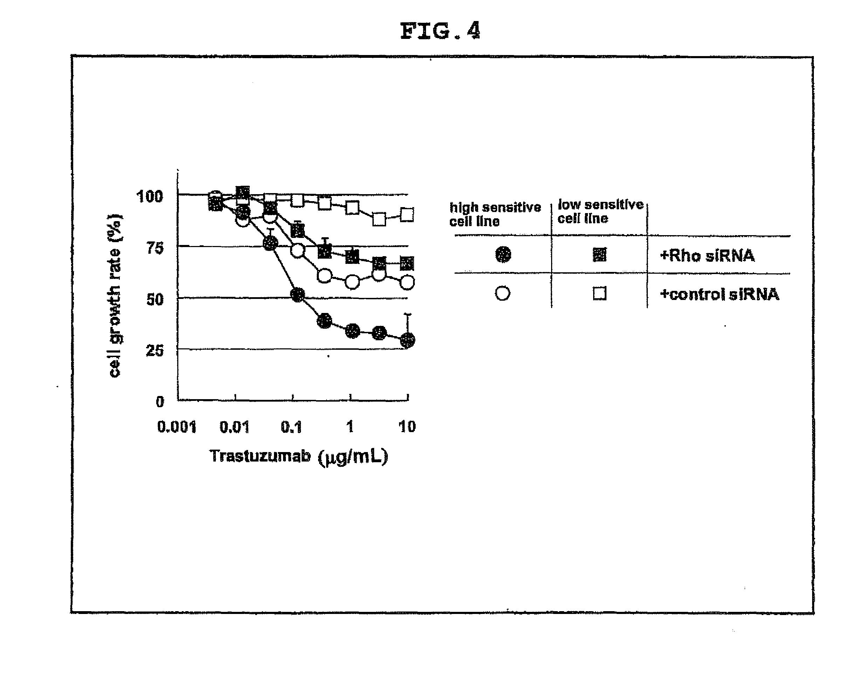

Fig. 4 shows that the sensitivity to a HER2 inhibitor is enhanced and a HER2 inhibitor becomes effective against low sensitive cell line by knockdown of Rho with Rho siRNA.

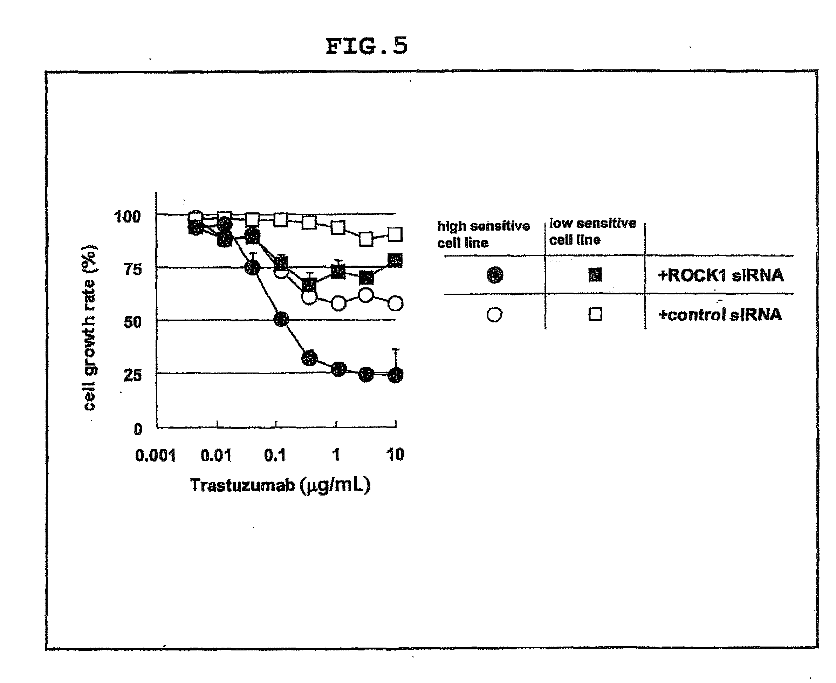

Fig. 5 shows that the sensitivity to a HER2 inhibitor is enhanced by knockdown of ROCK1 with ROCK1 siRNA.

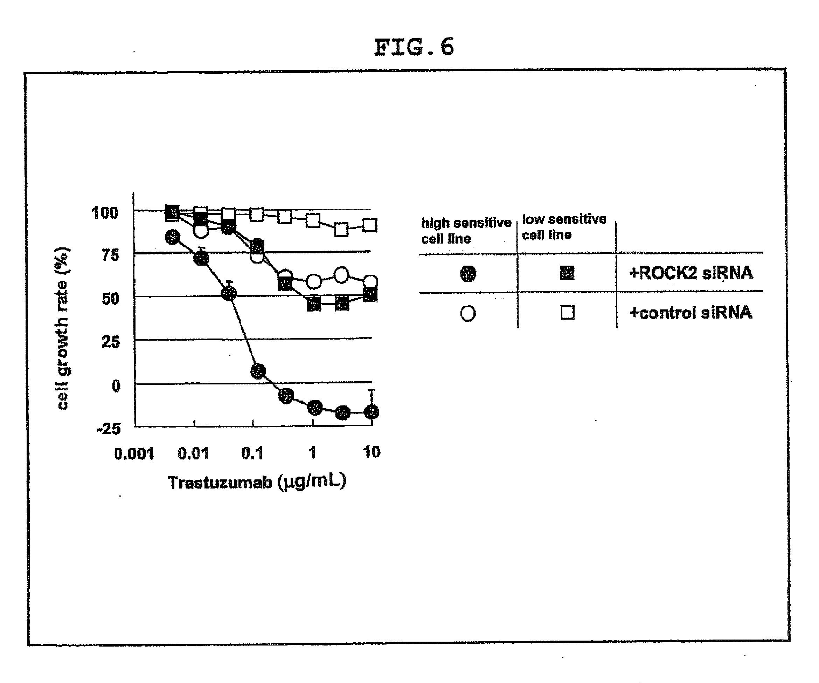

Fig. 6 shows that the sensitivity to a HER2 inhibitor is enhanced by knockdown of ROCK2 with ROCK2 siRNA.

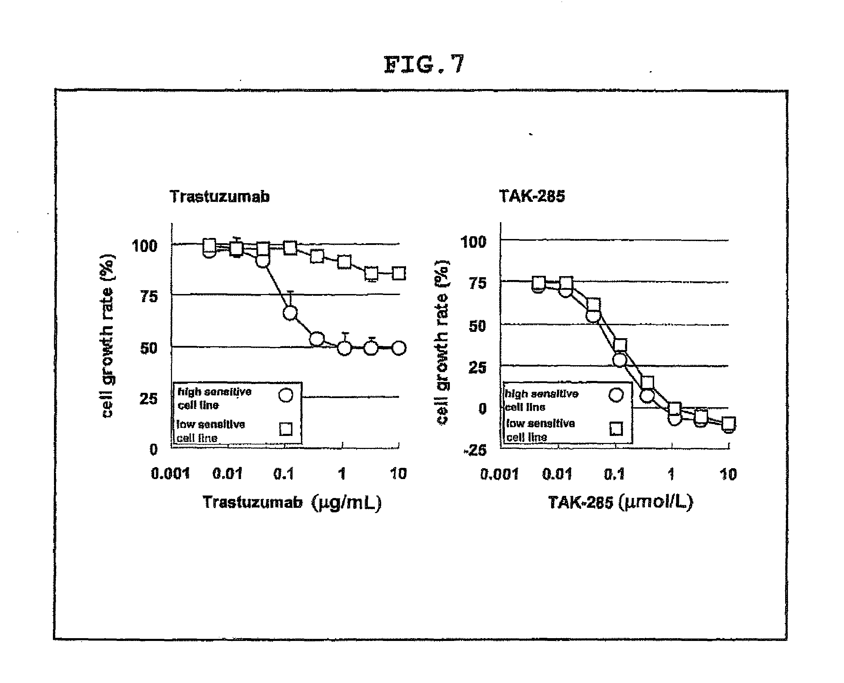

In Fig. 7, the left figure shows that high sensitive cell line and low sensitive cell line are different in the sensitivity against trastuzumab, and the right figure shows that the proliferation of both the high sensitive cell line and the low sensitive cell line is suppressed in a concentration-dependent manner by the addition of compound A.

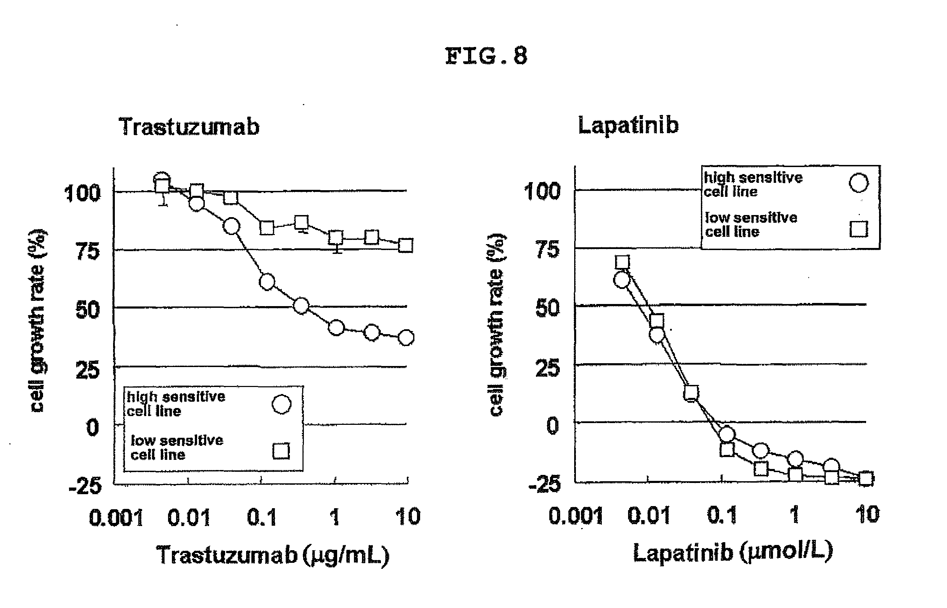

In Fig. 8, the left figure shows that high sensitive cell line and low sensitive cell line are different in the sensitivity against trastuzumab, and the right figure shows that the proliferation of both the high sensitive cell line and the low sensitive cell line is suppressed in a concentration-dependent manner by the addition of lapatinib.

### (Detailed Description of the Invention)

Cofilin in the present invention includes two isoforms of a protein comprising the same or substantially the same amino acid sequence as the amino acid sequence shown by SEQ ID NO:2 (cofilin 1; also referred to as CFL1) and a protein comprising the same or substantially the same amino acid sequence as the amino acid sequence shown by SEQ ID NO:4 (cofilin 2; also referred to as CFL2).
PAK1 (p21-activated kinase 1) in the present invention is a protein comprising the same or substantially the same amino acid sequence as the amino acid sequence shown by SEQ ID NO: 6.
LIMK (LIM domain kinase) in the present invention includes two isoforms of a protein (LIMK1) comprising the same or substantially the same amino acid sequence as the amino acid sequence shown by the amino acid sequence shown by SEQ ID NO: 8 and a protein (LIMK2) comprising the same or substantially the same amino acid sequence as the amino acid sequence shown by SEQ ID NO: 10.
RHO (Ras homolog) in the present invention is a protein (RHO) comprising the same or substantially the same amino acid sequence as the amino acid sequence shown by SEQ ID NO: 12.
ROCK (Rho-associated, coiled-coil containing protein kinase) in the present invention includes two isoforms of a protein (ROCK1) comprising the same or substantially the same amino acid sequence as the amino acid sequence shown by the amino acid sequence shown by SEQ ID NO: 14 and a protein (ROCK2) comprising the same or substantially the same amino acid sequence as the amino acid sequence shown by SEQ ID NO: 16.
Herein, proteins and peptides are described with the left end indicating the N-terminus (amino terminus) and the right end indicating the C-terminus (carboxyl terminus), according to the common practice of peptide designation.

The "amino acid sequence substantially the same as the amino acid sequence shown by SEQ ID NO: 2" means a natural allele variant or polymorphism of human CFL1 (RefSeq Accession No. NP_005498.1) consisting of the amino acid sequence shown by SEQ ID NO: 2 (same for chimpanzee and dogs), its ortholog in other warm-blooded animal [for example, mouse ortholog (having 98.8% identity with human CFL1 at amino acid level) registered with GenBank under RefSeq Accession No. NP_031713.1, rat ortholog (having 99.4% identity with human CFL1 at amino acid level) registered under RefSeq Accession No. NP_058843.1 and the like], and a natural allele variant or polymorphism of the ortholog.
The "amino acid sequence substantially the same as the amino acid sequence shown by SEQ ID NO: 4" means a natural allele variant or polymorphism of human CFL2 (RefSeq Accession No. NP_068733.1) consisting of the amino acid sequence shown by SEQ ID NO: 4 (same for dogs), its ortholog in other warm-blooded animal [for example, mouse ortholog (having 99.4% identity with human CFL2 at amino acid level) registered with GenBank under RefSeq Accession No. NP_031714.1, rat ortholog (having 99.4% identity with human CFL2 at amino acid level) registered under RefSeq Accession No. XP_345675.3, chimpanzee ortholog (having 100% identity with human CFL2 in the overlapped region at amino acid level) registered under RefSeq Accession No. XP_509898.1, chicken ortholog (having 97.0% identity with human CFL2 at amino acid level) registered under RefSeq Accession No. NP_001004406.1 and the like], and a natural allele variant or polymorphism of the ortholog.
The "amino acid sequence substantially the same as the amino acid sequence shown by SEQ ID NO: 6" means a natural allele variant or polymorphism of human PAK1 (RefSeq Accession No. NP_002567.3) consisting of the amino acid sequence shown by SEQ ID NO: 6, its ortholog in other warm-blooded animal [for example, mouse ortholog (having 97.6% identity with human PAK1 at amino acid level) registered with GenBank under RefSeq Accession No. NP_035165.1, rat ortholog (having 98.9% identity with human PAK1 at amino acid level) registered under RefSeq Accession No. NP_058894.1, dog ortholog (having 96.7% identity with human PAK1 at amino acid level) registered under RefSeq Accession No. XP_849651.1, chimpanzee ortholog (having 93.9% identity with human PAK1 at amino acid level) registered under RefSeq Accession No. XP_508657.2, chicken ortholog (having 94.3% identity with human PAK1 at amino acid level) registered under RefSeq Accession No. XP_417275.1 and the like], and a natural allele variant or polymorphism of the ortholog.
The "amino acid sequence substantially the same as the amino acid sequence shown by SEQ ID NO: 8" means a natural allele variant or polymorphism of human LIMK1 (RefSeq Accession No. NP_002305.1) consisting of the amino acid sequence shown by SEQ ID NO: 8, its ortholog in other warm-blooded animal [for example, mouse ortholog (having 95.2% identity with human LIMK1 at amino acid level) registered with GenBank under RefSeq Accession No. NP_034847.1, rat ortholog (having 95.2% identity with human LIMK1 at amino acid level) registered under RefSeq Accession No. NP_113915.1, dog ortholog (having 94.1% identity with human LIMK1 at amino acid level) registered under RefSeq Accession No. XP_849646.1, chimpanzee ortholog (having 98.6% identity with human LIMK1 at amino acid level) registered under RefSeq Accession No. XP_00114876.1 and the like], and a natural allele variant or polymorphism of the ortholog.
The "amino acid sequence substantially the same as the amino acid sequence shown by SEQ ID NO: 10" means a natural allele variant or polymorphism of human LIMK2 (RefSeq Accession No. NP_005560.1) consisting of the amino acid sequence shown by SEQ ID NO: 10, its ortholog in other warm-blooded animal [for example, mouse ortholog (having 93.1% identity with human LIMK2 at amino acid level) registered with GenBank under RefSeq Accession No. NP_034848.1, rat ortholog (having 92.9% identity with human LIMK2 at amino acid level) registered under RefSeq Accession No. NP_077049.2, dog ortholog (having 95.5% identity with human LIMK2 at amino acid level) registered under RefSeq Accession No. XP_852696.1 and the like], and a natural allele variant or polymorphism of the ortholog.
The "amino acid sequence substantially the same as the amino acid sequence shown by SEQ ID NO: 12" means a natural allele variant or polymorphism of human Rho (RefSeq Accession No. NP_001655.1) consisting of the amino acid sequence shown by SEQ ID NO: 12, its ortholog in other warm-blooded animal [for example, mouse ortholog (having 99.5% identity with human Rho at amino acid level) registered with GenBank under RefSeq Accession No. NP_058082.2, rat ortholog (having 99.5% identity with human Rho at amino acid level) registered under RefSeq Accession No. NP_476473.1, dog ortholog (having 99.5% identity with human Rho at amino acid level) registered under RefSeq Accession No. NP_001003273.1 and the like], and a natural allele variant or polymorphism of the ortholog.
The "amino acid sequence substantially the same as the amino acid sequence shown by SEQ ID NO: 14" means a natural allele variant or polymorphism of human ROCK1 (RefSeq Accession No. NP_005397.1) consisting of the amino acid sequence shown by SEQ ID NO: 14, its ortholog in other warm-blooded animal [for example, mouse ortholog (having 96.6% identity with human ROCK1 at amino acid level) registered with GenBank under RefSeq Accession No. NP_033097.1, rat ortholog (having 94.8% identity with human ROCK1 at amino acid level) registered under RefSeq Accession No. NP_112360.1, chimpanzee ortholog (having 99.9% identity with human ROCK1 at amino acid level) registered under RefSeq Accession No. XP_512051.2 and the like], and a natural allele variant or polymorphism of the ortholog.
The "amino acid sequence substantially the same as the amino acid sequence shown by SEQ ID NO: 16" means a natural allele variant or polymorphism of human ROCK2 (RefSeq Accession No. NP_004841.2) consisting of the amino acid sequence shown by SEQ ID NO: 16, its ortholog in other warm-blooded animal [for example, mouse ortholog (having 96.8% identity with human ROCK2 at amino acid level) registered with GenBank under RefSeq Accession No. NP_033098.2, bovine ortholog (having 97.8% identity with human ROCK2 at amino acid level) registered under RefSeq Accession No. NP_776877.1, chimpanzee ortholog (having 99.9% identity with human Rho at amino acid level) registered under RefSeq Accession No. XP_525689.2, rat ortholog (having 96.4% identity with human Rho at amino acid level) registered under RefSeq Accession No. NP_037154.1 and the like], and a natural allele variant or polymorphism of the ortholog.

In the present specification, cofilin (CFL1 and CFL2), PAK1, LIMK (LIMK1 and LIMK2), and Rho and ROCK (ROCK1 and ROCK2) are comprehensively sometimes to be abbreviated as "the target protein of the present invention", and the gene encoding same is abbreviated as the "target gene of the present invention".

In the present specification, the cofilin inhibitor refers to a substance that inhibits expression, activation (phosphorylation) or action of cofilin in cytoskeletal signaling. Here, the "inhibition of activation" includes dephosphorylation.
In the present specification, the PAK1 inhibitor refers to a substance that inhibits expression, activation (phosphorylation) or action of PAK1 in cytoskeletal signaling. Here, the "inhibition of activation" includes dephosphorylation.
In the present specification, the LIMK inhibitor refers to a substance that inhibits expression, activation (phosphorylation) or action of LIMK in cytoskeletal signaling. Here, the "inhibition of activation" includes dephosphorylation.
In the present specification, the Rho inhibitor refers to a substance that inhibits expression, activation (phosphorylation) or action of Rho in cytoskeletal signaling. Here, the "inhibition of activation" includes dephosphorylation.
In the present specification, the ROCK (ROCK1 and ROCK2) inhibitor refers to a substance that inhibits expression, activation (phosphorylation) or action of ROCK in cytoskeletal signaling. Here, the "inhibition of activation" includes dephosphorylation.

In the present invention, "a substance that inhibits the expression of target protein of the present invention" may be one that acts in any stage at the target gene transcription level, post-transcriptional regulation level, translation-into-protein level, post-translational modification level and the like. Therefore, examples of a substance that inhibits the expression of target protein include a substance that inhibits the transcription of the target gene, a substance that inhibits the processing of the primary transcription product into mRNA, a substance that inhibits the transportation of mRNA to cytoplasm, a substance that promotes the degradation of mRNA, a substance that inhibits the translation of mRNA into protein, a substance that inhibits the post-translational modification of target polypeptide and the like. Although any one that acts in any stage can be preferably used, a substance that inhibits the translation of mRNA into protein is preferred in that the production of target protein is directly inhibited.

As a substance capable of specifically inhibiting the translation of the mRNA of target gene into protein, preferably, a nucleic acid comprising a base sequence complementary or substantially complementary to the base sequence of one of these mRNAs or a portion thereof can be mentioned.
As a base sequence of CFL1 gene mRNA, the same or substantially the same base sequence as the base sequence shown by SEQ ID NO: 1 (human CFL1) can be mentioned. Here, the "substantially the same base sequence" refers to the base sequence of an ortholog, natural allele variant or polymorphism in other warm-blooded animal, as in the aforementioned target protein of the present invention.
As a base sequence of CFL2 gene mRNA, the same or substantially the same base sequence as the base sequence shown by SEQ ID NO: 3 (human CFL2) can be mentioned. Here, the "substantially the same base sequence" refers to the base sequence of an ortholog, natural allele variant or polymorphism in other warm-blooded animal, as in the aforementioned target protein of the present invention.
As a base sequence of PAK1 gene mRNA, the same or substantially the same base sequence as the base sequence shown by SEQ ID NO: 5 (human PAK1) can be mentioned. Here, the "substantially the same base sequence" refers to the base sequence of an ortholog, natural allele variant or polymorphism in other warm-blooded animal, as in the aforementioned target protein of the present invention.
As a base sequence of LIMK1 gene mRNA, the same or substantially the same base sequence as the base sequence shown by SEQ ID NO: 7 (human LIMK1) can be mentioned. Here, the "substantially the same base sequence" refers to the base sequence of an ortholog, natural allele variant or polymorphism in other warm-blooded animal, as in the aforementioned target protein of the present invention.
As a base sequence of LIMK2 gene mRNA, the same or substantially the same base sequence as the base sequence shown by SEQ ID NO: 9 (human LIMK2) can be mentioned. Here, the "substantially the same base sequence" refers to the base sequence of an ortholog, natural allele variant or polymorphism in other warm-blooded animal, as in the aforementioned target protein of the present invention.
As a base sequence of Rho gene mRNA, the same or substantially the same base sequence as the base sequence shown by SEQ ID NO: 11 (human Rho) can be mentioned. Here, the "substantially the same base sequence" refers to the base sequence of an ortholog, natural allele variant or polymorphism in other warm-blooded animal, as in the aforementioned target protein of the present invention.
As a base sequence of ROCK1 gene mRNA, the same or substantially the same base sequence as the base sequence shown by SEQ ID NO: 13 (human ROCK1) can be mentioned. Here, the "substantially the same base sequence" refers to the base sequence of an ortholog, natural allele variant or polymorphism in other warm-blooded animal, as in the aforementioned target protein of the present invention.
As a base sequence of the ROCK2 gene mRNA, the same or substantially the same base sequence as the base sequence shown by SEQ ID NO: 15 (human ROCK2) can be mentioned. Here, the "substantially the same base sequence" refers to the base sequence of an ortholog, natural allele variant or polymorphism in other warm-blooded animal, as in the aforementioned target protein of the present invention.
A base sequence substantially complementary to the base sequence of the mRNA of the target gene means a base sequence having a complementarity such that the base sequence is capable of binding to the target sequence for the mRNA to inhibit the translation thereof; specifically, for example, the base sequence is a base sequence having a homology of about 80% or more, preferably about 90% or more, more preferably about 95% or more, and most preferably about 98% or more, with respect to the overlapping region, to a base sequence completely complementary to the base sequence of the mRNA (i.e., the base sequence of a complementary strand of the mRNA).
Homology of the base sequences in the present specification can be calculated under the following conditions (an expectation value = 10; gaps are allowed; filtering = ON; match score = 1; mismatch score = -3) using a homology calculating algorithm NCBI BLAST (National Center for Biotechnology Information Basic Local Alignment Search Tool). As examples of other algorithms for determination of base sequence homology, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], and the algorithm described in Pearson et al., Proc. Natl. Acad. Sci. USA, 85:2444-2448 (1988) [the algorithm is incorporated in the FASTA program in the GCG software package] and the like can be mentioned, and these can also be preferably used in the same way.

More specifically, as a base sequence complementary or substantially complementary to the base sequence of the mRNA of the target gene, a base sequence complementary or substantially complementary to (a) the base sequence encoding the target gene or (b) a base sequence that hybridizes with the complementary chain of the base sequence under highly stringent conditions and encodes a protein having substantially the same quality of activity as the target protein can be mentioned. Here, "substantially the same quality of activity" is as defined above.
Highly stringent conditions refer to, for example, conditions involving a sodium concentration of about 19 to about 40mM, preferably about 19 to about 20mM, and a temperature of about 50 to about 70°C, preferably about 60 to about 65°C. In particular, a preferred case is such that the sodium salt concentration is about 19mM and the temperature is about 65°C.

"A portion of a base sequence complementary or substantially complementary to the base sequence of the mRNA of the target gene" is not particularly limited with respect to the length and position thereof, as far as the portion is capable of binding specifically to the mRNA of the target gene, and capable of inhibiting the protein translation from the mRNA; in terms of sequence specificity, the portion comprises at least 10 bases or more, preferably about 15 bases or more, and more preferably about 20 bases or more, of a portion complementary or substantially complementary to the target sequence.

Specifically, as a nucleic acid comprising a base sequence complementary or substantially complementary to the base sequence of the mRNA of the target gene or a portion thereof, any one of the following (a) to (c) can be preferably mentioned.
(a) An antisense nucleic acid against the mRNA of the target gene
(b) An siRNA against the mRNA of the target gene
(c) A nucleic acid capable of producing an siRNA against the mRNA of the target gene

### (a) An antisense nucleic acid against the mRNA of the target gene

"An antisense nucleic acid against the mRNA of the target gene" in the present invention is a nucleic acid comprising a base sequence complementary or substantially complementary to the base sequence of the mRNA or a portion thereof, and having the function of suppressing protein synthesis by binding to the target mRNA while forming a specific and stable double strand therewith.
Examples of the antisense nucleic acid include polydeoxyribonucleotides comprising 2-deoxy-D-ribose, polyribonucleotides comprising D-ribose, other types of polynucleotides being N-glycosides of the purine or pyrimidine base, other polymers having a non-nucleotide backbone (for example, commercially available protein nucleic acids and nucleic acid polymers specific for synthetic sequences) or other polymers comprising a special linkage (provided that the polymers comprise nucleotides having such an alignment that allows base pairing or base attachment, as found in DNA or RNA) and the like. These may be double-stranded DNAs, single-stranded DNAs, double-stranded RNAs, single-stranded RNAs, or DNA:RNA hybrids, and may also be unmodified polynucleotides (or unmodified oligonucleotides); those with known modifications, for example, those with labels known in the art, those with caps, those methylated, those with substitution of one or more naturally occurring nucleotides with their analogues, those with intramolecular modifications of nucleotides such as those with uncharged linkages (for example, methyl phosphonates, phosphotriesters, phosphoramidates, carbamates and the like) and those with charged linkages or sulfur-containing linkages (e.g., phosphorothioates, phosphorodithioates and the like); those having side chain groups such as proteins (e.g., nucleases, nuclease inhibitors, toxins, antibodies, signal peptides, poly-L-lysine and the like) or saccharides (e.g., monosaccharides and the like); those with intercalators (e.g., acridine, psoralen and the like); those with chelators (for example, metals, radioactive metals, boron, oxidative metals and the like); those with alkylating agents; or those with modified linkages (for example, α anomeric nucleic acids and the like). Here, "nucleosides", "nucleotides" and "nucleic acids" may include those not only comprising the purine and pyrimidine bases, but also comprising other modified heterocyclic bases. Such modified products may comprise a methylated purine and pyrimidine, an acylated purine and pyrimidine, and other heterocyclic ring. Modified nucleosides and modified nucleotides may have a modification in the sugar moiety thereof; for example, one or more hydroxyl groups may be substituted by halogens, aliphatic groups and the like, or may be converted into functional groups such as ethers and amines.

As stated above, the antisense nucleic acid may be a DNA or RNA, or a DNA/RNA chimera. When the antisense nucleic acid is a DNA, a RNA:DNA hybrid formed by a target RNA and antisense DNA can be recognized by endogenous RNase H to cause selective degradation of the target RNA. Therefore, in the case of an antisense DNA intended to cause degradation by RNase H, the target sequence may be not only a sequence in the mRNA, but also the sequence of an intron region in the primary translation product of the target gene.

The target region for an antisense nucleic acid of the present invention is not particularly limited with respect to the length thereof, as far as the translation into target protein is inhibited as a result of hybridization of the antisense nucleic acid; the target region may be the entire sequence or a partial sequence of the mRNA that encodes the protein, and the length is about 10 bases for the shortest, and the entire sequence of the mRNA or primary transcription product for the longest. Taking into account the issues of the ease of synthesis, antigenicity, and internalization and the like, an oligonucleotide consisting of about 10 to about 40 bases, particularly about 15 to about 30 bases, is preferable, but this is not to be construed as limiting. Specifically, the 5' end hairpin loops, 5' end 6-base-pair repeats, 5' untranslated regions, translation initiation codons, protein coding regions, ORF translation stop codons, 3' untranslated regions, 3' end palindrome regions, 3' end hairpin loops and the like of the target gene can be chosen as preferable target regions for the antisense nucleic acid, but these are not to be construed as limiting.

Furthermore, an antisense nucleic acid of the present invention may be one that not only hybridizes with the mRNA or primary transcription product of the target gene to inhibit the translation into protein, but also is capable of binding to these genes, which are double-stranded DNAs, to form a triple strand (triplex) and inhibit the transcription into RNA (anti-gene).

Although the nucleotide molecules that constitute the antisense nucleic acid may be natural-type RNAs or DNAs, the molecules can comprise various chemical modifications in order to increase the stability (chemical and/or to-enzyme) or specific activity (affinity for RNA). For example, to prevent degradation by hydrolases such as nuclease, the phosphoric acid residue (phosphate) of each nucleotide that constitutes the antisense nucleic acid can be substituted with, for example, a chemically modified phosphoric acid residue such as phosphorothioate (PS), methylphosphonate, or phosphorodithionate. The hydroxyl group at the 2'-position of the sugar (ribose) of each nucleotide may be replaced with -OR (R represents, for example, CH₃(2'-O-Me), CH₂CH₂OCH₃(2'-O-MOE), CH₂CH₂NHC(NH)NH₂, CH₂CONHCH₃, CH₂CH₂CN or the like). Furthermore, a base moiety (pyrimidine, purine) may be chemically modified; for example, introduction of a methyl group or a cationic functional group into the 5-position of the pyrimidine base, substitution of the 2-position carbonyl group with thiocarbonyl and the like can be mentioned.

Regarding the conformation of the sugar moiety of RNA, two types are dominant: C2'-endo (S-type) and C3'-endo (N-type); in single-stranded RNA, the sugar moiety occurs in an equilibrium of the two types, but when a double strand is formed, the conformation is fixed for the type N. Therefore, BNA (LNA) (Imanishi, T. et al., Chem. Commun., 1653-9, 2002; Jepsen, J.S. et al., Oligonucleotides, 14, 130-46, 2004), or ENA (Morita, K. et al., Nucleosides Nucleotides Nucleic Acids, 22, 1619-21, 2003), an RNA derivative wherein the conformation of the sugar moiety is fixed to the type N by bridging the 2' oxygen and 4' carbon so as to confer strong bindability to the target RNA, can also be preferably used.

An antisense oligonucleotide of the present invention can be prepared by determining the target sequence for the mRNA or primary transcription product on the basis of the cDNA sequence or genomic DNA sequence of the target gene, and synthesizing a sequence complementary thereto using a commercially available automated DNA/RNA synthesizer (Applied Biosystems, Beckman and the like). All antisense nucleic acids comprising the aforementioned various modifications can be chemically synthesized by techniques known per se.

### (b) siRNA against mRNA of target gene

Herein, a double-stranded RNA consisting of an oligo-RNA complementary to the mRNA of the target gene and a complementary chain thereof, what is called an siRNA, is also defined as being included in nucleic acids comprising a base sequence complementary or substantially complementary to the base sequence of the mRNA of the target gene or a portion thereof. It had been known that so-called RNA interference (RNAi), which is a phenomenon wherein if short double-stranded RNA is introduced into a cell, mRNAs complementary to the RNA are degraded, occurs in nematodes, insects, plants and the like; since this phenomenon was confirmed to also occur widely in animal cells [Nature, 411(6836): 494-498 (2001)], RNAi has been widely utilized as an alternative technique to ribozymes. An siRNA can be designed as appropriate on the basis of base sequence information on the target mRNA using commercially available software (e.g., RNAi Designer; Invitrogen). Specifically, examples of preferable siRNAs of the present invention include, but are not limited to, siRNAs used in Examples described below and the like.

Ribonucleoside molecules constituting an siRNA may also undergo the same modifications as in the above-described antisense nucleic acids in order to increase the stability, specific activity and the like. However, in the case of an siRNA, if all ribonucleoside molecules in the natural type RNA are substituted by the modified form, the RNAi activity is sometimes lost, so that it is necessary that the minimum number of modified nucleosides be introduced to allow the RISC complex to function.

An siRNA can be prepared by synthesizing a sense strand and antisense strand of a target sequence on the mRNA using an automated DNA/RNA synthesizer, respectively, and denaturing the strands in an appropriate annealing buffer solution at about 90 to about 95°C for about 1 minute, and thereafter annealing the strands at about 30 to about 70°C for about 1 to about 8 hours. An siRNA can also be prepared by synthesizing a short hairpin RNA (shRNA) serving as an siRNA precursor, and cleaving this using a dicer.

### (c) Nucleic acids capable of producing siRNA against mRNA of target gene

Herein, a nucleic acid designed to be capable of producing the above-described siRNA against the mRNA of the target gene in vivo is also defined as being included in nucleic acids comprising a base sequence complementary or substantially complementary to the base sequence of the mRNA of the target gene or a portion thereof. As such nucleic acids, the aforementioned shRNA, expression vectors constructed to express the shRNA and the like can be mentioned. An shRNA can be prepared by designing an oligo-RNA comprising a base sequence prepared by joining a sense chain and antisense chain of a target sequence on mRNA via a spacer sequence having a length allowing it to form an appropriate loop structure (for example, about 15 to 25 bases) inserted therebetween, and synthesizing this using an automated DNA/RNA synthesizer. An expression vector comprising an shRNA expression cassette can be prepared by preparing a double-stranded DNA that encodes the above-described shRNA by a conventional method, and thereafter inserting the DNA into an appropriate expression vector. As the shRNA expression vector, one having a Pol III promoter such as U6 or H1 can be used. In this case, an shRNA transcribed in an animal cell carrying the expression vector forms a loop by itself, and is thereafter processed by an endogenous enzyme dicer and the like, whereby a mature siRNA is formed.

As another preferred example of a nucleic acid comprising a base sequence complementary or substantially complementary to the base sequence of the mRNA of target gene or a portion thereof, a ribozyme capable of specifically cleaving the mRNA in the coding region can be mentioned. Although "ribozyme", in the narrow sense, refers to an RNA possessing enzymatic activity to cleave nucleic acids, the term is used herein as a concept encompassing any DNA possessing sequence-specific nucleic acid cleavage activity. The most versatile ribozyme is self-splicing RNA, which is found in infectious RNAs such as viroid and virusoid, and is known in the hammerhead type, hairpin type and the like. The hammerhead type exhibits enzyme activity with about 40 bases, and it is possible to specifically cleave only a target mRNA by rendering several bases at both ends adjacent to the hammerhead structure portion (about 10 bases in total) complementary to the desired cleavage site of mRNA. Because this type of ribozyme has RNA as the only substrate, the same has a further advantage that genomic DNA is never attacked. When the mRNA encoding the target protein of the present invention has a double strand structure by itself, the target sequence can be made to be single stranded by using a hybrid ribozyme coupled to an RNA motif derived from a virus nucleic acid capable of binding specifically to RNA helicase [Proc. Natl. Acad. Sci. USA, 98(10): 5572-5577 (2001)]. Furthermore, when ribozyme is used in the form of an expression vector comprising the DNA that encodes the same, the ribozyme may be a hybrid ribozyme further coupled with a sequence of altered tRNA to promote the localization of the transcription product to cytoplasm [Nucleic Acids Res., 29(13): 2780-2788 (2001)].

A nucleic acid comprising a base sequence complementary or substantially complementary to the base sequence of the mRNA of the target gene or a portion thereof can be supplied in a special form such as liposomes or microspheres, or applied to gene therapy, or given in a form added to something. Those used for such addition include polycations that act to neutralize the charge of phosphate backbone, such as polylysines, and hydrophobic ones such as lipids (e.g., phospholipids, cholesterols and the like) that enhance the interaction with cell membrane or increase nucleic acid uptake. Lipids preferred for addition are cholesterols and derivatives thereof (e.g., cholesteryl chloroformate, cholic acid and the like). These moieties may be attached to the 3' end or 5' end of a nucleic acid, and can also be attached via a base, sugar, or intramolecular nucleoside linkage. Other groups may be capping groups placed specifically at the 3' end or 5' end of the nucleic acid to prevent degradation by nucleases such as exonuclease and RNase. Such capping groups include, but are not limited to, hydroxyl protecting groups known in the art, including glycols such as polyethylene glycol and tetraethylene glycol.

The inhibitory activities of these nucleic acids on the expression of the target protein can be examined using a transformant carrying the target gene, an in vivo and in vitro expression system for the target gene, or an in vivo or in vitro translation system for the target protein.

A substance that inhibits the expression of the target protein in the present invention is not limited to the above-described nucleic acids comprising a base sequence complementary or substantially complementary to the base sequence of the mRNA encoding the target protein or a portion thereof. As far as the substance directly or indirectly inhibits the production of the target protein, it may be other substance such as peptide, protein, organism-derived nonpeptidic compound (carbohydrates, lipid etc.), synthetic compound, microorganism culture, cell extract, plant extract, animal tissue extract and the like. These substances may be novel substances or known substances. Such a substance can be acquired by, for example, the screening method of the present invention described below.

The "substance that inhibits activation of the target protein" in the present invention may be any as long as it inhibits a produced target protein from being present in an activated state, i.e., phosphorylated state, and divided into a substance that inhibits phosphorylation of a target protein and a substance that promotes dephosphorylation thereof. Examples of the former include, but are not limited to, a peptide to be, competitively with a target protein, a substrate for an enzyme that phosphorylates the target protein (e.g., a peptide to be a LIMK substrate competitively with cofilin, a peptide to be a PAK1 or ROCK1 or ROCK2 substrate competitively with LIMK, a peptide to be a Rac substrate competitively with PAK1, a peptide to be a Rho substrate competitively with ROCK1 or ROCK2 etc.) and the like, and examples of the latter include, but are not limited to, phosphatase that acts on a target protein (e.g., slingshot (ssh) that dephosphorylates cofilin etc.) and the like. Examples of the peptide to be, competitively with a target protein, a substrate for an enzyme that phosphorylates the target protein include a fragment thereof containing a target protein phosphorylation site (for example, Ser (third from the N terminal) for cofilin, etc.), which lacks a physiologically active domain of the target protein (actin polymerization activity etc. for cofilin, LIMK and cofilin activation (phosphorylation) activity etc. for PAK1, ROCK1, ROCK2 and LIMK, and ROCK1 or ROCK2 activation (phosphorylation) activity for Rho, etc.) and the like. The phosphorylation site and physiologically active domain of the target protein of the present invention are known, and those of ordinary skill in the art can easily design or synthesize a peptide that can competitively act as a substrate (phosphoric acid group receptor) with the target protein, based on the information of the amino acid sequences (for example, SEQ ID NO: 2, 4, 6, 8, 10, 12, 14 or 16 for human) of cofilin, PAK1, LIMK, Rho, ROCK1 and ROCK2.

In the present invention, "a substance that inhibits the action of the target protein of the present invention" may be any as long as it inhibits the target protein once produced functionally and activated (phosphorylated) from exhibiting its physiological activities (actin polymerization activity etc. for cofilin, LIMK and cofilin activation (phosphorylation) activity for PAK1 or ROCK1 or ROCK2 and LIMK, respectively, etc., ROCK1 or ROCK2 activation (phosphorylation) activity etc. for Rho); for example, a substance that inhibits dissociation of the phosphorylated cofilin from actin fiber, a substance that inhibits binding of PAK1 or ROCK1 or ROCK2 and LIMK to LIMK and cofilin and/or phosphorylation thereof, a substance that inhibits binding of Rho to ROCK1/2 and/or phosphorylation thereof and the like.

Specifically, as an example of a substance that inhibits the action of the target protein (particularly Rho, ROCK1, ROCK2, PAK1 and LIMK) of the present invention, an antibody against said protein can be mentioned. The antibody may be a polyclonal antibody or a monoclonal antibody. These antibodies can be produced according to a method of antibody or antiserum production known per se. The isotype of the antibody is not particularly limited, and is preferably IgG, IgM or IgA, particularly preferably IgG. The antibody is not particularly limited, as far as it has at least a complementarity determining region (CDR) for specifically recognizing and binding to a target antigen, and the antibody may be, in addition to a complete antibody molecule, for example, a fragment such as Fab, Fab', or F(ab')₂, a conjugate molecule prepared by a gene engineering technique, such as scFv, scFv-Fc, minibody, or diabody, or a derivative thereof modified with a molecule having protein-stabilizing action, such as polyethylene glycol (PEG).
In a preferred embodiment, the antibody against the target protein of the present invention is used as a medicament for a human recipient, the antibody (preferably monoclonal antibody) is an antibody having a reduced risk of exhibiting antigenicity when administered to humans, specifically a complete human antibody, a humanized antibody, a mouse-human chimera antibody or the like, and particularly preferably a complete human antibody. A humanized antibody and a chimera antibody can be prepared by gene engineering according to a conventional method. Although a complete human antibody can also be produced from a human-human (or mouse) hybridoma, it is desirable, for supplying a large amount of antibody stably and at low cost, that the antibody be produced using a human antibody-producing mouse or the phage display method.

Since the target protein of the present invention is an intracellular signal transduction molecule constituting the cytoskeleton signaling cascade, a substance that inhibits the activity of said protein is desirably a substance of excellent entry into cells. Therefore, a more preferable substance that inhibits the action of the target protein of the present invention is a low-molecular compound that complies with Lipinski's Rule. Such a compound can be acquired by, for example, using the screening method of the present invention described below.

Since a substance that inhibits expression, activation (phosphorylation) or action of the target protein of the present invention, namely, an inhibitor of the target protein of the present invention shows superior anticancer action such as cell proliferation suppressive effect and the like even on a trastuzumab resistant (low sensitive) cancer, it is useful for the prophylaxis and/or treatment of a HER2-expressing cancer, irrespective of the sensitivity against trastuzumab.
Therefore, a medicament containing one or more medicaments selected from the inhibitors of the target proteins of the present invention (cofilin, PAK1, LIMK, Rho, ROCK1, ROCK2) can be used as an agent for preventing or treating a trastuzumab-resistant (low sensitive) cancer and the like.

In the present specification, cancer means a HER2 positive cancer, and refers to various carcinomas (particularly breast cancer (e.g., invasive ductal carcinoma, ductal cancer in situ, inflammatory breast cancer, etc.), prostate cancer (e.g., hormone-dependent prostate cancer, non-hormone dependent prostate cancer, etc.), pancreatic cancer (e.g., pancreatic duct cancer, etc.), gastric cancer (e.g., papillary adenocarcinoma, mucinous adenocarcinoma, adenosquamous cancer, etc.), lung cancer (e.g., non-small cell lung cancer, small cell lung cancer, malignant mesothelioma, etc.), colon cancer (e.g., gastrointestinal stromal tumor, etc.), rectal cancer (e.g., gastrointestinal stromal tumor, etc.), colorectal cancer (e.g., familial colorectal cancer, hereditary nonpolyposis colorectal cancer, gastrointestinal stromal tumor, etc.), small intestinal cancer (e.g., non-Hodgkin lymphoma, gastrointestinal stromal tumor, etc.), esophagus cancer, duodenal cancer, cancer of tongue, pharyngeal cancer (e.g., nasopharyngeal carcinoma, oropharyngeal carcinoma, hypopharyngeal carcinoma, etc.), salivary gland cancer, brain tumor (e.g., pineal astrocytoma, pilocytic astrocytoma, diffuse astrocytoma, anaplastic astrocytoma, etc.), schwannoma, liver cancer (e.g., primary liver cancer, extrahepatic bile duct cancer, etc.), kidney cancer (e.g., renal cell carcinoma, transitional cell cancer of renal pelvis and ureter, etc.), bile duct cancer, endometrial cancer, carcinoma of the uterine cervix, ovary cancer (e.g., ovarian epithelial cancer, extragonadal germ cell tumor, ovarian germ cell tumor, ovarial low malignant potential tumor, etc.), urinary bladder cancer, urethral cancer, skin cancer (e.g., ocular melanoma, Merkel cell carcinoma, etc.), Hemangioma, malignant lymphoma, malignant melanoma, thyroid cancer (e.g., medullary thyroid cancer, etc.), parathyroid cancer, nasal cancer, paranasal cancer, bone tumor (e.g., osteosarcoma, Ewing's tumor, uterus sarcoma, soft tissue sarcoma, etc.), vascular fibroma, retinoblastoma, penile cancer, testis tumor, solid cancer in childhood (e.g., Wilms' tumor, childhood kidney tumor, etc.), Kaposi's sarcoma, Kaposi's sarcoma related to AIDS, maxillary sinus tumor, fibrous histiocytoma, leiomyosarcoma, rhabdomyosarcoma, leukemia (e.g., acute myelocytic leukemia, acute lymphoblastic leukemia, etc.), etc.), and the like. It particularly refers to breast cancer, prostate cancer, gastric cancer, lung cancer, colon cancer, rectal cancer, colorectal cancer, esophagus cancer, pharyngeal cancer, ovary cancer, urinary bladder cancer and the like.

### (1) Medicament containing antisense nucleic acid, siRNA, or precursor nucleic acid thereof

A medicament comprising the above-mentioned nucleic acid comprising a base sequence complementary or substantially complementary to the base sequence of the mRNA of the target gene of the present invention or a portion thereof is of low toxicity, and can be administered as a liquid as it is, or as an appropriate dosage form of pharmaceutical composition, to humans or non-human mammals (e.g., mice, rats, rabbits, sheep, pigs, bovines, cats, dogs, monkeys and the like) orally or parenterally (e.g., intravascular administration, subcutaneous administration and the like).

When the nucleic acid of the present invention is used as an agent for preventing or treating cancer, the nucleic acid can be prepared and administered according to a method known per se. That is, the nucleic acid alone or after being functionally inserted into an appropriate expression vector for mammalian cells, such as a retrovirus vector, adenovirus vector, or adenovirus-associated virus vector, can be prepared according to a standard means. The nucleic acid can be administered as it is, or along with an auxiliary for promoting its uptake, using a gene gun or a catheter such as a hydrogel catheter. Alternatively, the nucleic acid can be prepared as an aerosol and topically administered into the trachea as an inhalant.
Furthermore, for the purpose of improving the disposition, extending the half-life, and increasing the intracellular uptake efficiency, the aforementioned nucleic acid may be prepared as a preparation (injection) alone or with a carrier such as a liposome, and administered intravenously, subcutaneously and the like.

A nucleic acid of the present invention may be administered as it is, or as an appropriate pharmaceutical composition. The pharmaceutical composition used for administration may contain both a nucleic acid of the present invention and a pharmacologically acceptable carrier, diluent or excipient. Such a pharmaceutical composition is supplied in the form of a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections and drip infusion injections. Such an injection can be prepared according to a publicly known method. An injection can be prepared by, for example, dissolving, suspending or emulsifying the above-described nucleic acid of the present invention in a sterile aqueous or oily solution in common use for injections. As examples of aqueous solutions for injection, physiological saline, an isotonic solution containing glucose or other auxiliary agent, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is preferably filled in an appropriate ampoule. Suppositories used for rectal administration may be prepared by mixing the above-described nucleic acid in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a publicly known method, and may contain a carrier, diluent or excipient in common use in the field of pharmaceutical production. As examples of the carrier or excipient for tablets, lactose, starch, sucrose, magnesium stearate and the like can be used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. It is preferable that a nucleic acid of the present invention be contained at, for example, normally 5 to 500 mg, particularly 5 to 100 mg for injections, or 10 to 250 mg for other dosage forms, per medication unit dosage form.

The dose of the above-described medicament containing the nucleic acid of the present invention varies depending on the subject of administration, target disease, symptoms, route of administration and the like; for example, when the medicament is used for the treatment/prevention of cancer of an adult, it is convenient to administer the nucleic acid of the present invention usually at about 0.01 to 20 mg/kg body weight, preferably about 0.1 to 10 mg/kg body weight, and more preferably about 0.1 to 5 mg/kg body weight, based on a single dose, about 1 to 5 times a day, preferably about 1 to 3 times a day, by intravenous injection. In the case of other modes of parenteral administration and oral administration, similar doses may be administered. In case the symptom is particularly severe, the dose may be increased according to the symptom.

Each of the aforementioned compositions may comprise any other active ingredient that does not produce an unwanted interaction when formulated with a nucleic acid of the present invention.

### (2) Medicament containing peptide, phosphatase, antibody and the like

A medicament containing the aforementioned peptide, phosphatase etc. that inhibit presence of the target protein of the present invention in an activated (phosphorylated) state, or an antibody against the target protein (particularly human antibody, humanized antibody etc.) is of low toxicity, and can be administered as a liquid as it is, or as an appropriate dosage form of pharmaceutical composition, to humans or mammals (e.g., mice, rats, rabbits, sheep, pigs, bovines, cats, dogs, monkeys and the like) orally or parenterally (e.g., intravascular administration, subcutaneous administration and the like).

The above-described peptide, phosphatase, antibody and the like may be administered as it is, or as an appropriate pharmaceutical composition. The pharmaceutical composition used for administration may contain both the above-described peptide, phosphatase, antibody and the like, and a pharmacologically acceptable carrier, diluent or excipient. Such a pharmaceutical composition is provided as a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. An injection can be prepared by, for example, dissolving, suspending or emulsifying the above-described peptide, phosphatase, antibody and the like in a sterile aqueous or oily solution in common use for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with solubilizers such as benzyl benzoate, benzyl alcohol. The injection solution prepared is preferably filled in an appropriate ampoule. Suppositories used for rectal administration may be prepared by mixing the above-described peptide, phosphatase, antibody and the like in an ordinary suppository base.

For example, the composition for oral administration includes solid or liquid preparations, specifically, tablets (including sugar-coated tables and film-coated tablets), pills, granules, powder, capsules (including soft capsules), syrup, emulsions, suspensions, etc. Such a composition is manufactured by publicly known methods and may contain a carrier, a diluent or excipient conventionally used in the field of pharmaceutical preparations. Examples of the carrier or excipient for tablets are lactose, starch, sucrose, magnesium stearate, etc.

Advantageously, the pharmaceutical compositions for parenteral or oral administration described above are prepared into a medication unit dosage form suited to fit a dose of the active ingredients. Such a medication unit dosage form includes, for example, tablets, pills, capsules, injections (ampoules), suppositories, etc. It is preferable that the antibody be contained normally at 5 to 500 mg, particularly 5 to 100 mg for injections, or 10 to 250 mg for other dosage forms, per medication unit dosage form.

The dose of the above-described medicament containing the above-described peptide, phosphatase, antibody and the like varies depending on the subject of administration, target disease, symptoms, route of administration and the like; for example, when the medicament is used for the treatment/prevention of cancer in an adult, it is convenient to administer the peptide, phosphatase, antibody and the like usually at about 0.01 to 20 mg/kg body weight, preferably about 0.1 to 10 mg/kg body weight, and more preferably 0.1 to 5 mg/kg body weight, based on a single dose, about 1 to 5 times a day, preferably about 1 to 3 times a day, by intravenous injection. In the case of other parenteral administrations and oral administration, a similar dose can be administered. If the symptom is particularly severe, the dosage may be increased depending on the symptom.

The above-described peptide, phosphatase, antibody and the like can be administered as it is, or as an appropriate pharmaceutical composition. The pharmaceutical composition used for the above-described administration contains both the above-described antibody and a pharmacologically acceptable carrier, diluent or excipient. Such a composition is supplied in the form of a dosage form suitable for oral or parenteral administration (e.g., intravascular injection, subcutaneous injection and the like).
Each of the aforementioned compositions may comprise any other active ingredient that does not produce an unwanted interaction when formulated with the above-described peptide, phosphatase, antibody and the like.

Moreover, used in the present invention one or more medicaments selected from a cofilin inhibitor, a PAK1 inhibitor, a LIMK inhibitor, a Rho inhibitor, a ROCK1 inhibitor and a ROCK2 inhibitor can be screened for by the following screening method. A preferable embodiment of the screening method of the present invention is a method including evaluating the cytoskeleton signal transduction inhibitory activity of a test compound and selecting a compound having a cytoskeleton signal transduction inhibitory activity. The cytoskeleton signal transduction inhibitory activity means an activity that can inhibit signal transduction via a protein involved in cytoskeleton signal transduction by expression inhibitory, activation inhibitory, destabilization and the like of the protein involved in the cytoskeleton signal transduction. Therefore, the cytoskeleton signal transduction inhibitory activity can be evaluated based on the inhibitory activity against cytoskeleton signal transduction. The thus-selected compound suppresses the activity of cofilin, which is a downstream molecule, and becomes a candidate compound as an agent for the treatment or prophylaxis of a trastuzumab-resistant cancer.

The expression inhibitory activity, activation inhibitory activity, destabilization activity and the like of a protein involved in the cytoskeleton signal transduction can be measured according to a known method. Specifically, for example, the cytoskeleton signal transduction inhibitory activity can be evaluated by measuring the PAK1 inhibitory activity, Rho inhibitory activity, ROCK inhibitory activity, LIMK inhibitory activity and the like. Here, the PAK1 inhibitory activity, Rho inhibitory activity, ROCK inhibitory activity, LIMK inhibitory activity or cofilin inhibitory activity means an activity that can inhibit signal transduction via PAK1 protein, Rho protein, ROCK protein, LIMK protein or cofilin protein by expression inhibition, activation inhibition, inactivation and the like of PAK1 protein, Rho protein, ROCK protein or LIMK protein.

The PAK inhibitory activity can be measured, for example, by modifying the measurement experiment of LIMK activation by PAK1 described in Nat Cell Biol. (1999) 1, 253-259, performing the experiment using a test substance, and considering the activation inhibitory rate. The Rho inhibitory activity can be measured, for example, by the method described in Journal of Neurochemistry (2002) 81, 9-16. The ROCK inhibitory activity can be measured, for example, by the method described in Biochem. J. (2000) 351, 95-105. The LIMK inhibitory activity can be measured, for example, by modifying the measurement experiment of kinase activity of LIMK described in Nature (1998) 393, 809-812 or Biochem. J. (1999) 343, 99-105, performing the experiment using a test substance, and considering the activation inhibitory rate.

As examples of test compounds, peptides, proteins, antibodies, non-peptide compounds, synthetic compounds, fermentation products, cell extracts, plant extracts, animal tissue extracts, plasma and the like can be mentioned; these compounds may be novel or publicly known. The test compound may form a salt, and as a salt of the test compound, a physiologically acceptable metal salt, an ammonium salt, a salt with an organic base, a salt with an inorganic acid, a salt with an organic acid, a salt with a basic or acidic amino acid and the like can be mentioned. Preferable examples of the metal salt include alkali metal salts such as sodium salt, potassium salt and the like; alkaline earth metal salts such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic base include salts with hydrochloric acid, hydrobromic acid, nitric acid, sulfuric acid, phosphoric acid and the like. Preferable examples of the salt with organic acid include salts with formic acid, acetic acid, trifluoroacetic acid, propionic acid, phthalic acid, fumaric acid, oxalic acid, tartaric acid, maleic acid, citric acid, succinic acid, malic acid, methanesulfonic acid, benzoic acid, benzenesulfonic acid, p-toluenesulfonic acid and the like. Preferable examples of the salt with basic amino acid include salts with arginine, lysine, ornithine and the like, and preferable examples of the salt with acidic amino acid include salts with aspartic acid, glutamic acid and the like.

More specifically, for example, provided are
(1) a method of screening for a cofilin inhibitor, a PAK inhibitor, a LIMK inhibitor, a Rho inhibitor, a ROCK1 inhibitor, or a ROCK2 inhibitor, comprising comparing the cytoskeleton signal transduction activity of a cell between
   (i) when the test compound is contacted with the cell, and
   (ii) when the test compound is not contacted with the cell,
(2) a method of screening for a cofilin inhibitor, a PAK inhibitor, a LIMK inhibitor, a Rho inhibitor, a ROCK1 inhibitor, or a ROCK2 inhibitor, comprising comparing the expression levels of a gene encoding a protein involved in the cytoskeleton signal transduction such as PAK1 protein, Rho protein, ROCK protein, LIMK protein, cofilin protein and the like of a cell between (i) when the test compound is contacted with the cell, and (ii) when the test compound is not contacted with the cell,
(3) a method of screening for a cofilin inhibitor, a PAK inhibitor, a LIMK inhibitor, a Rho inhibitor, a ROCK1 inhibitor, or a ROCK2 inhibitor, comprising comparing the activity of a protein involved in the cytoskeleton signal transduction such as PAK1 protein, Rho protein, ROCK protein, LIMK protein, cofilin protein and the like between (i) in the presence of the test compound, and (ii) in the absence of the test compound,
(4) a method of screening for a cofilin inhibitor, a PAK inhibitor, a LIMK inhibitor, a Rho inhibitor, a ROCK1 inhibitor, or a ROCK2 inhibitor, comprising comparing the stability of a protein involved in the cytoskeleton signal transduction such as PAK1 protein, Rho protein, ROCK protein, LIMK protein, cofilin protein and the like between (i) in the presence of the test compound, and (ii) in the absence of the test compound.

(1) Method of screening for a cofilin inhibitor, a PAK inhibitor, a LIMK inhibitor, a Rho inhibitor, a ROCK1 inhibitor, or a ROCK2 inhibitor, comprising comparing the cytoskeleton signal transduction activity of a cell between
   (i) when the test compound is contacted with the cell, and
   (ii) when the test compound is not contacted with the cell
   In this method, a test compound is first contacted with the cell expressing a protein involved in the cytoskeleton signal transduction. The derivation of the "cells" to be used may be, but is not limited to, cells derived from human, mouse, cat, dog, bovine, sheep, bird and the like. As the "cell expressing a protein involved in the cytoskeleton signal transduction", a cell expressing a protein involved in the cytoskeleton signal transduction, or a cell into which an exogenous gene encoding a protein involved in the cytoskeleton signal transduction has been introduced, and in which the gene is expressed can be utilized. The cell in which an exogenous gene encoding a protein involved in the cytoskeleton signal transduction is expressed can be generally produced by introducing, into a host cell, an expression vector inserted with a gene encoding a protein involved in the cytoskeleton signal transduction. The expression vector can be produced by a general genetic engineering technique.

Next, the cytoskeleton signal transduction activity (e.g., phosphorylation activity) is measured. Specifically, for example, in (i) and (ii), the above-mentioned cell is cultured, and the cytoskeleton signal transduction activity thereof is measured. The cytoskeleton signal transduction activity can be measured by a known method, for example, by measuring the phosphorylation of Rac, PAK1, Rho, ROCK1, ROCK2 or LIMK, Western blotting or ELISA using a phosphorylation antibody that specifically reacts with those proteins, or phosphorylation of cofilin downstream thereof by western blotting or ELISA using a phosphorylation antibody that specifically reacts with those proteins and the like. As the test compound, those similar to the aforementioned can be used.

Then, a compound that suppresses (decreases) cytoskeleton signal transduction as compared to the absence of contact with the test compound (control) is selected. For example, a test compound that suppresses the cytoskeleton signal transduction activity in the above-mentioned (i) by about 20% or above, preferably 30% or above, more preferably about 50% or above, as compared to that in the above-mentioned (ii) can be selected as a compound that suppresses (decreases) cytoskeleton signal transduction. The thus-selected compound suppresses the activity of cofilin, which is a downstream molecule, and becomes a candidate compound as an agent for the treatment or prophylaxis of cancer.

(2) A method of screening for a cofilin inhibitor, a PAK inhibitor, a LIMK inhibitor, a Rho inhibitor, a ROCK1 inhibitor, or a ROCK2 inhibitor, comprising comparing the expression levels of a gene encoding a protein involved in the cytoskeleton signal transduction such as PAK1 protein, Rho protein, ROCK protein, LIMK protein, cofilin protein and the like of a cell between (i) when the test compound is contacted with the cell, and (ii) when the test compound is not contacted with the cell
   In this method, in the same manner as above, a test compound is first contacted with a cell that expresses a gene encoding a protein involved in the cytoskeleton signal transduction. As the cell, a cell that expresses a gene encoding a protein involved in the cytoskeleton signal transduction, such as PAK1 protein, Rho protein, ROCK protein, LIMK protein, cofilin protein and the like, is preferably used, for example, human vulva cancer cell A431, human large intestine cancer cells HCT-15, SW620, human breast cancer cells BT-474, SK-Br-3, MCF-7, MDA-MB-231, human non-small cell lung cancer cell A549, human ovary cancer cell SK-OV-3 and the like.

Next, the expression of a gene encoding a protein involved in the cytoskeleton signal transduction is measured. Specifically, for example, in (i) and (ii), the above-mentioned cell is cultured, and the expression level of a gene encoding a protein involved in the cytoskeleton signal transduction is measured. The expression level of a gene can be measured by measuring the transcription level or translation level and the like by a known method. For example, the transcription level of the gene can be measured by extracting mRNA from a cell that expresses a gene encoding a protein involved in the cytoskeleton signal transduction by a conventional method, and performing Northern hybridization or RT-PCR using the mRNA as a template. Alternatively, a promoter region of a gene encoding a protein involved in the cytoskeleton signal transduction is isolated by a conventional method, a marker gene (e.g., gene detectable with luminescence, fluorescence, color development and the like of luciferase, GFP, galactosidase and the like as indices can be nonlimitatively mentioned) is ligated downstream thereof, and the activity of the marker gene is observed, whereby the transcription level of the gene can be measured. In addition, a protein fraction is recovered from a cell that expresses a gene encoding a protein involved in the cytoskeleton signal transduction, and the expression of a protein involved in cytoskeleton signal transduction is detected by electrophoresis such as SDS-PAGE and the like, whereby the translation level of the gene can also be measured. Furthermore, it is possible to measure the translation level of a gene by performing Western blotting using an antibody against a protein involved in the MAPK signal transduction and detecting the protein expression. The antibody to be used for the detection of a protein involved in the cytoskeleton signal transduction is not particularly limited as long as it can be detected, and, for example, both a monoclonal antibody and a polyclonal antibody can be used. As the test compound, those similar to the above are used.

Then, a compound that suppresses (decreases) the expression level of a gene encoding a protein involved in the cytoskeleton signal transduction as compared to the absence of contact with a test compound (control) is selected. The thus-selected compound suppresses the activity of cofilin, which is a downstream molecule, and becomes a candidate compound as an agent for the treatment or prophylaxis of cancer.

(3) Method of screening for a cofilin inhibitor, a PAK inhibitor, a LIMK inhibitor, a Rho inhibitor, a ROCK1 inhibitor, or a ROCK2 inhibitor, comprising comparing the activity of a protein involved in the cytoskeleton signal transduction such as PAK1 protein, Rho protein, ROCK protein, LIMK protein, cofilin protein and the like between (i) in the presence of the test compound, and (ii) in the absence of the test compound
   For example, in (i) and (ii) above, the activity of a protein involved in the cytoskeleton signal transduction such as PAK1 protein, Rho protein, ROCK protein, LIMK protein, cofilin protein and the like is measured. Then, a compound that suppresses (decreases) the activity of a gene encoding a protein involved in the cytoskeleton signal transduction as compared to the absence of a test compound (control) is selected. The activity of a protein involved in the cytoskeleton signal transduction can be measured by a known method, for example, by measuring the phosphorylation of Rac, PAK1, Rho, ROCK1, ROCK2 or LIMK, Western blotting or ELISA using a phosphorylation antibody that specifically reacts with those proteins, or phosphorylation of cofilin downstream thereof by Western blotting or ELISA using a phosphorylation antibody that specifically reacts with those proteins and the like. As the test compound, those similar to the aforementioned can be used. The thus-selected compound suppresses the activity of cofilin, which is a downstream molecule, and becomes a candidate compound as an agent for the treatment or prophylaxis of cancer.

(4) Method of screening for a cofilin inhibitor, a PAK inhibitor, a LIMK inhibitor, a Rho inhibitor, a ROCK1 inhibitor, or a ROCK2 inhibitor, comprising comparing the stability of a protein involved in the cytoskeleton signal transduction such as PAK1 protein, Rho protein, ROCK protein, LIMK protein, cofilin protein and the like between (i) in the presence of the test compound, and (ii) in the absence of the test compound.
   In (i) and (ii) above, the stability of a protein involved in the cytoskeleton signal transduction is measured. The stability of a protein involved in the cytoskeleton signal transduction can be measured by a known method, for example, the method described in Cancer Res. 65: 596-604 (2005) (³⁵S-labelled pulse chase method) and the like. Then, a compound that suppresses (decreases) the stability of a protein involved in the cytoskeleton signal transduction as compared to the absence of a test compound (control) is selected. The thus-selected compound suppresses the activity of cofilin, which is a downstream molecule, and becomes a candidate compound as an agent for the treatment or prophylaxis of cancer.

The compound selected by the above-mentioned methods is preferably screened for using suppression of the phosphorylation level of cofilin protein, which is a downstream molecule, as an index. For example, PAK1 inhibitor, LIMK inhibitor, Rho inhibitor, ROCK1 inhibitor or ROCK2 inhibitor is preferably screened for based on the comparison of the phosphorylation of the cofilin protein in the cell between (i) with a cell treatment with a test compound and (ii) without a cell treatment with a test compound.

Specifically, for example, in the above-mentioned (i) and (ii), the above-mentioned cells are cultured, and the phosphorylation level of the cofilin protein thereof is measured. The phosphorylation level of the cofilin protein can be measured by a known method such as Westernblot or ELISA/EIA and the like. For example, the above-mentioned cells are cultured and lysed, and Westernblot or ELISA/EIA using an antibody against phosphorylated cofilin is performed to detect the phosphorylation level of the protein, whereby the suppressive activity on the kinase activity can be measured. The antibody to be used for the detection of phosphorylated cofilin is not particularly limited as long as it can be detected, and, for example, both a monoclonal antibody and a polyclonal antibody can be used. As the test compound, one selected by the above-mentioned method is used.

Then, a compound that suppresses (decreases) the phosphorylation level of cofilin protein as compared to the absence of contact with a test compound (control) is selected. The thus-selected compound suppresses the activity of cofilin, which is a downstream molecule, and becomes a candidate compound as an agent for the treatment or prophylaxis of cancer.

The cofilin, PAK1, LIMK, Rho, ROCK1 or ROCK2 inhibitor obtained using the above-mentioned screening methods can be formulated into a preparation as an agent for preventing or treating cancer by a conventional method such as blending, kneading, granulation, tabletting, coating, sterilization treatment, emulsification and the like according to the preparation form. For production of the preparation, for example, each section of the Japanese Pharmacopoeia
Preparation General Rules and the like can be referred to. In addition, the agent for preventing or treating cancer of the present invention may be formed into a sustained-release preparation containing the active ingredient and a biodegradable polymer compound. The sustained-release preparation can be formulated by the method described in JP-A-9-263545.
While the content of one or more medicaments selected from a cofilin inhibitor, a PAK1 inhibitor, a LIMK inhibitor, a Rho inhibitor, a ROCK1 inhibitor and a ROCK2 inhibitor in the agent for preventing or treating cancer of the present invention varies depending on the preparation form, it is generally about 0.01 - 100 wt%, preferably about 0.1 - 50 wt%, more preferably about 0.5 - 20 wt%, relative to the whole preparation.
When one or more medicaments selected from a cofilin inhibitor, a PAK1 inhibitor, a LIMK inhibitor, a Rho inhibitor, a ROCK1 inhibitor and a ROCK2 inhibitor are used as an agent for preventing or treating cancer, they can be administered orally or parenterally as they are, or in the form of a solid agent such as powder, fine granules, granules, tablet, capsule and the like or a liquid such as injection and the like by mixing with an appropriate pharmaceutically acceptable carrier, such as excipient (e.g., starch, lactose, sucrose, calcium carbonate, calcium phosphate and the like), binder (e.g., starch, gum arabic, carboxymethylcellulose, hydroxypropylcellulose, crystalline cellulose, alginic acid, gelatin, polyvinylpyrrolidone and the like), lubricant (for example, stearic acid, magnesium stearate, calcium stearate, talc and the like), disintegrant (e.g., calcium carboxymethylcellulose, talc and the like), diluent (e.g., water for injection, saline and the like), additive (stabilizer, preservative, colorant, flavor, dissolution aid, emulsifier, buffer agent, isotonicity agent and the like) as necessary and the like by a conventional method.
The content of the carrier in the agent for preventing or treating cancer of the present invention is generally about 0 - 99.9 wt%, preferably about 10 - 99.9 wt%, more preferably about 10 - 90 wt%, relative to the whole preparation.
While the dose varies depending on the kind of one or more medicaments selected from a cofilin inhibitor, a PAK1 inhibitor, a LIMK inhibitor, a Rho inhibitor, a ROCK1 inhibitor and a ROCK2 inhibitor, level of symptoms, age, sex, body weight and difference in the sensitivity of the subject of administration, timing of administration, administration route, administration intervals, properties, dosage form and kind of a formulated pharmaceutical composition, and the like, for oral administration to an adult patient with breast cancer, for example, it is about 0.005 - 100 mg, preferably about 0.05 - 50 mg, more preferably about 0.2 - 30 mg, of the substance of the present invention per kg body weight per day in one to 3 portions.
When the agent for preventing or treating cancer of the present invention is a sustained-release preparation, the dose varies depending on the kind and content of one or more medicaments selected from a cofilin inhibitor, a PAK1 inhibitor, a LIMK inhibitor, a Rho inhibitor, a ROCK1 inhibitor and a ROCK2 inhibitor, dosage form, duration of drug release, animal as subject of administration (e.g., mammals such as human, rat, mouse, cat, dog, rabbit, bovine, swine and the like), and administration object. When applied, for example, by parenteral administration, about 0.1 mg to about 100 mg of one or more medicaments selected from a cofilin inhibitor, a PAK1 inhibitor, a LIMK inhibitor, a Rho inhibitor, a ROCK1 inhibitor and a ROCK2 inhibitor only needs to be released from the administered preparation in one week.

Since activation of cofilin is considered to provide cancer cells with resistance to trastuzumab, conventional HER2 inhibitors can be effectively used for the prophylaxis or treatment of a trastuzumab-resistant cancer by using a direct or indirect cofilin inhibitor (e.g., cofilin inhibitor, PAK1 inhibitor, LIMK inhibitor, Rho inhibitor, ROCK1 inhibitor or ROCK2 inhibitor) together with a conventional HER2 inhibitor.
Examples of the conventional HER2 inhibitor include anti-HER2 antibody (e.g., trastuzumab (herceptin (trademark))), Lapatinib (GW572016) and the like.

As other conventional HER2 inhibitor, a compound represented by the formula (I):

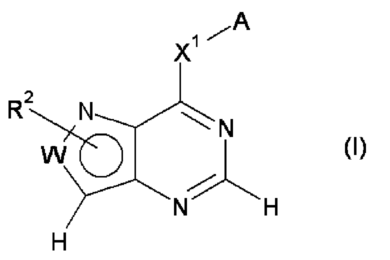

wherein W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or a hetero atom on the aryl group or the heteroaryl group represented by A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and R² is a hydrogen atom or an optionally substituted group bonded via a carbon atom or a sulfur atom, or
R¹ and R², or R² and R³ are optionally bonded to form an optionally substituted ring structure, provided that the compounds represented by the formulas

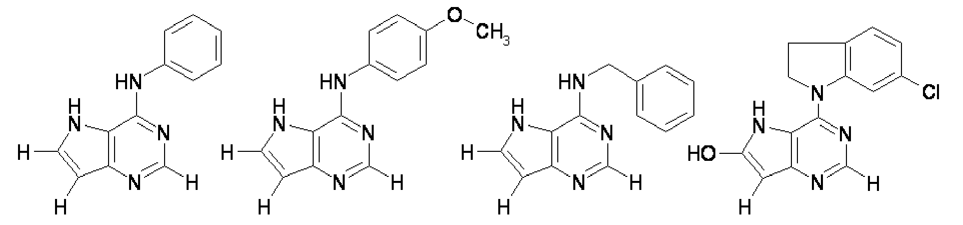

are excluded, or a salt thereof is used, and more preferably, a compound represented by the formula (Ia):

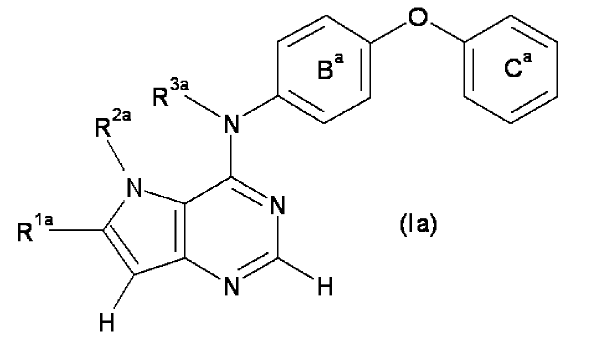

wherein R^{1a} is a hydrogen atom, or an optionally substituted group bonded via a carbon atom, a nitrogen atom or an oxygen atom,
R^{2a} is a hydrogen atom, or an optionally substituted group bonded via a carbon atom or a sulfur atom, or
R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or R^{3a} is optionally bonded via a carbon atom of the adjacent phenyl group to form an optionally substituted ring structure,
B^{a} is an optionally substituted benzene ring, and C^{a} is an optionally substituted C₆₋₁₈ aryl group, or a salt thereof is used.

As a still other conventional HER2 inhibitor, a compound represented by the formula (I'):

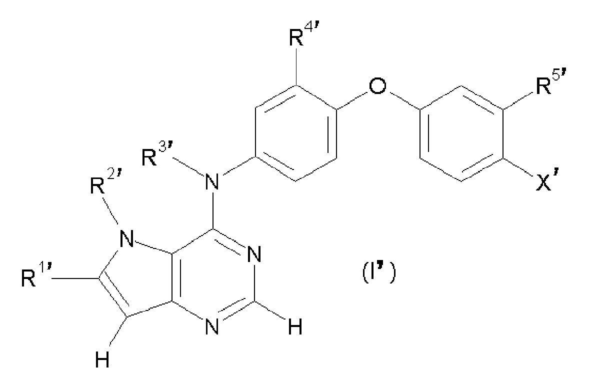

wherein
R^{1'} is a hydrogen atom,
R^{2'} is a C₁₋₆ alkyl group substituted by a group represented by -NR^{6'}-CO-(CH₂)n-SO₂- (optionally halogenated C₁₋₄ alkyl) wherein n is an integer of 1 to 4, R^{6'} is a hydrogen atom or a C₁₋₄ alkyl group, and -(CH₂)ₙ- is optionally substituted by C₁₋₄ alkyl,
R^{3'} is a hydrogen atom or a C₁₋₆ alkyl group,
R^{4'} is a halogen atom or a C₁₋₆ alkyl group,
R^{5'} is a halogen atom or a C₁₋₆ alkyl group, or
X' is a hydrogen atom or a halogen atom,
or a salt thereof, excluding N-[2-(4-{[3-chloro-4-(3-chlorophenoxy)phenyl]aminol-5H-pyrrolo[3,2-d]pyrimidin-5-yl)ethyl]-2-(methylsulfonyl)acetamide, is used.

As a yet other conventional HER2 inhibitor, a compound represented by the formula (I"):

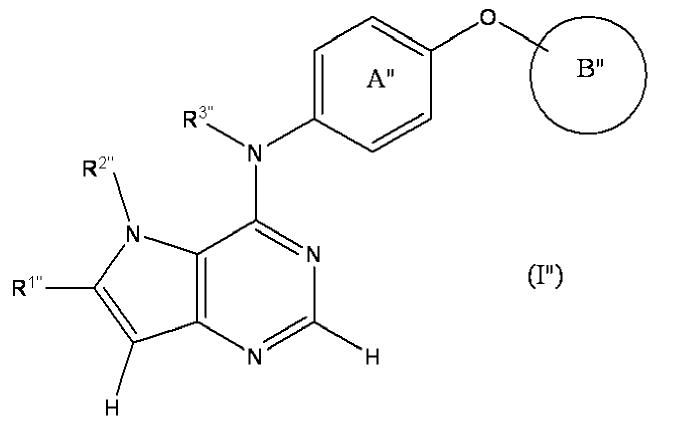

wherein
R^{1"} is a hydrogen atom, or an optionally substituted group bonded via a carbon atom, a nitrogen atom or an oxygen atom, R^{2"} is a hydrogen atom, or an optionally substituted group bonded via a carbon atom or a sulfur atom,
or, R^{1"} and R^{2"}, or R^{2"} and R^{3"} are optionally bonded to each other to form an optionally substituted ring structure;
R^{3"} is a hydrogen atom or an optionally substituted aliphatic hydrocarbon group,
or R^{3"} is optionally bonded to a carbon atom of ring A" to form an optionally substituted ring structure;
ring A" is an optionally substituted benzene ring;
ring B" is
(i) an optionally substituted fused ring, or
(ii) a pyridine ring having optionally substituted carbamoyl wherein the pyridine ring is optionally further substituted;
or a salt thereof is used.

The compounds of the formula (I) and the formula (Ia) (hereinafter sometimes to be abbreviated as compound (I) and compound (Ia), respectively, or simply referred to as compound (I)) are explained in the following.

In compound (I), unless otherwise specified, the "aryl" in the "aryl group" and the substituents includes a monocyclic aryl group and a fused polycyclic aryl group. As the "aryl group", for example, a C₆₋₁₈ aryl group can be mentioned. As the "C₆₋₁₈ aryl group", for example, phenyl, biphenylyl, naphthyl, anthryl, phenanthryl and acenaphthylenyl can be mentioned.

In compound (I), as the "heterocyclic group" (and "heterocyclyl-" in the substituents), for example, a 5- to 8-membered heteroaryl group or a 5- to 8-membered saturated or unsaturated aliphatic heterocyclic group containing, as an atom constituting a ring system (ring atom), one or more (preferably 1 to 4, more preferably 1 or 2) hetero atoms selected from an oxygen atom, an optionally oxidized sulfur atom and a nitrogen atom and the like (preferably, an oxygen atom, a sulfur atom and a nitrogen atom etc.) can be mentioned.

In compound (I), unless otherwise specified, as the "aliphatic hydrocarbon group", a straight chain or branched aliphatic hydrocarbon group having 1 to 15 carbon atoms (preferably, 1 to 8 carbon atoms) can be mentioned. As such "aliphatic hydrocarbon group", for example, a C₁₋₈ alkyl group, a C₂₋₈ alkenyl group, a C₂₋₈ alkynyl group, a C₃₋₈ cycloalkyl group and the like can be mentioned.

In compound (I), unless otherwise specified, as the "heteroaryl group", an monocyclic aromatic heterocyclic group (e.g., 5- or 6-membered monocyclic aromatic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like) and an aromatic fused heterocyclic group (e.g., 8- to 12-membered aromatic fused heterocyclic group such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like) and the like can be mentioned. As the aromatic fused heterocyclic group, a heterocycle wherein the aforementioned 5- or 6-membered monocyclic aromatic heterocyclic group is fused with a benzene ring and a heterocycle wherein the same or different two heterocycles of the aforementioned 5- or 6-membered monocyclic aromatic heterocyclic group are fused are preferable.

In compound (I), unless otherwise specified, as the "aliphatic heterocyclic group", for example, a 3- to 8-membered (preferably 5- or 6-membered) saturated or unsaturated (preferably saturated) aliphatic heterocyclic group such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like, and the like can be mentioned.

In compound (I), unless otherwise specified, as the "C₁₋₈ alkyl group", for example, methyl, ethyl, n-propyl, i-propyl, n-butyl, i-butyl, s-butyl, t-butyl, n-pentyl, i-pentyl, t-pentyl, neopentyl, n-hexyl, i-hexyl, n-heptyl and n-octyl and the like can be mentioned, with preference given to a C₁₋₆ alkyl group. In compound (I), moreover, unless otherwise specified, as the "C₁₋₄ alkyl group", for example, methyl, ethyl, n-propyl, i-propyl, n-butyl and i-butyl can be mentioned.

In compound (I), unless otherwise specified, as the "C₂₋₈ alkenyl group", for example, vinyl, (1- or 2-)propenyl, (1-, 2- or 3-)butenyl, pentenyl, octenyl and (1,3-)butadienyl can be mentioned, with preference given to a C₂₋₄ alkenyl group.

In compound (I), unless otherwise specified, as the "C₂₋₈ alkynyl group", for example, ethynyl, (1- or 2-)propynyl, (1-, 2- or 3-)butynyl, pentynyl and octynyl can be mentioned, with preference given to a C₂₋₄ alkynyl group.

In compound (I), unless otherwise specified, as the "C₃₋₈ cycloalkyl group", for example, cyclopropyl, cyclobutyl, cyclopentyl, cyclohexyl, cycloheptyl and cyclooctyl can be mentioned, with preference given to a C₃₋₆ cycloalkyl group.

In compound (I), unless otherwise specified, as the "C₁₋₄ alkylene", for example, methylene, ethylene, trimethylene, tetramethylene and propylene and the like can be mentioned.

In compound (I), unless otherwise specified, as the "-O-(C₁₋₄ alkylene)-", for example, -OCH₂-, -OCH₂CH₂-, -O(CH₂)₃-, -O(CH₂)₄-, -OCH(CH₃)-, -OC(CH₃)₂-, -OCH(CH₃)CH₂-, -OCH₂CH(CH₃) -, -OC(CH₃)₂CH₂- and -OCH₂C(CH₃)₂- and the like can be mentioned.

In compound (I), unless otherwise specified, as the "C₆₋₁₈ aryl-carbonyl group", for example, benzoyl, naphthoyl, anthrylcarbonyl, phenanthrylcarbonyl and acenaphthylenylcarbonyl and the like can be mentioned.

In compound (I), unless otherwise specified, as the "C₆₋₁₈ aryl-C₁₋₄ alkyl-carbonyl group", for example, benzylcarbonyl, 3-phenylpropionyl, 2-phenylpropionyl, 4-phenylbutyryl and 5-phenylpentanoyl and the like can be mentioned.

In compound (I), unless otherwise specified, as the "halogen", fluorine, chlorine, bromine and iodine can be mentioned.

As the "5- to 8-membered heterocyclyl-carbonyl group containing 1 to 3 hetero atoms selected from a nitrogen atom, an oxygen atom and a sulfur atom", "a 5- to 8-membered cyclic amino-carbonyl group optionally having 1 or 2 hetero atoms selected from a nitrogen atom, an oxygen atom and a sulfur atom" is preferable, for example, pyrrolidin-1-ylcarbonyl, piperidin-1-ylcarbonyl, piperazin-1-ylcarbonyl, morpholin-4-ylcarbonyl, thiomorpholin-4-ylcarbonyl and the like can be mentioned.

In the above-mentioned formula, as the "aryl group" for A, a C₆₋₁₈ aryl group is preferable, and phenyl is more preferable.

The "aryl group" for A is optionally substituted by a group represented by the formula -Y²-B wherein Y² is a single bond, -O-, -O-(C₁₋₃ alkylene)- (preferably -OCH₂-), -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted.

As Y², a single bond, -O- or -OCH₂- is preferable, and -O- or -OCH₂- is more preferable.

As the "aryl group" for B, a C₆₋₁₈ aryl group is preferable, and phenyl is more preferable.

As the "heterocyclic group" for B, the aforementioned "5-or 6-membered monocyclic aromatic heterocyclic group" is preferable, and pyridyl is more preferable.

The "aryl group", "heterocyclic group", "C₆₋₁₈ aryl-carbonyl group" or "C₆₋₁₈ aryl-C₁₋₄ alkyl-carbonyl group" for B may have, for example, 1 to 5, the same or different substituents selected from halogen, optionally halogenated C₁₋₄ alkyl, hydroxy, optionally halogenated C₁₋₄ alkyloxy, C₁₋₄ alkyloxymethyl, hydroxyl-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino, at any substitutable position(s).

The "aryl group" for A may further have, besides the above-mentioned group represented by the formula -Y²-B, 1 to 5, the same or different substituents at substitutable optional position(s). As such substituent, substituents similar to those exemplified for the "aryl group" or "heterocyclic group" for B can be mentioned.

As the "aliphatic hydrocarbon group" for R³, a C₁₋₈ alkyl group, a C₂₋₈ alkenyl group, a C₂₋₈ alkynyl group and a C₃₋₈ cycloalkyl group are preferable.

The "aliphatic hydrocarbon group" for R³ is optionally substituted by 1 to 3 substituents selected from halogen, hydroxy, C₁₋₄ alkyloxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.

The "C₁₋₄ alkylene" and "-O- (C₁₋₄ alkylene)-" for Y¹ are optionally substituted by 1 to 3 substituents selected from halogen, hydroxy, C₁₋₄ alkyloxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.

As X¹, -NR³- wherein R³ is as defined above is preferable.

As the "optionally substituted group bonded via a carbon atom, a nitrogen atom or an oxygen atom" for R¹, a group of the formula -X²-R⁴ can be mentioned, wherein X² is a single bond, -NH- or -O-, and R⁴ is a hydrogen atom, a cyano group, or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted.

The "C₁₋₈ alkyl group", "C₂₋₈ alkenyl group", "C₂₋₈ alkynyl group", "C₁₋₈ alkyl-carbonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "heterocyclic group", "heterocyclyl-C₁₋₄ alkyl group", "heterocyclyl-carbonyl group" and "heterocyclyl-C₁₋₄ alkyl-carbonyl group" are, for example, optionally substituted by one or more (preferably 1 to 5, more preferably 1 to 3) substituent(s) selected from the group consisting of
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom)
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl (hereinafter to be sometimes to be referred to as substituent group T).

In these formulas,
m is an integer of 0 to 4,
n is an integer of 1 to 4,
Q is hydroxy, carboxy, cyano, nitro, -NR⁶R⁷, -CONR⁶R⁷ or - SO₂NR⁶R⁷,
Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-_{,} -NR⁸-CO-NH-, -NR⁸-SO₂-_{,} or -NR⁸-C(=NH)-NH-, and
Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-_{,} -NR⁸-CO-NH-, -NR⁸-C(=NH)-NH-, -NR⁸-SO₂-, or -SO₂-NR⁸-. In these formulas, (CH₂)ₘ and (CH₂)ₙ are optionally substituted by one or more (preferably 1 to 5, more preferably 1 to 3) substituents selected from, for example, halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH- or -C≡C-.

In these formulas, R⁶ and R⁷ are the same or different and each is a hydrogen atom or C₁₋₄ alkyl, or R⁶ and R⁷ form a ring together with a nitrogen atom. In these formulas, moreover, R⁸ is a hydrogen atom or C₁₋₄ alkyl and R⁹ is C₁₋₄ alkyl. When R⁶ and R⁷ form a ring together with a nitrogen atom, as the nitrogen-containing heterocyclic group, for example, a 3- to 8-membered (preferably 5- or 6-membered) saturated or unsaturated (preferably saturated) aliphatic heterocyclic group such as azetidinyl, pyrrolidinyl, piperidinyl, homopiperidinyl, heptamethyleneimino, morpholinyl, thiomorpholinyl, piperazinyl, homopiperazinyl and the like, and the like can be mentioned.

As X², a single bond is preferable.

As R⁴, a hydrogen atom or a C₁₋₈ alkyl group, a C₂₋₈ alkenyl group, a C₆₋₁₈ aryl group or heterocyclic group, each of which is optionally substituted is preferable. As the "C₆₋₁₈ aryl group" for R⁴, phenyl is preferable. As the "heterocyclic group" for R⁴, the aforementioned "5- or 6-membered monocyclic aromatic heterocyclic group" is preferable, and furyl is more preferable.

As the "optionally substituted group bonded via a carbon atom or a sulfur atom" for R², a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted, can be mentioned.

The "C₁₋₈ alkyl group", "C₂₋₈ alkenyl group", "C₂₋₈ alkynyl group", "C₁₋₈ alkyl-carbonyl group", "C₁₋₈ alkyl-sulfonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "C₆₋₁₈ aryl-sulfonyl group", "heterocyclic group", "heterocyclyl-C₁₋₄ alkyl group", "heterocyclyl-carbonyl group" and "heterocyclyl-C₁₋₄ alkyl-carbonyl group" are optionally substituted by, for example, one or more (preferably 1 to 5, more preferably 1 to 3) substituents selected from the above-mentioned substituent group T.

As R², a hydrogen atom or a C₁₋₈ alkyl group, a C₆₋₁₈ aryl group, a C₆₋₁₈ aryl-C₁₋₄ alkyl group, a C₆₋₁₈ aryl-carbonyl group, a C₆₋₁₈ aryl-sulfonyl group or heterocyclyl-C₁₋₄ alkyl group, each of which is optionally substituted, is preferable.

As the "C₆₋₁₈ aryl group" for R², phenyl is preferable. As the "C₆₋₁₈ aryl-C₁₋₄ alkyl group" for R², benzyl is preferable.
As the "C₆₋₁₈ aryl-carbonyl group" for R², benzoyl is preferable. As the "C₆₋₁₈ aryl-sulfonyl group" for R², phenylsulfonyl is preferable. As the "heterocyclic group" or "heterocyclyl-" of "heterocyclyl-C₁₋₄ alkyl group", "heterocyclyl-carbonyl group" and "heterocyclyl-C₁₋₄ alkyl-carbonyl group" for R², the aforementioned "5- or 6-membered monocyclic aromatic heterocyclic group" or the aforementioned "aliphatic heterocyclic group" is preferable, and furyl or tetrahydrofuryl is more preferable.

In the substituents that a group represented by R² may have, when R⁶ and R⁷ form a ring together with a nitrogen atom, the "ring" optionally further has 1 to 5 (preferably 1 to 3) the same or different substituents. As such substituents, substituents similar to those exemplified for "aryl group" or "heterocyclic group" for B can be mentioned.

The aforementioned "carbamoyl group" and "ureido group" optionally have 1 or 2 optionally substituted C₁₋₈ alkyl group (s). Alternatively, the "carbamoyl group" and "ureido group" may have two substituents and they may form an optionally substituted ring, together with the adjacent nitrogen atom. As the "ring" of the "optionally substituted ring", rings similar to those formed by R⁶ and R⁷ together with a nitrogen atom as exemplified above can be mentioned. As the "substituent" of the "optionally substituted C₁₋₈ alkyl group" and as the "substituent" of the "optionally substituted ring", groups similar to the substituents of the above-mentioned substituent group T can be mentioned.

As the "optionally substituted carbamoyl group", carbamoyl, C₁₋₈ alkylcarbamoyl, di(C₁₋₈ alkyl) carbamoyl, C₆₋₁₈ aryl-C₁₋₄ alkylcarbamoyl, azetidin-1-ylcarbonyl, pyrrolidin-1-ylcarbonyl, piperidin-1-ylcarbonyl, piperazin-1-ylcarbonyl, morpholin-4-ylcarbonyl, thiomorpholin-4-ylcarbonyl, (C₁₋₄ alkyl)piperidin-1-ylcarbonyl, (C₆₋₁₈ aryl-C₁₋₄ alkyl)piperidin-1-ylcarbonyl and the like can be mentioned.

As the "optionally substituted ureido group", ureido, 3-(C₁₋₈ alkyl)ureido, 3,3-di(C₁₋₈ alkyl)ureido, 3-(C₆₋₁₈ aryl-C₁₋₄ alkyl)ureido, azetidin-1-ylcarbonylamino, pyrrolidin-1-ylcarbonylamino, piperidin-1-ylcarbonylamino, piperazin-1-ylcarbonylamino, morpholin-4-ylcarbonylamino, thiomorpholin-4-ylcarbonylamino, (C₁₋₄ alkyl)piperidin-1-ylcarbonylamino, (C₆₋₁₈ aryl-C₁₋₄ alkyl)piperidin-1-ylcarbonylamino and the like can be mentioned.

As the "ring structure" of the optionally substituted ring structure formed by R³ bonded to a carbon atom or a hetero atom on the aryl group or the heteroaryl group represented by A, a saturated or unsaturated (preferably saturated) 4- to 8-membered (preferably 5- or 6-membered) nitrogen-containing heterocycle can be mentioned. Specifically,

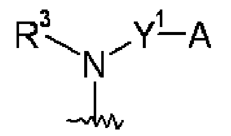

is

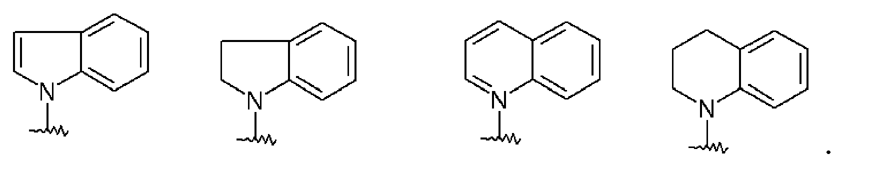

The "ring structure" may have 1 to 5 (preferably 1 to 3, more preferably 1 or 2), the same or different substituents at any substitutable position(s). As such substituents, substituents similar to those exemplified for "aryl group" or "heterocyclic group" for B can be mentioned.

As the "ring structure" of the optionally substituted ring structure formed by R¹ and R² bonded to each other, a saturated or unsaturated (preferably saturated) 4- to 8-membered (preferably 5- or 6-membered) heterocycle can be mentioned. When R¹ and R² are bonded to form an optionally substituted ring structure, for example,

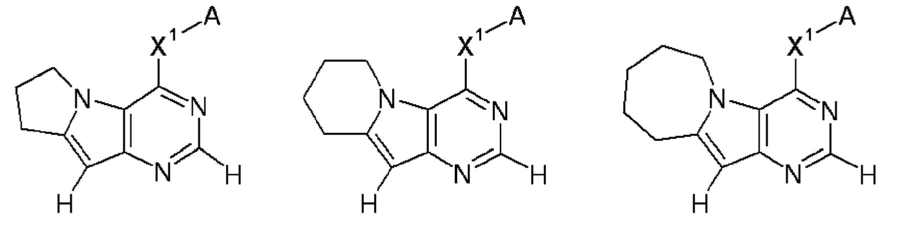

wherein each symbol is as defined above, and the like can be mentioned.

As the "ring structure" of the optionally substituted ring structure formed by R² and R³ bonded to each other, a saturated or unsaturated (preferably saturated) 4- to 8-membered (preferably 5- to 7-membered) heterocycle can be mentioned. When R² and R³ are bonded to form an optionally substituted ring structure, for example,

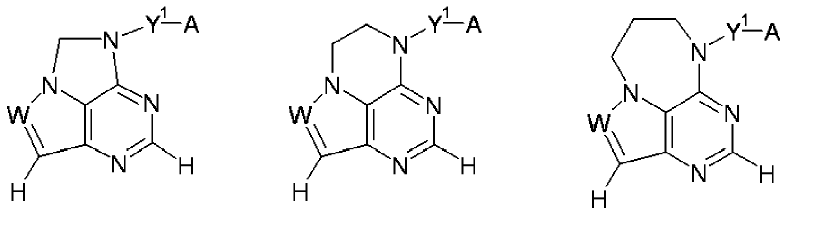

wherein each symbol is as defined above, and the like can be mentioned. The "ring structure" formed by R¹ and R², or R² and R³ bonded to each other may have 1 to 5 (preferably 1 to 3, more preferably 1 or 2), the same or different substituents selected from the above-mentioned substituent group T at any substitutable position(s).

When W is C(R¹), compound (I) is represented by the following formula (IA):

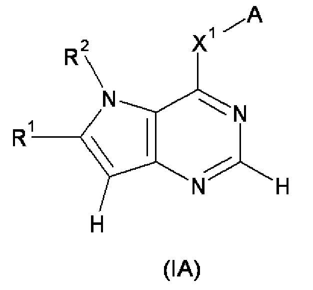

wherein each symbol is as defined above.

When W is N, compound (I) is represented by the following formula (IB) or (IC):

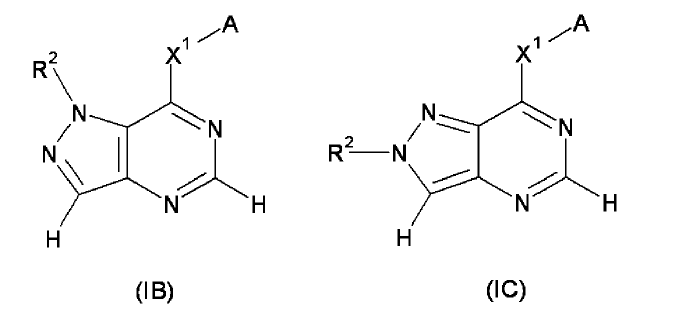

wherein each symbol is as defined above.

Specifically, as compound (I), the following compound (Ia) is specifically preferably used.

### [Compound (Ia)]

A compound represented by the formula:

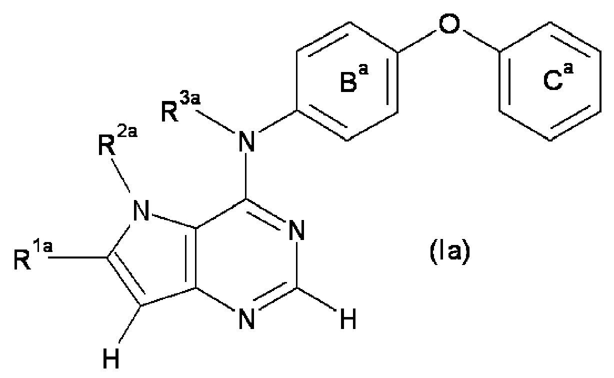

wherein R^{1a} is a hydrogen atom or an optionally substituted group bonded via a carbon atom, a nitrogen atom or an oxygen atom,
R^{2a} is an optionally substituted group bonded via a carbon atom or a sulfur atom, or
R^{1a} and R^{2a}, or R^{2a} and R^{3a} are optionally bonded to form an optionally substituted ring structure,
R^{3a} is a hydrogen atom or an optionally substituted aliphatic hydrocarbon group, or
R^{3a} is optionally bonded to a carbon atom of the adjacent phenyl group to form an optionally substituted ring structure, B^{a} is an optionally substituted benzene ring, and
C^{a} is an optionally substituted C₆₋₁₈ aryl group, or a salt thereof.

As the "optionally substituted group bonded via a carbon atom, a nitrogen atom or an oxygen atom" for R^{1a}, those similar to the "optionally substituted group bonded via a carbon atom, a nitrogen atom or an oxygen atom" for R¹ can be used.

As the "optionally substituted group bonded via a carbon atom or a sulfur atom" for R^{2a}, those similar to the "optionally substituted group bonded via a carbon atom or a sulfur atom" for R² can be used.

As the "optionally substituted ring structure" formed by R^{1a} and R^{2a}, or R^{2a} and R^{3a} bonded to each other, those similar to the "optionally substituted ring structure" formed by R¹ and R², or R² and R³ bonded to each other can be used.

As the "an optionally substituted aliphatic hydrocarbon group" for R^{3a}, those similar to the "optionally substituted aliphatic hydrocarbon group" for R³ can be used.

As the "optionally substituted ring structure" formed by R^{3a} bonded to a carbon atom of the adjacent phenyl group, "optionally substituted ring structure" formed by R³ bonded to a carbon atom of the adjacent phenyl group can be used.

As the substituent of the "optionally substituted benzene ring" for B^{a}, for example, 1 to 5, the same or different substituents selected from halogen, optionally halogenated C₁₋₄ alkyl, hydroxy, optionally halogenated C₁₋₄ alkyloxy, C₁₋₄ alkyloxymethyl, hydroxyl-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino are used.

As the "C₆₋₁₈ aryl group" of the "optionally substituted C₆₋₁₈ aryl group" for C^{a}, for example, phenyl, biphenylyl, naphthyl, anthryl, phenanthryl, acenaphthylenyl and the like are used. Of these, a phenyl group is preferable.

As the "substituent" of the "optionally substituted C₆₋₁₈ aryl group" for C^{a}, those similar to the "optionally substituted benzene ring" for B^{a} can be used.

As R^{2a}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each optionally substituted by 1 to 5 substituents selected from the group consisting of
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl
wherein m is an integer of 0 to 4,
n is an integer of 1 to 4,
Q is hydroxy, carboxy, cyano, nitro, -NR⁶R⁷, -CONR⁶R⁷, - OCONH₂ or -SO₂NR⁶R⁷,
Z¹ is -O-, -CO-, -C(OH)R⁸-, -C(=N-OR⁸)-, -S-, -SO-, -SO₂-, - N(COR⁸)-, -N(CO₂R⁹)-, -N(SO₂R⁹)-, -CO-O-, -O-CO-, -CO-NR⁸-, - NR⁸-CO-, -NR⁸-CO₂-_{,} -NR⁸-CO-NH-, -NR⁸-SO₂- or -NR⁸-C(=NH)-NH-, Z² is -O-, -CO-, -C(OH)R⁸-, -C(=N-OR⁸)-, -S-, -SO-, -SO₂-, - NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-_{,} -NR⁸-CO-NH-, -NR⁸-C(=NH)-NH-, -NR⁸-SO₂- or -SO₂-NR⁸-,
(CH₂)ₘ and (CH₂)ₙ is optionally substituted by 1 to 5 substituents selected from halogen, optionally halogenated C₁₋₄ alkyl and hydroxy, when m or n is not less than 2, a subset -CH₂CH₂- of (CH₂)ₘ and (CH₂)ₙ may be replaced by - CH=CH- or -C≡C-,
R⁶ and R⁷ are the same or different and each is a hydrogen atom or a C₁₋₄ alkyl group, or R⁶ and R⁷ are bond to form, together with a nitrogen atom, a 3- to 8-membered saturated or unsaturated aliphatic heterocyclic group,
R⁸ is a hydrogen atom or C₁₋₄ alkyl and
R⁹ is C₁₋₄ alkyl,
is preferable.

As compound (Ia), a compound wherein
B^{a} is a benzene ring optionally substituted by 1 to 4 substituents selected from halogen, C₁₋₄ alkyl, hydroxy-C₁₋₄ alkyl and C₁₋₄ alkyloxy;
C^{a} is a phenyl group optionally substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, and includes imidazolyl, triazolyl and the like), (v) optionally halogenated C₁₋₄ alkyloxy, (vi) C₁₋₄ alkyl-carbonyl, (vii) cyano, (viii) carbamoyl optionally substituted by C₁₋₈ alkyl and (ix) C₁₋₄ alkoxy-carbonyl;
R^{1a} is
(i) a hydrogen atom,
(ii) a cyano group, or
(iii) a C₁₋₄ alkyl group or a C₂₋₄ alkenyl group, each of which is optionally substituted by -NR⁸-CO-(CH₂)ₙ-NR⁶R⁷
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂)ₙ is optionally replaced by -CH=CH-;
R^{2a} is a C₁₋₈ alkyl group, a C₂₋₈ alkenyl group or a C₂₋₈ alkynyl group, each of which is optionally substituted by substituent(s) selected from
(a) hydroxy,
(b) carboxy,
(c) cyano,
(d) optionally halogenated C₁₋₄ alkyloxy,
(e) -O-(CH₂)ₙ-OH,
(f) -O-(CH₂)ₙ-O-CO-NH₂,
(g) -O-(CH₂)ₙ-O- (optionally halogenated C₁₋₄ alkyl),
(h) -O-(CH₂)ₙ-SO₂- (optionally halogenated C₁₋₄ alkyl),
(i) -O-(CH₂)ₙ-SO₂-C₆₋₁₈ aryl,
(j) -O-(CH₂)ₙ-SO₂-(CH₂)ₙ-OH,
(k) -O-(CH₂)ₙ-NR⁸-CO-C₁₋₄ alkyl,
(1) -O-(CH₂)ₙ-NR⁸-CO-(CH₂)ₙ-SO₂-C₁₋₄ alkyl,
(m) -O-(CH₂)ₙ-NR⁸-SO₂-(optionally halogenated C₁₋₄ alkyl),
(n) -CO-NR⁸-(CH₂)ₙ-OH,
(o) -CO-NR⁸-(CH₂)ₙ-SO₂-(optionally halogenated C₁₋₄ alkyl),
(p) -CO-NR⁸-O-C₁₋₄ alkyl,
(q) -NR⁶R⁷,
(r) -NR⁸-(CH₂)ₙ-OH,
(s) -NR⁸-(CH₂)ₙ-SO₂-C₁₋₄ alkyl,
(t) -NR⁸-CO-(optionally halogenated C₁₋₄ alkyl),
(u) -NR⁸-CO-(CH₂)ₙ-OH,
(V) -NR⁸-CO-(CH₂)ₙ-CN,
(w) -NR⁸-CO-(CH₂)ₙ-NR⁶R⁷,
(x) -NR⁸-CO-(CH₂)ₙ-O-C₁₋₄ alkyl,
(y) -NR⁸-CO-(CH₂)ₙ-SO-(optionally halogenated C₁₋₄ alkyl),
(z) -NR⁸-CO-(CH₂)ₙ-SO₂-(optionally halogenated C₁₋₄ alkyl),
(aa) -NR⁸-CO-(CH₂)ₙ-SO₂-C₃₋₈ cycloalkyl,
(bb) -NR⁸-CO-(CH₂)ₙ-NR⁸-SO₂-C₁₋₄ alkyl,
(cc) -NR⁸-CO₂-(CH₂)ₙ-SO₂-C₁₋₄ alkyl,
(dd) -NR⁸-CO-NH-(CH₂)ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C(=NH)-NH-C₁₋₄ alkyl,
(hh) -NR⁸-SO₂-(CH₂)ₙ-SO₂-C₁₋₄ alkyl,
(ii) -S-(CH₂)ₙ-OH,
(jj) -SO- (CH₂)ₙ-OH,
(kk) -SO₂-(CH₂)ₙ-OH, and
(11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group,
   R⁸ is a hydrogen atom or a C₁₋₄ alkyl group, (CH₂) is optionally substituted by optionally halogenated C₁₋₄ alkyl or hydroxy, and when n is not less than 2, a subset -CH₂CH₂- of (CH₂)ₙ is optionally replaced by -CH=CH-; and
   R^{3a} is a hydrogen atom or a C₁₋₆ alkyl group; or
   R^{1a} and R^{2a} are optionally bonded to form

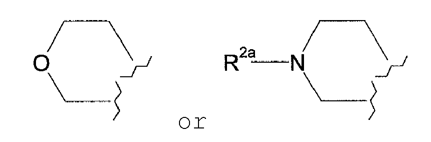

R^{2a} and R^{3a} are optionally bonded to form C₂₋₄ alkylene optionally substituted by an imino group, is preferable.
As R⁸, a hydrogen atom, methyl, ethyl and the like are preferable, and a hydrogen atom is particularly preferable.
Among those, as R^{2a}, a C₁₋₈ alkyl group, a C₂₋₈ alkenyl group or a C₂₋₈ alkynyl group, each of which is optionally substituted by substituent(s) selected from
(a) hydroxy,
(b) carboxy,
(c) cyano,
(d) optionally halogenated C₁₋₄ alkyloxy,
(e) -O-(CH₂)ₙ-OH (wherein (CH₂)ₙ is optionally substituted by hydroxy),
(f) -O-(CH₂)ₙ-O-CO-NH₂,
(g) -O-(CH₂)ₙ-O-(optionally halogenated C₁₋₄ alkyl),
(h) -O-(CH₂)ₙ-SO₂-(optionally halogenated C₁₋₄ alkyl),
(i) -O-(CH₂)ₙ-SO₂-C₆₋₁₈ aryl,
(j) -O-(CH₂)ₙ-SO₂-(CH₂)ₙ-OH,
(k) -O-(CH₂)ₙ-NR⁸-CO-C₁₋₄ alkyl,
(1) -O-CH₂)ₙ-NR⁸-CO-(CH₂)ₙ-SO₂-C₁₋₄ alkyl,
(m) -O-(CH₂)ₙ-NR⁸-SO₂-(optionally halogenated C₁₋₄ alkyl),
(n) -CO-NR⁸-(CH₂)ₙ-OH,
(o) -CO-NR⁸-(CH₂)ₙ-SO₂-(optionally halogenated C₁₋₄ alkyl),
(p) -CO-NR⁸-O-C₁₋₄ alkyl,
(q) -NR⁶R⁷,
(r) -NR⁸-(CH₂)ₙ-OH,
(s) -NR⁸-(CH₂)ₙ-SO₂-C₁₋₄ alkyl,
(t) -NR⁸-CO- (optionally halogenated C₁₋₄ alkyl),
(u) -NR⁸-CO-(CH₂)ₙ-OH (wherein (CH₂) is optionally substituted by optionally halogenated C₁₋₄ alkyl or hydroxy),
(v) -NR⁸-CO-(CH₂)ₙ-CN,
(w) -NR⁸-CO-(CH²)ₙ-NR⁶R⁷ (when n is not less than 2, a subset -CH₂CH₂- of (CH₂)ₙ is optionally replaced by -CH=CH-),
(x) -NR⁸-CO-(CH₂)ₙ-O-C₁₋₄ alkyl,
(y) -NR⁸-CO-(CH₂)ₙ-SO-(optionally halogenated C₁₋₄ alkyl),
(z) -NR⁸-CO-(CH₂)ₙ-SO₂-(optionally halogenated C₁₋₄ alkyl) (wherein (CH₂)ₙ is optionally substituted by C₁₋₄ alkyl),
(aa) -NR⁸-CO-(CH₂)ₙ-SO₂-C₃₋₈ cycloalkyl,
(bb) -NR⁸-CO-(CH₂)ₙ-NR⁸-SO₂-C₁₋₄ alkyl,
(cc) -NR⁸-CO₂-(CH₂)ₙ-SO₂-C₁₋₄ alkyl,
(dd) -NR⁸-CO-NH-(CH₂)ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C(=NH)-NH-C₁₋₄ alkyl,
(hh) -NR⁸-SO₂-(CH₂)ₙ-SO₂-C₁₋₄ alkyl,
(ii) -S-(CH₂)ₙ-OH,
(jj) -SO-(CH₂)ₙ-OH,
(kk) -SO₂-(CH₂)ₙ-OH, and
(11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group, is preferable.
   As R⁸, a hydrogen atom, methyl, ethyl and the like are preferable, and a hydrogen atom is particularly preferable.

As compound (Ia), moreover, a compound wherein
B^{a} is a benzene ring optionally substituted by 1 to 4 substituents selected from halogen and optionally halogenated C₁₋₄ alkyl;
C^{a} is a phenyl group substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5-to 8-membered heterocycle has 1 to 3 hetero atoms selected from a nitrogen atom, an oxygen atom and an optionally oxidized sulfur atom, and includes imidazolyl and the like), (v) optionally halogenated C₁₋₄ alkyloxy, (vi) cyano, and (vii) carbamoyl optionally substituted by C₁₋₈ alkyl;
R^{1a} is a hydrogen atom;
R^{2a} is a C₁₋₈ alkyl group, a C₂₋₈ alkenyl group or a C₂₋₈ alkynyl group, each of which is substituted by substituent(s) selected from
(a) hydroxy,
(b) optionally halogenated C₁₋₄ alkyloxy,
(c) -O-(CH₂)ₙ-OH,
(d) -O-(CH₂)ₙ-O-CO-NH₂,
(e) -O-(CH₂)ₙ-O-C₁₋₄ alkyl,
(f) -O-(CH₂)ₙ-SO₂-(optionally halogenated C₁₋₄ alkyl),
(g) -O-(CH₂)ₙ-SO₂-C₆₋₁₈ aryl,
(h) -O-(CH₂)ₙ-SO₂-(CH₂)ₙ-OH,
(i) -O-(CH₂)ₙ-NR⁸-SO₂-(optionally halogenated C₁₋₄ alkyl),
(j) -CO-NR⁸-(CH₂)ₙ-OH,
(k) -CO-NR⁸-(CH₂)ₙ-SO₂-(optionally halogenated C₁₋₄ alkyl),
(l) -NR⁶R⁷,
(m) -NR⁸-(CH₂)ₙ-OH,
(n) -NR⁸-(CH₂)ₙ-SO₂-C₁₋₄ alkyl,
(o) -NR⁸-CO-(CH₂)ₙ-OH,
(p) -NR⁸-CO-(CH₂)ₙ-O-C₁₋₄ alkyl,
(q) -NR⁸-CO-(CH₂)ₙ-SO-(optionally halogenated C₁₋₄ alkyl),
(r) -NR⁸-CO-(CH₂)ₙ-SO₂-(optionally halogenated C₁₋₄ alkyl),
(s) -NR⁸-CO-(CH₂)ₙ-SO₂-C₃₋₈ cycloalkyl,
(t) -NR⁸-CO₂-(CH₂)ₙ-SO₂-C₁₋₄ alkyl,
(u) -NR⁸-CO-NH-(CH₂)ₙ-SO₂-C₁₋₄ alkyl,
(v) -NR⁸-SO₂-(CH₂)ₙ-SO₂-C₁₋₄ alkyl,
(w) -S-(CH₂)ₙ-OH,
(x) -SO-(CH₂)ₙ-OH,
(y) -SO₂-(CH₂)ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group,
   R⁸ is a hydrogen atom or a C₁₋₄ alkyl group, and (CH₂)ₙ is optionally substituted by C₁₋₄ alkyl or hydroxy;
R^{3a} is a hydrogen atom or a C₁₋₆ alkyl group; or
R^{1a} and R^{2a} are optionally bonded to form

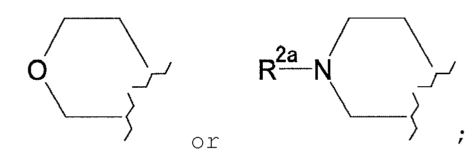

or

R^{2a} and R^{3a} are optionally bonded to form C₂₋₄ alkylene, is preferable.

Of these, as R^{2a}, a C₁₋₈ alkyl group, a C₂₋₈ alkenyl group or a C₂₋₈ alkynyl group (particularly, a C₁₋₈ alkyl group), each of which is substituted by substituent(s) selected from
(a) hydroxy,
(b) optionally halogenated C₁₋₄ alkyloxy,
(c) -O-(CH₂)ₙ-OH (wherein (CH₂) is optionally substituted by hydroxy),
(d) -O-(CH₂)ₙ-O-CO-NH₂,
(e) -O-(CH₂)-O-C₁₋₄ alkyl,
(f) -O-(CH₂)ₙ-SO₂-(optionally halogenated C₁₋₄ alkyl),
(g) -O-(CH₂)ₙ-SO₂-C₆₋₁₈ aryl,
(h) -O-(CH₂)ₙ-SO₂-(CH₂)ₙ-OH,
(i) -O-(CH₂)ₙ-NR⁸-SO₂-(optionally halogenated C₁₋₄ alkyl),
(j) -CO-NR⁸-(CH₂)ₙ-OH,
(k) -CO-NR⁸-(CH₂)ₙ-SO₂-(optionally halogenated C₁₋₄ alkyl),
(1) -NR⁶R⁷,
(m) -NR⁸-(CH₂)ₙ-OH,
(n) -NR⁸-(CH₂)ₙ-SO₂-C₁₋₄ alkyl,
(o) -NR⁸-CO-(CH₂)ₙ-OH (wherein (CH₂)ₙ is optionally substituted by C₁₋₄ alkyl),
(p) -NR⁸-CO-(CH₂)ₙ-O-C₁₋₄ alkyl,
(q) -NR⁸-CO-(CH₂)ₙ-SO-(optionally halogenated C₁₋₄ alkyl),
(r) -NR⁸-CO-(CH₂)ₙ-SO₂-(optionally halogenated C₁₋₄ alkyl) (wherein (CH₂)ₙ is optionally substituted by C₁₋₄ alkyl),
(s) -NR⁸-CO-(CH₂)ₙ-SO₂-C₃₋₈ cycloalkyl,
(t) -NR⁸-CO₂-(CH₂)ₙ-SO₂-C₁₋₄ alkyl,
(u) -NR⁸-CO-NH-(CH₂)ₙ-SO₂-C₁₋₄ alkyl,
(v) -NR⁸-SO₂-(CH₂)ₙ-SO₂-C₁₋₄ alkyl,
(w) -S-(CH₂)ₙ-OH,
(x) -SO-(CH₂)ₙ-OH,
(y) -SO₂-(CH₂)ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
wherein n is an integer of 1 to 4, R⁶ and R⁷ are the same or different and each is a hydrogen atom or a C₁₋₄ alkyl group, R⁸ is a hydrogen atom or a C₁₋₄ alkyl group, is preferable.

As R^{2a},
(i) a C₅₋₈ alkyl group substituted by hydroxy,
(ii) a C₁₋₈ alkyl group substituted by substituent(s) selected from
   (a) halogenated C₁₋₄ alkyloxy,
   (b) -O-(CH₂)ₙ-OH,
   (c) -O-(CH₂)ₙ-O-CO-NH₂,
   (d) -O-(CH₂)ₙ-O-(optionally halogenated C₁₋₄ alkyl),
   (e) -O-(CH₂)ₙ-SO₂- (optionally halogenated C₁₋₄ alkyl),
   (f) -O-(CH₂)ₙ-SO₂-C₆₋₁₈ aryl,
   (g) -O-(CH₂)ₙ-NR⁸-SO₂-(optionally halogenated C₁₋₄ alkyl),
   (h) -CO-NR⁸-(CH₂)ₙ-OH,
   (i) -CO-NR⁸-(CH₂)ₙ-SO₂-(optionally halogenated C₁₋₄ alkyl),
   (j) -NR⁸-(CH₂)ₙ-SO₂-C₁₋₄ alkyl,
   (k) -NR⁸-CO-(CH₂)ₙ-OH,
   (l) -NR⁸-CO-(CH₂)ₙ-O-C₁₋₄ alkyl,
   (m) -NR⁸-CO-(CH₂)ₙ-SO-(optionally halogenated C₁₋₄ alkyl),
   (n) -NR⁸-CO-(CH₂)ₙ-SO₂-(optionally halogenated C₁₋₄ alkyl),
   (o) -NR⁸-CO-(CH₂)ₙ-SO₂-C₃₋₈ cycloalkyl,
   (p) -NR⁸-CO₂-(CH₂)ₙ-SO₂-C₁₋₄ alkyl,
   (q) -NR⁸-CO-NH-(CH₂)ₙ-SO₂-C₁₋₄ alkyl,
   (r) -NR⁸-SO₂-(CH₂)ₙ-SO₂-C₁₋₄ alkyl,
   (s) -S-(CH₂₎ₙ-OH,
   (t) -SO-(CH₂)ₙ-OH,
   (u) -SO₂-(CH₂)ₙ-OH, and
   (v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, R⁸ is a hydrogen atom or a C₁₋₄ alkyl group, and (CH₂)ₙ is optionally substituted by C₁₋₄ alkyl or hydroxy,
(iii) a C₂₋₈ alkenyl group optionally substituted by hydroxy, or
(iv) a C₂₋₈ alkynyl group optionally substituted by hydroxy is preferable, and particularly,
as R^{2a},
(i) a C₅₋₈ alkyl group substituted by hydroxy,
(ii) a C₁₋₈ alkyl group substituted by substituent(s) selected from
   (a) halogenated C₁₋₄ alkyloxy,
   (b) -O-(CH₂)ₙ-OH (wherein (CH₂)ₙ is optionally substituted by hydroxy),
   (c) -O-(CH₂)ₙ-O-CO-NH₂,
   (d) -O-(CH₂)ₙ-O-(optionally halogenated C₁₋₄ alkyl),
   (e) -O-(CH₂)ₙ-SO₂-(optionally halogenated C₁₋₄ alkyl),
   (f) -O-(CH₂)ₙ-SO₂-C₆₋₁₈ aryl,
   (g) -O-(CH₂)ₙ-NR⁸-SO₂-(optionally halogenated C₁₋₄ alkyl),
   (h) -CO-NR⁸-(CH₂)ₙ-OH,
   (i) -CO-NR⁸-(CH₂)ₙ-SO₂-(optionally halogenated C₁₋₄ alkyl),
   (j) -NR⁸-(CH₂)ₙ-SO₂-C₁₋₄ alkyl,
   (k) -NR⁸-CO-(CH₂)ₙ-OH (wherein (CH₂)ₙ is optionally substituted by C₁₋₄ alkyl),
   (l) -NR⁸-CO-(CH₂)ₙ-O-C₁₋₄ alkyl,
   (m) -NR⁸-CO-(CH₂)ₙ-SO-(optionally halogenated C₁₋₄ alkyl),
   (n) -NR⁸-CO-(CH₂)ₙ-SO₂-(optionally halogenated C₁₋₄ alkyl) (wherein (CH₂)ₙ is optionally substituted by C₁₋₄ alkyl),
   (o) -NR⁸-CO-(CH₂)ₙ-SO₂-C₃₋₈ cycloalkyl,
   (p) -NR⁸-CO₂-(CH₂)ₙ-SO₂-C₁₋₄ alkyl,
   (q) -NR⁸-CO-NH-(CH₂)ₙ-SO₂-C₁₋₄ alkyl,
   (r) -NR⁸-SO₂-(CH₂)ₙ-SO₂-C₁₋₄ alkyl,
   (s) -S-(CH₂)ₙ-OH,
   (t) -SO-(CH₂)ₙ-OH,
   (u) -SO₂-(CH₂)ₙ-OH, and
   (v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, and R⁸ is a hydrogen atom or a C₁₋₄ alkyl group,
(iii) a C₂₋₈ alkenyl group optionally substituted by hydroxy, or (iv) a C₂₋₈ alkynyl group optionally substituted by hydroxy, is preferable.

As R⁸, a hydrogen atom, methyl, ethyl and the like are preferable, and a hydrogen atom is particularly preferable.

Compound (I) is preferably a compound wherein A is an aryl group substituted by a group represented by the formula - Y²-B
wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted,
which aryl group is optionally further substituted.

A preferable embodiment of compound (I) is a compound
wherein W is C(R¹);
A is an aryl group substituted by a group represented by the formula -Y²-B
wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted,
which aryl group is optionally further substituted;
R¹ is a group represented by the formula -X²-R⁴ wherein X² is a single bond, -NH- or -O-, and R⁴ is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted;
R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkylsulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted; and
X¹ is -NR³- wherein R³ is a hydrogen atom or an optionally substituted aliphatic hydrocarbon group.

Another preferable embodiment of compound (I) is a compound wherein W is N;
X¹ is -NR³-
wherein R³ is a hydrogen atom or an optionally substituted aliphatic hydrocarbon group; and
A is an aryl group substituted by a group represented by the formula -Y²-B
wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted, which aryl group is optionally further substituted; and
R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkylsulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted.

A still another preferable embodiment of compound (I) is a compound wherein W is N;
X¹ is -NR³-;
A is an aryl group substituted by a group represented by the formula -Y²-B
wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each
of which is optionally substituted,
which aryl group is optionally further substituted; and
R² and R³ are bonded to form an optionally substituted ring structure.

As the salts of the compound represented by the formula (I), for example, metal salt, ammonium salt, salts with organic base, salts with inorganic acid, salts with organic acid, salts with basic or acidic amino acid and the like can be mentioned. As preferable examples of the metal salt, for example, alkali metal salts such as sodium salt, potassium salt and the like; alkaline earth metal salts such as calcium salt, magnesium salt, barium salt and the like; aluminum salt and the like can be mentioned. As preferable examples of the salts with organic base, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like can be mentioned. As preferable examples of salts with inorganic acid, for example, salts with hydrochloric acid, hydrobromic acid, nitric acid, sulfuric acid, phosphoric acid and the like can be mentioned. As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned. As preferable examples of the salts with basic amino acid, for example, salts with arginine, lysine, ornithine and the like can be mentioned, and as preferable examples of the salts with acidic amino acid, for example, salts with aspartic acid, glutamic acid and the like can be mentioned.

Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, p-toluenesulfonic acid and the like can be mentioned.

Each term used for the compound of the formula (I') (hereinafter sometimes to be abbreviated as compound (I')) is explained in the following.

In compound (I'), unless otherwise specified, the "halogen atom" (and "halogen" in substituents) is, for example, a fluorine atom, a chlorine atom, a bromine atom or an iodine atom.

In compound (I'), unless otherwise specified, the "alkyl group" is, for example, straight chain or branched alkyl group having a carbon number of 1 to 10 (e.g., 1 - 10, 1 - 8, 1 - 6, 2 - 6, 1 - 4), such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

In compound (I'), unless otherwise specified, the "C₁₋₁₀ alkyl group is, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl or the like.

In compound (I'), unless otherwise specified, the "C₁₋₈ alkyl group" is, for example, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl or the like.

In compound (I'), unless otherwise specified, the "C₁₋₆ alkyl group" is, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.

In compound (I'), unless otherwise specified, the "C₂₋₆ alkyl group" is, for example, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.

In compound (I'), unless otherwise specified, the "C₁₋₄ alkyl group" is, for example, methyl, ethyl, propyl, isopropyl, butyl, isobutyl, sec-butyl, tert-butyl or the like.

In compound (I'), unless otherwise specified, the "alkenyl group" is, for example, an alkenyl group having a carbon number of 2 to 10 (e.g., 2 - 10, 2 - 8, 2 - 6, 2 - 4), such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

In compound (I'), unless otherwise specified, the "C₂₋₁₀ alkenyl group" is, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl or the like.

In compound (I'), unless otherwise specified, the "C₂₋₈ alkenyl group" is, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl or the like.

In compound (I'), unless otherwise specified, the "C₂₋₆ alkenyl group" is, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl or the like.

In compound (I'), unless otherwise specified, the "C₂₋₄ alkenyl group" is, for example, ethenyl, 1-propenyl, 2-propenyl, 2-methyl-1-propenyl, 1-butenyl, 2-butenyl, 3-butenyl or the like.

In compound (I'), unless otherwise specified, the "alkynyl group" is, for example, an alkynyl group having a carbon number of 2 to 10 (e.g., 2 - 10, 2 - 8, 2 - 6, 2 - 4), such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

In compound (I'), unless otherwise specified, the "C₂₋₁₀ alkynyl group" is, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl or the like.

In compound (I'), unless otherwise specified, the "C₂₋₈ alkynyl group" is, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl or the like.

In compound (I'), unless otherwise specified, the "C₂₋₆ alkynyl group" is, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl or the like.

In compound (I'), unless otherwise specified, the "C₂₋₄ alkynyl group" is, for example, ethynyl, 1-propynyl, 2-propynyl, 1-butynyl, 2-butynyl, 3-butynyl or the like.

In compound (I'), unless otherwise specified, the "cycloalkyl group" is, for example, a cycloalkyl group having a carbon number of 3 to 10 (e.g., 3 - 10, 3 - 8, 3 - 7, 3 - 6, 5 - 8), such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.

In compound (I'), unless otherwise specified, the "C₃₋₁₀ cycloalkyl group" is, for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl or the like.

In compound (I'), unless otherwise specified, the "C₃₋₈ cycloalkyl group" is, for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl or the like.

In compound (I'), unless otherwise specified, the "C₃₋₇ cycloalkyl group" is, for example, cyclopropyl, cyclobutyl, cyclopentyl, cyclohexyl, cycloheptyl or the like.

In compound (I'), unless otherwise specified, the "C₅₋₈ cycloalkyl group" is, for example, cyclopentyl, cyclohexyl, cycloheptyl, cyclooctyl or the like.

In compound (I'), unless otherwise specified, the "cycloalkenyl group" is, for example, a cycloalkenyl group having a carbon number of 3 to 10, such as 2-cyclopenten-1-yl, 3-cyclopenten-1-yl, 2-cyclohexen-1-yl, 3-cyclohexen-1-yl and the like.

In compound (I'), unless otherwise specified, the "C₃₋₁₀ cycloalkenyl group" is, for example, 2-cyclopenten-1-yl, 3-cyclopenten-1-yl, 2-cyclohexen-1-yl, 3-cyclohexen-1-yl or the like.

In compound (I'), unless otherwise specified, the "cycloalkadienyl group" is, for example, a cycloalkadienyl group having a carbon number of 4 to 10, such as 2,4-cyclopentadien-1-yl, 2,4-cyclohexadien-1-yl, 2,5-cyclohexadien-1-yl and the like.

In compound (I'), unless otherwise specified, the "C₄₋₁₀ cycloalkadienyl group" is, for example, 2,4-cyclopentadien-1-yl, 2,4-cyclohexadien-1-yl, 2,5-cyclohexadien-1-yl or the like.

In compound (I'), unless otherwise specified, the "aryl group" includes a monocyclic aryl group and a condensed polycyclic aryl group. The "aryl group" is, for example, an aryl group having a carbon number of 6 to 18 (e.g., 6 - 18, 6 - 14, 6 - 10), such as phenyl, naphthyl, anthryl, phenanthryl, acenaphthyl, biphenylyl and the like.

In compound (I'), unless otherwise specified, the "C₆₋₁₈ aryl group" is, for example, phenyl, naphthyl, anthryl, phenanthryl, acenaphthyl, biphenylyl or the like.

In compound (I'), unless otherwise specified, the "C₆₋₁₄ aryl group" is, for example, phenyl, naphthyl, anthryl, phenanthryl, acenaphthyl, biphenylyl or the like.

In compound (I'), unless otherwise specified, the "C₆₋₁₀ aryl group" is, for example, phenyl, naphthyl or the like.

In compound (I'), unless otherwise specified, the "aralkyl group" is, for example, an aralkyl group having a carbon number of 7 to 16, such as benzyl, phenethyl, phenylpropyl, naphthylmethyl, biphenylylmethyl and the like.

In compound (I'), unless otherwise specified, the "C₇₋₁₆ aralkyl group" is, for example, benzyl, phenethyl, phenylpropyl, naphthylmethyl, biphenylylmethyl or the like.

In compound (I'), unless otherwise specified, the "alkanoyl group" is, for example, an alkanoyl group having a carbon number of 1 to 7 (e.g., 1 - 7, 1 - 6), such as, formyl, C₁₋₆ alkyl-carbonyl (e.g., acetyl, propionyl, butyryl, valeryl, pivaloyl) and the like.

In compound (I'), unless otherwise specified, the "C₁₋₆ alkanoyl group" is, for example, formyl, C₁₋₆ alkyl-carbonyl (e.g., acetyl, propionyl, butyryl, valeryl, pivaloyl) or the like.

In compound (I'), unless otherwise specified, the "alkoxy group" is, for example, an alkoxy group having 1 to 6 carbon atoms (e.g., 1 - 6, 2 - 6, 1 - 4), such as methoxy, ethoxy, n-propoxy, isopropoxy, n-butoxy, tert-butoxy, n-pentyloxy, n-hexyloxy and the like.

In compound (I'), unless otherwise specified, the "C₁₋₆ alkoxy group" is, for example, methoxy, ethoxy, n-propoxy, isopropoxy, n-butoxy, tert-butoxy, n-pentyloxy, n-hexyloxy or the like.

In compound (I'), unless otherwise specified, the "C₂₋₆ alkoxy group" is, for example, ethoxy, n-propoxy, isopropoxy, n-butoxy, tert-butoxy, n-pentyloxy, n-hexyloxy or the like.

In compound (I'), unless otherwise specified, the "C₁₋₄ alkoxy group" is, for example, methoxy, ethoxy, n-propoxy, isopropoxy, n-butoxy, tert-butoxy or the like.

In compound (I'), unless otherwise specified, the "alkylene" is, for example, alkylene having a carbon number of 1 to 4 (e.g., 1 - 4, 1 - 3), such as -CH₂-, -CH₂CH₂-, -(CH₂)₃-, -(CH₂)₄-, -CH(CH₃)-, -C(CH₃)₂-, -CH(CH₃)CH₂-, -CH₂CH(CH₃)-, - C(CH₃)₂CH₂-, -CH₂C(CH₃)₂- and the like.

In compound (I'), unless otherwise specified, the "C₁₋₄ alkylene" is, for example, -CH₂-, -CH₂CH₂-, -(CH₂)₃-, -(CH₂)₄-, - CH(CH₃)-, -C(CH₃)₂-, -CH(CH₃)CH₂-, -CH₂CH(CH₃)-, -C(CH₃)₂CH₂-, - CH₂C(CH₃)₂- or the like.

In compound (I'), unless otherwise specified, the "C₁₋₃ alkylene" is, for example, -CH₂-, -CH₂CH₂-, -(CH₂)₃-, -(CH₂)₄-, - CH(CH₃)-, -C(CH₃)₂-, -CH(CH₃)CH₂-, -CH₂CH(CH₃)- or the like.

In compound (I'), unless otherwise specified, the "hydrocarbon group" of the "optionally substituted hydrocarbon group" is, for example, alkyl group, alkenyl group, alkynyl group, cycloalkyl group, cycloalkenyl group, cycloalkadienyl group, aryl group, aralkyl group, arylalkenyl group, cycloalkyl-alkyl group or the like, with preference given to C₁₋₁₀ alkyl group, C₂₋₁₀ alkenyl group, C₂₋₁₀ alkynyl group, C₃₋₁₀ cycloalkyl group, C₃₋₁₀ cycloalkenyl group, C₄₋₁₀ cycloalkadienyl group, C₆₋₁₄ aryl group, C₇₋₁₆ aralkyl group, C₈₋₁₃ arylalkenyl group, C₃₋₁₀ cycloalkyl-C₁₋₆ alkyl group and the like.

The above-mentioned C₃₋₁₀ cycloalkyl group, C₃₋₁₀ cycloalkenyl group and C₄₋₁₀ cycloalkadienyl group may be each condensed with a benzene ring. Examples of the fused ring group include indanyl, dihydronaphthyl, tetrahydronaphthyl, fluorenyl and the like. In addition, bridged hydrocarbon group such as norbornanyl, adamantyl and the like can also be mentioned as the above-mentioned hydrocarbon group.

Examples of the C₈₋₁₃ arylalkenyl group include styryl and the like.

Examples of the C₃₋₁₀ cycloalkyl-C₁₋₆ alkyl group include cyclopropylmethyl, cyclohexylmethyl and the like.

The C₁₋₁₀ alkyl group, C₂₋₁₀ alkenyl group and C₂₋₁₀ alkynyl group exemplified as the above-mentioned "hydrocarbon group" optionally have 1 to 3 substituents at substitutable positions.

Examples of the substituent include
(1) a C₃₋₁₀ cycloalkyl group (e.g., cyclopropyl, cyclohexyl) optionally substituted by 1 to 3 substituents selected from the group consisting of
   halogen;
   hydroxy;
   carboxyl;
   sulfo;
   cyano;
   azido;
   nitro;
   nitroso;
   optionally halogenated C₁₋₄ alkyl;
   optionally halogenated C₂₋₄ alkenyl;
   optionally halogenated C₂₋₄ alkynyl;
   C₃₋₇ cycloalkyl;
   C₆₋₁₄ aryl;
   C₇₋₁₆ aralkyl;
   formyl;
   optionally halogenated C₁₋₆ alkyl-carbonyl;
   optionally halogenated C₁₋₆ alkoxy-carbonyl;
   optionally halogenated C₁₋₆ alkylsulfonyl;
   carbamoyl;
   carbamoyl mono- or di-substituted by optionally halogenated C₁₋₆ alkyl;
   mono- or di-C₆₋₁₄ aryl-carbamoyl;
   thiocarbamoyl optionally mono- or di-substituted by optionally halogenated C₁₋₆ alkyl;
   ureido optionally mono- or di-substituted by optionally halogenated C₁₋₆ alkyl;
   mono- or di-C₆₋₁₄ aryl-ureido;
   sulfamoyl optionally mono- or di-substituted by optionally halogenated C₁₋₆ alkyl;
   optionally halogenated C₁₋₆ alkoxy;
   optionally halogenated C₂₋₆ alkenyloxy;
   C₃₋₁₀ cycloalkyloxy;
   C₇₋₁₆ aralkyloxy;
   C₆₋₁₄ aryloxy;
   C₁₋₆ alkyl-carbonyloxy;
   C₃₋₁₀ cycloalkyl-C₁₋₆ alkoxy;
   C₁₋₆ alkylsulfonyloxy;
   mercapto;
   optionally halogenated C₁₋₆ alkylthio;
   C₇₋₁₆ aralkylthio;
   C₆₋₁₄ arylthio;
   C₁₋₆ alkylsulfinyl;
   oxo;
   C₁₋₃ alkylenedioxy (e.g., methylenedioxy, ethylenedioxy); hydroxyimino optionally substituted by C₁₋₆ alkyl; and the like (substituent group S);
(2) a C₆₋₁₄ aryl group (e.g., phenyl, naphthyl) optionally substituted by 1 to 3 substituents selected from substituent group S;
(3) a heterocyclic group optionally substituted by 1 to 3 substituents selected from substituent group S;
(4) an amino group optionally substituted by 1 or 2 substituents selected from the group consisting of
   C₁₋₆ alkyl optionally substituted by substituent selected from the group consisting of halogen, hydroxy, C₃₋₇ cycloalkyl, C₁₋₆ alkylsulfonyl, C₁₋₆ alkoxy and the like;
   optionally halogenated C₂₋₄ alkenyl;
   optionally halogenated C₂₋₄ alkynyl;
   C₃₋₇ cycloalkyl;
   C₆₋₁₄ aryl;
   C₇₋₁₆ aralkyl;
   a 4- to 7-membered (preferably 5- or 6-membered) heterocyclic group (e.g., nonaromatic heterocyclic group such as morpholinyl and the like) containing, as a ring constitution atom besides carbon atom, 1 to 4 hetero atoms selected from the group consisting of oxygen atom, sulfur atom and nitrogen atom;
   formyl;
   C₁₋₆ alkyl-carbonyl optionally substituted by substituent selected from the group consisting of halogen, hydroxy, C₃₋₇ cycloalkyl, C₁₋₆ alkylsulfonyl, C₁₋₆ alkoxy and the like;
   C₁₋₆ alkoxy-carbonyl;
   C₆₋₁₄ aryl-carbonyl (e.g., benzoyl);
   C₇₋₁₆ aralkyl-carbonyl (e.g., benzylcarbonyl, phenethylcarbonyl);
   C₃₋₇ cycloalkyl-carbonyl;
   C₁₋₆ alkyl-carbamoyl (e.g., methylaminocarbonyl, ethylaminocarbonyl);
   C₆₋₁₄ aryl-carbamoyl (e.g., phenylaminocarbonyl, 1-naphthylaminocarbonyl, 2-naphthylaminocarbonyl);
   C₇₋₁₆ aralkyl-carbamoyl (e.g., benzylaminocarbonyl);
   C₁₋₆ alkylsulfonyl (e.g., methylsulfonyl, ethylsulfonyl, isopropylsulfonyl);
   C₆₋₁₄ arylsulfonyl (e.g., benzenesulfonyl, toluenesulfonyl, 1-naphthalenesulfonyl, 2-naphthalenesulfonyl);
   C₇₋₁₆ aralkylsulfonyl (e.g., benzylsulfonyl); and the like (substituent group T);
(5) amidino group;
(6) optionally formylated or halogenated C₁₋₆ alkyl-carbonyl group;
(7) optionally halogenated C₁₋₆ alkoxy-carbonyl group;
(8) optionally halogenated C₁₋₆ alkylsulfonyl group (e.g., methylsulfonyl);
(9) carbamoyl group optionally substituted by 1 or 2 substituents selected from substituent group T;
(10) thiocarbamoyl group optionally mono- or di-substituted by optionally halogenated C₁₋₆ alkyl group;
(11) ureido group optionally substituted by 1 or 2 substituents selected from substituent group T;
(12) sulfamoyl group optionally substituted by 1 or 2 substituents selected from substituent group T;
(13) carboxyl group;
(14) hydroxy group;
(15) C₁₋₆ alkoxy group optionally substituted by 1 to 3 substituents selected from the group consisting of halogen, carboxyl, C₁₋₆ alkoxy and C₁₋₆ alkoxy-carbonyl;
(16) optionally halogenated C₂₋₆ alkenyloxy group (e.g., ethenyloxy);
(17) C₃₋₁₀ cycloalkyloxy group (e.g., cyclohexyloxy);
(18) C₇₋₁₆ aralkyloxy group (e.g., benzyloxy);
(19) C₆₋₁₄ aryloxy group (e.g., phenyloxy, naphthyloxy);
(20) C₁₋₆ alkyl-carbonyloxy group (e.g., acetyloxy, tert-butylcarbonyloxy);
(21) mercapto group;
(22) optionally halogenated C₁₋₆ alkylthio group (e.g., methylthio, ethylthio);
(23) C₇₋₁₆ aralkylthio group (e.g., benzylthio);
(24) C₆₋₁₄ arylthio group (e.g., phenylthio, naphthylthio);
(25) sulfo group;
(26) cyano group;
(27) azido group;
(28) nitro group;
(29) nitroso group;
(30) halogen atom;
(31) C₁₋₆ alkylsulfinyl group (e.g., methylsulfinyl);
(32) oxo group;
(33) C₃₋₁₀ cycloalkyl-C₁₋₆ alkoxy group (e.g., cyclopropylmethoxy);
(34) C₁₋₃ alkylenedioxy group (e.g., methylenedioxy, ethylenedioxy);
(35) hydroxyimino group optionally substituted by C₁₋₆ alkyl; and the like (substituent group U). When the number of substituents is two or more, the respective substituents may be the same or different.

The C₃₋₁₀ cycloalkyl group, C₃₋₁₀ cycloalkenyl group, C₄₋₁₀ cycloalkadienyl group, C₆₋₁₄ aryl group, C₇₋₁₆ aralkyl group, C₈₋₁₃ arylalkenyl group and C₃₋₁₀ cycloalkyl-C₁₋₆ alkyl group exemplified as the above-mentioned "hydrocarbon group" optionally have 1 to 3 substituents at substitutable positions.

Examples of the substituent include
(1) substituent selected from substituent group U;
(2) C₁₋₁₀ alkyl group optionally substituted by 1 to 3 substituents selected from substituent group U;
(3) C₂₋₁₀ alkenyl group (e.g., ethenyl, 1-propenyl) optionally substituted by 1 to 3 substituents selected from substituent group U;
(4) C₇₋₁₆ aralkyl group (e.g., benzyl) optionally substituted by 1 to 3 substituents selected from substituent group U; and the like (substituent group V). When the number of substituents is two or more, the respective substituents may be the same or different.

In compound (I'), unless otherwise specified, examples of the "heterocyclic group" of the "optionally substituted heterocyclic group" include an aromatic heterocyclic group and a non-aromatic heterocyclic group.

Examples of the "aromatic heterocyclic group" include a 4- to 7-membered (preferably 5- or 6-membered) monocyclic aromatic heterocyclic group containing, as a ring-constituting atom besides carbon atoms, 1 to 4 heteroatoms selected from the group consisting of an oxygen atom, a sulfur atom and a nitrogen atom, and a fused aromatic heterocyclic group. Examples of the fused aromatic heterocyclic group include a group derived from a fused ring, wherein a ring corresponding to such 4- to 7-membered monocyclic aromatic heterocyclic group, and 1 or 2 rings selected from the group consisting of a 5- or 6-membered aromatic heterocycle containing 1 or 2 nitrogen atoms, a 5-membered aromatic heterocycle containing one sulfur atom and a benzene ring and the like are fused, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like; and the like.

Examples of the "non-aromatic heterocyclic group" include a 4- to 7-membered (preferably 5- or 6-membered) monocyclic non-aromatic heterocyclic group containing, as a ring-constituting atom besides carbon atoms, 1 to 4 heteroatoms selected from the group consisting of an oxygen atom, a sulfur atom and a nitrogen atom, and a fused non-aromatic heterocyclic group. Examples of the fused non-aromatic heterocyclic group include a group derived from a fused ring,
wherein a ring corresponding to such 4- to 7-membered monocyclic non-aromatic heterocyclic group, and 1 or 2 rings selected from the group consisting of a 5- or 6-membered heterocycle containing 1 or 2 nitrogen atoms, a 5-membered heterocycle containing one sulfur atom and a benzene ring and the like are fused, and the like.

Preferable examples of the non-aromatic heterocyclic group include monocyclic non-aromatic heterocyclic groups such as oxetanyl (e.g., 2-oxetanyl, 3-oxetanyl), pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like; fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like; and the like.

The "heterocyclic group" of the "optionally substituted heterocyclic group" may have 1 to 3 substituents at the substitutable positions. Examples of such substituent include substituents selected from the group consisting of substituent group V. When the number of the substituents is not less than 2, the respective substituents may be the same or different.

In compound (I'), unless otherwise specified, the "aliphatic hydrocarbon group" of the "optionally substituted aliphatic hydrocarbon group" is, for example, a straight chain or branched aliphatic hydrocarbon group having a carbon number of 1 - 10 (preferably, 1 - 8). Examples of the "aliphatic hydrocarbon group" include C₁₋₁₀ alkyl group, C₂₋₁₀ alkenyl group, C₂₋₁₀ alkynyl group and C₃₋₁₀ cycloalkyl group (each group is as defined above).

The "aliphatic hydrocarbon group" is optionally substituted by 1 to 3 substituents selected from substituent group V, particularly, halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkylsulfonylamino. When the substituents are two, the respective substituents may be the same or different.

In compound (I'), unless otherwise specified, the "acyl group" is, for example, -COR^{Y1}, -CO-OR^{Y1}, -SO₂R^{Y1}, -SOR^{Y1}, - PO(OR^{Y1}) (OR^{Y2}) [R^{Y1} and R^{Y2} are the same or different and each is hydrogen atom, optionally substituted hydrocarbon group, or optionally substituted heterocyclic group] or the like.

In compound (I'), unless otherwise specified, the "amino group" of the "optionally substituted amino group", the "carbamoyl group" of the "optionally substituted carbamoyl group", the "ureido group" of the "optionally substituted ureido group", and the "sulfamoyl group" of the "optionally substituted sulfamoyl group" optionally have 1 or 2 substituents at substitutable positions. Examples of the substituent include optionally substituted hydrocarbon group, optionally substituted heterocyclic group, acyl group and the like, with preference given to 1 or 2 substituents selected from the group consisting of substituent group T. When the substituents are two, the respective substituents may be the same or different.

When the nitrogen atom constituting the above-mentioned amino group, carbamoyl group, ureido group, or sulfamoyl group is substituted by two substituents, these substituents may form, together with the adjacent nitrogen atom, a nitrogen-containing heterocycle. Examples of the "nitrogen-containing heterocycle" include a 3- to 8-membered nitrogen-containing heterocycle containing, as a ring constituting atom besides carbon atom, at least one nitrogen atom, and further, 1 or 2 hetero atoms selected from the group consisting of oxygen atom, sulfur atom and nitrogen atom. Preferable examples of the nitrogen-containing heterocycle include 5- or 6-membered cyclic amine (e.g., 1-pyrrolidine, piperidine, 1-piperazine, morpholine) optionally containing an oxygen atom.

In compound (I'), unless otherwise specified, the "imino group" of the "optionally substituted imino group" optionally have 1 or 2 substituents at substitutable positions. Examples of the substituent include optionally substituted hydrocarbon group, optionally substituted heterocyclic group, acyl group and the like, with preference given to the substituents of substituent group T. When the substituents are two, the respective substituents may be the same or different.

In compound (I'), unless otherwise specified, the "optionally substituted group bonded via a carbon atom, nitrogen atom or oxygen atom" is, for example, a group represented by the formula -X^{x}-R^{x}, amino group or hydroxy group.

In the above-mentioned formula, X^{x} is a bond, -NR^{Y}- (R^{Y} is a hydrogen atom or a C₁₋₆ alkyl group), or -O-.

In the above-mentioned the formula, R^{x} is cyano group, or C₁₋₈ alkyl group, C₂₋₈ alkenyl group, C₂₋₈ alkynyl group, carbamoyl group, C₁₋₈ alkyl-carbonyl group, C₃₋₈ cycloalkyl group, C₆₋₁₈ aryl group, C₆₋₁₈ aryl-C₁₋₄ alkyl group, C₆₋₁₈ aryl-carbonyl group, C₆₋₁₈ aryl-C₁₋₄ alkyl-carbonyl group, heterocyclic group, heterocyclyl-C₁₋₄ alkyl group, heterocyclylcarbonyl group or heterocyclyl-C₁₋₄ alkyl-carbonyl group, each of which is optionally substituted.

In the above-mentioned the formula, the "C₁₋₈ alkyl group", "C₂₋₈ alkenyl group", "C₂₋₈ alkynyl group", "carbamoyl group", "C₁₋₈ alkyl-carbonyl group", "C₃₋₈ cycloalkyl group", "C₆₋₁₈ aryl group", "C₆-₁₈ aryl-C₁₋₄ alkyl group", "C₆₋₁₈ aryl-carbonyl group", "C₆₋₁₈ aryl-C₁₋₄ alkyl-carbonyl group", "heterocyclic group", "heterocyclyl-C₁₋₄ alkyl group", "heterocyclylcarbonyl group" and "heterocyclyl-C₁₋₄ alkyl-carbonyl group" for R^{x} are optionally substituted by one or more (preferably 1 to 5, more preferably 1 to 3) substituents selected from the group consisting of, for example,
(a) halogen atom,
(b) oxo group,
(c) optionally halogenated C₁₋₄ alkyl group,
(d) -(CH₂)ₘ-Q^{x} group,
(e) -(CH₂)ₘ-Z^{1x}- optionally halogenated C₁₋₄ alkyl group,
(f) -(CH₂)ₘ-Z^{1x}-C₃₋₈ cycloalkyl group,
(g) -(CH₂)ₘ-Z^{2x}-(CH₂)ₙ-Q^{x} group,
(h) -(CH₂)ₘ-Z^{2X}-(CH₂)ₙ-Z^{1x}- optionally halogenated C₁₋₄ alkyl group,
(i) -(CH₂)ₘ-Z^{2x}-(CH₂)ₙ-Z^{1x}-C₃₋₈ cycloalkyl group,
(j) -(CH₂)ₘ-Z^{1x}-(optionally substituted heterocyclic group) (preferably, 5- to 8-membered heterocyclic group having 1 to 3 hetero atoms selected from the group consisting of nitrogen atom, oxygen atom, and optionally oxidized sulfur atom),
(k) -(CH₂)ₘ-Z^{2x}-C₁₋₄ alkoxy group, and
(l) -(CH₂)ₘ-Z^{2x}-(CH₂)ₙ-Z^{1x}-(CH₂)ₙ-Z^{1x}-C₁₋₄ alkyl group (substituent group X).

R^{Y} is preferably a hydrogen atom or methyl, particularly preferably a hydrogen atom.

In the above-mentioned the formula,
m is an integer of 0 to 4,
n is an integer of 1 to 4,
Q^{x} is hydroxy, carboxy, cyano, nitro, -NR^{1x}R^{2x}, -CONR^{1x}R^{2x} or - SO₂NR^{1x}R^{2x},
Z^{1x} is -O-, -CO-, -C(OH)R^{3x}-, -C(=N-OR^{3x})-, -S-, -SO-, -SO₂-, - N(COR^{3x})-, -N(CO₂R^{4x})-, -N(SO₂R^{4x})-, -CO-O-, -O-CO-, -CO-NR^{3x}-, - NR^{3x}-CO-, -NR^{3x}-CO₂-, -NR^{3x}-CO-NH-, -NR^{3x}-SO₂- or -NR^{3x}-C(=NH)-NH-, and
Z^{2x} is -O-, -CO-, -C(OH)R^{3x}-, -C(=N-OR^{3x})-, -S-, -SO-, -SO₂-, - NR^{3x}-, -N(COR^{3x})-, -N(CO₂R^{4x})-, -N(SO₂R^{4x})-, -CO-O-, -O-CO-, -CO-NR^{3x}-, -NR^{3x}-CO-, -NR^{3x}-CO₂-, -NR^{3x}-CO-NH-, -NR^{3x}-C(=NH)-NH-, - NR^{3x}-SO₂- or -SO₂-NR^{3x}-.

In addition, -(CH₂)ₘ- and -(CH₂)ₙ- in the above-mentioned formula are, for example, optionally substituted by one or more (preferably 1 to 5, more preferably 1 to 3) substituents selected from halogen, optionally halogenated C₁₋₄ alkyl and hydroxy, and when m or n is two or more, -CH₂CH₂- of -(CH₂)ₘ- or -(CH₂)ₙ- may be replaced with -CH=CH- or -C≡C-.

In the above-mentioned formula, R^{1x} and R^{2x} are the same or different and each is hydrogen atom or C₁₋₄ alkyl, or R^{1x} and R^{2x} may be bonded to form a ring together with nitrogen atom.
In the above-mentioned formula, R^{3x} is a hydrogen atom or C₁₋₄ alkyl and R^{4x} is C₁₋₄ alkyl.

When R^{1x} and R^{2x} are bonded to form a ring together with nitrogen atom, the nitrogen-containing heterocycle is, for example, a 3- to 8-membered (preferably 5- or 6-membered) saturated or unsaturated (preferably saturated) aliphatic heterocycle such as azetidine, pyrrolidine, piperidine, homopiperidine, heptamethylenimine, morpholine, thiomorpholine, piperazine, homopiperazine and the like.

In compound (I'), unless otherwise specified, "optionally substituted group bonded via a carbon atom or a sulfur atom" is, for example, C₁₋₈ alkyl group, C₂₋₈ alkenyl group, C₂₋₈ alkynyl group, carbamoyl group, C₁₋₈ alkyl-carbonyl group, C₁₋₈ alkylthio group, C₁₋₈ alkylsulfonyl group, C₃₋₈ cycloalkyl group, C₆₋₁₈ aryl group, C₆₋₁₈ aryl-C₁₋₄ alkyl group, C₆₋₁₈ aryl-carbonyl group, C₆₋₁₈ aryl-C₁₋₄ alkyl-carbonyl group, C₆₋₁₈ arylthio group, C₆₋₁₈ arylsulfonyl group, heterocyclic group, heterocyclyl-C₁₋₄ alkyl group, heterocyclylcarbonyl group, heterocyclyl-C₁₋₄ alkyl-carbonyl group, heterocyclylthio group, heterocyclyl-C₁₋₄ alkylthio group, each of which is optionally substituted, or the like.

The "C₁₋₈ alkyl group", "C₂₋₈ alkenyl group", "C₂₋₈ alkynyl group", "carbamoyl group", "C₁₋₈ alkyl-carbonyl group", "C₁₋₈ alkylthio group", "C₁₋₈ alkylsulfonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "C₆₋₁₈ arylthio group", "C₆₋₁₈ arylsulfonyl group", "heterocyclic group", "heterocyclyl-C₁₋₄ alkyl group", "heterocyclylcarbonyl group", "heterocyclyl-C₁₋₄ alkyl-carbonyl group", "heterocyclylthio group" and "heterocyclyl-C₁₋₄ alkylthio group" are optionally substituted by one or more (preferably 1 to 5, more preferably 1 to 3) substituents selected from, for example, the above-mentioned substituent group X.

[Compound (I')]

The present invention provides a compound represented by the formula (I') (compound (I')) or a salt thereof.

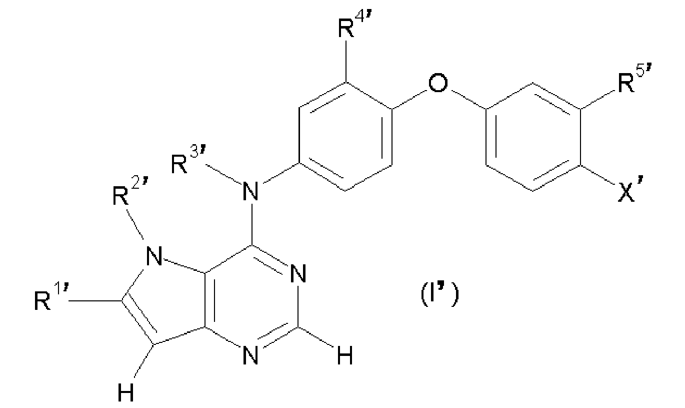

wherein each symbol is as defined above.

R² is preferably a C₁₋₆ alkyl group (particularly, ethyl group) substituted by a group represented by the formula "-NR^{6'}-CO-CR^{7'}R^{8'}-SO₂-C₁₋₄ alkyl".

In the formula, R^{6'} is a hydrogen atom or a methyl group, R^{7'} and R^{8'} are the same or different and each is a hydrogen atom or a methyl group, and R^{7'} and R^{8'} are preferably methyl groups.

R^{3'} is preferably a hydrogen atom.

The "halogen atom" for R^{4'} is preferably a chlorine atom. The "C₁₋₆ alkyl group" for R^{4'} is preferably a methyl group. R^{4'} is preferably a chlorine atom or a methyl group.

The "halogen atom" for R^{5'} is preferably a fluorine atom or a chlorine atom. The "C₁₋₆ alkyl group" for R^{5'} is preferably a methyl group. R^{5'} is preferably a fluorine atom, a chlorine atom or a methyl group.

The "halogen atom" for X' is preferably a fluorine atom. X' is preferably a hydrogen atom or a fluorine atom, more preferably a hydrogen atom.

A preferable embodiment of compound (I') is that wherein R^{1'} is a hydrogen atom, R^{2'} is a C₁₋₆ alkyl group (particularly, ethyl group) substituted by a group represented by -NR^{6'}-CO-CR^{7'}R^{8'}-SO₂-C₁₋₄ alkyl wherein R^{6'} is a hydrogen atom or a methyl group, and R^{7'} and R^{8'} are the same or different and each is a hydrogen atom or a methyl group,
R³ is a hydrogen atom,
R⁴ is a chlorine atom or a methyl group,
R^{5'} is a fluorine atom, a chlorine atom or a methyl group, and
X' is a hydrogen atom or a fluorine atom (preferably, a hydrogen atom).

A more preferable embodiment of compound (I') is that
wherein
R^{1'} is a hydrogen atom,
R^{2'} is a C₁₋₆ alkyl group (particularly, ethyl group) substituted by a group represented by -NR^{6'}-CO-CR^{7'}R^{8'}-SO₂-C₁₋₄ alkyl wherein R^{6'} is a hydrogen atom or a methyl group, and R^{7'} and R^{8'} are methyl groups,
R^{3'} is a hydrogen atom,
R^{4'} is a chlorine atom or a methyl group,
R^{5'} is a fluorine atom, a chlorine atom or a methyl group, and
X' is a hydrogen atom or a fluorine atom (preferably, a hydrogen atom).

As the salts of the compound represented by each of the above-mentioned formulas, for example, metal salt, ammonium salt, salts with organic base, salts with inorganic acid, salts with organic acid, salts with basic or acidic amino acid and the like can be mentioned.

As preferable examples of the metal salt, for example, alkali metal salts such as sodium salt, potassium salt and the like; alkaline earth metal salts such as calcium salt, magnesium salt, barium salt and the like; aluminum salt and the like can be mentioned.

As preferable examples of the salts with organic base, for example, salts with trimethylamine, triethylamine, pyridine, picoline, 2,6-lutidine, ethanolamine, diethanolamine, triethanolamine, tromethamine[tris(hydroxymethyl)methylamine], t-butylamine, cyclohexylamine, dicyclohexylamine, N,N'-dibenzylethylenediamine and the like can be mentioned.

As preferable examples of salts with inorganic acid, for example, salts with hydrochloric acid, hydrobromic acid, nitric acid, sulfuric acid, phosphoric acid and the like can be mentioned.

As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned.

As preferable examples of the salts with basic amino acid, for example, salts with arginine, lysine, ornithine and the like can be mentioned.

As preferable examples of the salts with acidic amino acid, for example, salts with aspartic acid, glutamic acid and the like can be mentioned.

Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

Each term used in the present specification is explained in the following by referring to the compound of the formula (I"):

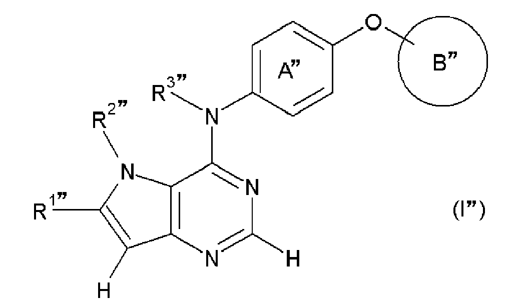

wherein
R^{1"} is a hydrogen atom, or an optionally substituted group bonded via a carbon atom, a nitrogen atom or an oxygen atom, R^{2"} is a hydrogen atom, or an optionally substituted group bonded via a carbon atom or a sulfur atom, or
R^{1"} and R^{2"}, or R^{2"} and R^{3"} are optionally bonded to each other to form an optionally substituted ring structure;
R^{3"} is a hydrogen atom or an optionally substituted aliphatic hydrocarbon group, or
R^{3"} is optionally bonded to a carbon atom of ring A" to form an optionally substituted ring structure;
ring A" is an optionally substituted benzene ring;
ring B" is
(i) an optionally substituted fused ring, or
(ii) a pyridine ring having optionally substituted carbamoyl wherein the pyridine ring is optionally further substituted; (hereinafter sometimes to be abbreviated as compound (I"))
   or a salt thereof.

R^{1"} is a hydrogen atom, a halogen atom, or an optionally substituted group bonded via a carbon atom, a nitrogen atom or an oxygen atom.
Examples of the "halogen atom" for R^{1"} include a fluorine atom, a chlorine atom, a bromine atom and an iodine atom.

Of the "optionally substituted group bonded via a carbon atom, a nitrogen atom or an oxygen atom" for R^{1"}, examples of the "optionally substituted group bonded via a carbon atom" include cyano, optionally substituted C₁₋₈ alkyl, optionally substituted C₂₋₈ alkenyl, optionally substituted C₂₋₈ alkynyl, optionally substituted carbamoyl, optionally substituted C₁₋₈ alkyl-carbonyl, optionally substituted C₃₋₈ cycloalkyl, optionally substituted C₆₋₁₈ aryl, optionally substituted C₆₋₁₈ aryl-C₁₋₄ alkyl, optionally substituted C₆₋₁₈ aryl-carbonyl, optionally substituted C₆₋₁₈ aryl-C₁₋₄ alkyl-carbonyl, an optionally substituted heterocyclic group, optionally substituted heterocyclyl-C₁₋₄ alkyl, optionally substituted heterocyclyl-carbonyl and optionally substituted heterocyclyl-C₁₋₄ alkyl-carbonyl.

Examples of the "C₁₋₈ alkyl" of the above-mentioned "optionally substituted C₁₋₈ alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl and the like.

The "C₁₋₈ alkyl" of the above-mentioned "optionally substituted C₁₋₈ alkyl" may have one or more (preferably 1 to 5, more preferably 1 to 3) substituents at the substitutable positions. Such substituent is selected from the group consisting of
(a) a halogen atom,
(b) oxo,
(c) optionally-halogenated C₁₋₄ alkyl (e.g., methyl, ethyl, propyl, isopropyl, butyl, isobutyl, sec-butyl, tert-butyl),
(d) C₃₋₈ cycloalkyl (e.g., cyclopropyl, cyclobutyl, cyclopentyl, cyclohexyl, cycloheptyl, cyclooctyl etc.),
(e) -(CH₂)ₘ-Q" group,
(f) -(CH₂)ₘ-Z^{1"}-(C₁₋₄ alkyl (e.g., methyl, ethyl, propyl, isopropyl, butyl, isobutyl, sec-butyl, tert-butyl) optionally substituted by substituent(s) selected from hydroxy, a halogen atom, cyano, C₁₋₄ alkoxy, amino and di-C₁₋₄ alkylamino),
(g) -(CH₂)ₘ-Z-(C₃₋₈ cycloalkyl (e.g., cyclopropyl, cyclobutyl, cyclopentyl, cyclohexyl, cycloheptyl, cyclooctyl etc.) optionally substituted by substituent(s) selected from hydroxyl and cyano),
(h) -(CH₂)ₘ-Z^{1"}-(C₆₋₁₀ aryl (e.g., phenyl etc.) optionally substituted by C₁₋₄ alkyl optionally substituted by halogen atom(s)),
(i) -(CH₂)ₘ-Z^{2"}-(CH₂)ₙ-Q" group,
(j) -(CH₂)ₘ-Z^{2"}-(CH2)ₙ-Z^{1"}-C₁₋₄ alkyl (e.g., methyl, ethyl, propyl, isopropyl, butyl, isobutyl, sec-butyl, tert-butyl),
(k) -(CH₂)ₘ-Z^{2"}-(CH₂)ₙ-Z^{1"}-C₃₋₈ cycloalkyl (e.g., cyclopropyl, cyclobutyl, cyclopentyl, cyclohexyl, cycloheptyl, cyclooctyl etc.),
(1) -(CH₂)ₘ-Z^{1"}-(a heterocyclic group optionally substituted by substituent(s) selected from C₁₋₄ alkyl, hydroxy and amino (preferably a 5- to 8-membered heterocyclic group having 1 to 3 heteroatoms selected from a nitrogen atom, an oxygen atom and an optionally oxidized sulfur atom)),
(m) -(CH₂)ₘ-Z^{2"}-(CH₂)ₙ-(a heterocyclic group (preferably a 5- to 8-membered heterocyclic group having 1 to 3 heteroatoms selected from a nitrogen atom, an oxygen atom and an optionally oxidized sulfur atom) optionally substituted by C₁₋₄ alkyl),
(n) -(CH₂)ₘ-Z^{2"}-C₁₋₄ alkoxy (e.g., methoxy, ethoxy, propoxy, isopropoxy, butoxy, isobutoxy, sec-butoxy, tert-butoxy),
(o) -(CH₂)ₘ-Z^{2"}-(CH₂)ₙ-Z^{1"}-(CH₂)ₙ-Z^{1"}-C₁₋₄ alkyl (e.g., methyl, ethyl, propyl, isopropyl, butyl, isobutyl, sec-butyl, tert-butyl), and
(p) 3- to 6-membered cyclic amino optionally substituted by substituent(s) selected from C₁₋₄ alkyl and oxo (hereinafter sometimes to be referred to as substituent group X). When the number of the substituents is 2 or more, the respective substituents may be the same or different.

In the above-mentioned formulas,
m is an integer of 0 to 4;
n is an integer of 1 to 4;
Q" is hydroxy, carboxy, cyano, nitro, -NR^{11"}R^{12"}, -CONR^{11"}R^{12"} or - S02NR^{11"}R^{12"};
Z^{1"} is -O-, -CO-, -C(OH)R^{13"}-, -C(=N-OR^{13"})-, -S-, -SO-, -SO₂-, - N(COR^{13"})-, -N(CO2R^{14"})-, -N(SO2R^{14"})-, -CO-O-, -O-CO-, -CO-NR^{13"}-, -NR^{13"}-CO-, -NR^{13"}-CO2-, -NR^{13"}-CO-NH-, -NR^{13"}-SO₂- or -NR-^{13"} C(=NH)-NH-; and
Z^{2"} is -O-, -CO-, -C(OH)R^{13"}-, -C(=N-OR^{13"})-, -S-, -SO-, -SO₂-, - NR^{13"}-, -N(COR^{13"})-, -N(CO2R^{14"})-, -N(SO2R^{14"})-, -CO-O-, -O-CO-, - CO-NR^{13"}-, -NR^{13"}-CO-, -NR^{13"}-CO₂-, -NR^{13"}-CO-NH-, -NR^{13"}-C(=NH)-NH-, -NR^{13"}-SO₂- or -SO₂-NR^{13"}-.

In addition, -(CH₂)ₘ- and -(CH₂₎ₙ- in the above-mentioned formulas are optionally substituted by, for example, one or more (preferably 1 to 5, more preferably 1 to 3) substituents selected from halogen, optionally halogenated C₁₋₄ alkyl and hydroxy. When m or n is not less than 2, a subset -CH₂CH₂- of -(CH₂)ₘ- and -(CH₂)ₙ- may be replaced by -CH=CH- or -C≡C-.

In the above-mentioned formulas, R^{11"} and R^{12"} are the same or different and each is a hydrogen atom or C₁₋₄ alkyl (e.g., methyl, ethyl, propyl, isopropyl, butyl, isobutyl, sec-butyl, tert-butyl), or R^{11"} and R^{12"} may be bonded to form a ring together with the nitrogen atom.

In addition, in the above-mentioned formulas, R^{13"} is a hydrogen atom or C₁₋₄ alkyl (e.g., methyl, ethyl, propyl, isopropyl, butyl, isobutyl, sec-butyl, tert-butyl), and R^{14"} is C₁₋₄ alkyl (e.g., methyl, ethyl, propyl, isopropyl, butyl, isobutyl, sec-butyl, tert-butyl).

When R^{11"} and R^{12"} are bonded to form a ring together with the nitrogen atom, Examples of the nitrogen-containing heterocyclic group include a 3- to 8-membered (preferably 5-or 6-membered) saturated or unsaturated (preferably saturated) aliphatic heterocycle such as azetidine, pyrrolidine, piperidine, homopiperidine, heptamethylenimine, morpholine, thiomorpholine, piperazine, homopiperazine and the like.

Examples of the "C₂₋₈ alkenyl" of the above-mentioned "optionally substituted C₂₋₈ alkenyl" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

The "C₂₋₈ alkenyl" of the above-mentioned "optionally substituted C₂₋₈ alkenyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

Examples of the "C₂₋₈ alkynyl" of the above-mentioned "optionally substituted C₂₋₈ alkynyl" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

The "C₂₋₈ alkynyl" of the above-mentioned "optionally substituted C₂₋₈ alkynyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

The "carbamoyl" of the above-mentioned "optionally substituted carbamoyl" may have 1 or 2 substituents at the substitutable positions. Examples of such substituent include substituents selected from substituent group X. When the number of the substituents is not less than 2, the respective substituents may be the same or different.

Examples of the "C₁₋₈ alkyl-carbonyl" of the above-mentioned "optionally substituted C₁₋₈ alkyl-carbonyl" include acetyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, neopentylcarbonyl, 1-ethylpropylcarbonyl, hexylcarbonyl, isohexylcarbonyl, 1,1-dimethylbutylcarbonyl, 2,2-dimethylbutylcarbonyl, 3,3-dimethylbutylcarbonyl, 2-ethylbutylcarbonyl, heptylcarbonyl, octylcarbonyl and the like.

The "C₁₋₈ alkyl-carbonyl" of the above-mentioned "optionally substituted C₁₋₈ alkyl-carbonyl" may have one or more (preferably 1 to 5, more preferably 1 to 3) substituents at the substitutable positions. Examples of such substituent include substituents selected from substituent group X. When the number of the substituents is not less than 2, the respective substituents may be the same or different.

Examples of the "C₃₋₈ cycloalkyl" of the above-mentioned "optionally substituted C₃₋₈ cycloalkyl" include cyclopropyl, cyclobutyl, cyclopentyl, cyclohexyl, cycloheptyl, cyclooctyl and the like.

The "C₃₋₈ cycloalkyl" of the above-mentioned "optionally substituted C₃₋₈ cycloalkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "C₆₋₁₈ aryl" of the above-mentioned "optionally substituted C₆₋₁₈ aryl" include phenyl, naphthyl, anthryl, phenanthryl, acenaphthyl, biphenylyl and the like.

The "C₆₋₁₈ aryl" of the above-mentioned "optionally substituted C₆₋₁₈ aryl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "C₆₋₁₈ aryl-C₁₋₄ alkyl" of the above-mentioned "optionally substituted C₆₋₁₈ aryl-C₁₋₄ alkyl" include benzyl, phenethyl, phenylpropyl, naphthylmethyl, biphenylylmethyl and the like.

The "C₆₋₁₈ aryl-C₁₋₄ alkyl" of the above-mentioned "optionally substituted C₆₋₁₈ aryl-C₁₋₄ alkyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "C₆₋₁₈ aryl-carbonyl" of the above-mentioned "optionally substituted C₆₋₁₈ aryl-carbonyl" include phenylcarbonyl, naphthylcarbonyl, anthrylcarbonyl, phenanthrylcarbonyl, acenaphthylcarbonyl, biphenylylcarbonyl and the like.

The "C₆₋₁₈ aryl-carbonyl" of the above-mentioned "optionally substituted C₆₋₁₈ aryl-carbonyl" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the "C₆₋₁₈ aryl-C₁₋₄ alkyl-carbonyl" of the above-mentioned "optionally substituted C₆₋₁₈ aryl-C₁₋₄ alkyl-carbonyl" include benzylcarbonyl, phenethylcarbonyl, phenylpropylcarbonyl, naphthylmethylcarbonyl, biphenylylmethylcarbonyl and the like.

The "C₆₋₁₈ aryl-C₁₋₄ alkyl-carbonyl" of the above-mentioned "optionally substituted C₆₋₁₈ aryl-C₁₋₄ alkyl-carbonyl" may have one or more (preferably 1 to 5, more preferably 1 to 3) substituents at the substitutable positions. Examples of such substituent include substituents selected from the below-mentioned substituent V. When the number of the substituents is not less than 2, the respective substituents may be the same or different.

Examples of the "heterocyclic group" of the above-mentioned "optionally substituted heterocyclic group" include an aromatic heterocyclic group and a non-aromatic heterocyclic group.

Here, examples of the "aromatic heterocyclic group" include a 4- to 7-membered (preferably 5- or 6-membered) monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group. Examples of the fused aromatic heterocyclic group include a group derived from a fused ring wherein a ring corresponding to such 4- to 7-membered monocyclic aromatic heterocyclic group, and 1 or 2 rings selected from a 5- or 6-membered aromatic heterocycle containing 1 or 2 nitrogen atoms, a 5-membered aromatic heterocycle containing one sulfur atom and a benzene ring and the like are condensed, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), pyrrolopyrimidinyl (e.g., 1H-pyrrolo[2,3-d]pyrimidin-2-yl, 1H-pyrrolo[2,3-d]pyrimidin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl) and the like;
and the like.

Examples of the "non-aromatic heterocyclic group" include a 4- to 7-membered (preferably 5- or 6-membered) monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group. Examples of the fused non-aromatic heterocyclic group include a group derived from a fused ring wherein a ring corresponding to such 4- to 7-membered monocyclic non-aromatic heterocyclic group, and 1 or 2 rings selected from a 5- or 6-membered heterocycle containing 1 or 2 nitrogen atoms, a 5-membered heterocycle containing one sulfur atom and a benzene ring and the like are fused, and the like.

Preferable examples of the non-aromatic heterocyclic group include monocyclic non-aromatic heterocyclic groups such as oxetanyl (e.g., 2-oxetanyl, 3-oxetanyl), pyrrolidinyl (e.g., 1-pyrrolidinyl, 2-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), pyrazolidinyl (e.g., pyrazolidin-1-yl, pyrazolidin-3-yl), pyrazolinyl (e.g., pyrazolin-1-yl), tetrahydropyrimidinyl (e.g., tetrahydropyrimidin-1-yl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl) and the like; fused non-aromatic heterocyclic groups such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl) and the like; and the like.

The "heterocyclic group" of the above-mentioned "optionally substituted heterocyclic group" may have one or more (preferably 1 to 5, more preferably 1 to 3) substituents at the substitutable positions. Examples of such substituent include substituents selected from the below-mentioned substituent group V. When the number of the substituents is not less than 2, the respective substituents may be the same or different.

Examples of the above-mentioned "optionally substituted heterocyclyl-C₁₋₄ alkyl" include a group wherein C₁₋₄ alkyl (e.g., methoxy, ethoxy, propoxy, isopropoxy, butoxy, isobutoxy, sec-butoxy, tert-butoxy) is substituted by the above-mentioned "optionally substituted heterocyclic group".

Examples of the above-mentioned "optionally substituted heterocyclyl-carbonyl" include a group wherein the above-mentioned "optionally substituted heterocyclic group" is bonded to carbonyl.

Examples of the above-mentioned "optionally substituted heterocyclyl-C₁₋₄ alkyl-carbonyl" include a group wherein the above-mentioned "optionally substituted heterocyclyl-C₁₋₄ alkyl" is bonded to carbonyl.

Of the "optionally substituted group bonded via a carbon atom, a nitrogen atom or an oxygen atom" for R^{1"}, examples of the "optionally substituted group bonded via a nitrogen atom" include
(i) amino,
(ii) amino mono-substituted by the above-mentioned "optionally substituted group bonded via a carbon atom", and
(iii) amino di-substituted by the above-mentioned "optionally substituted group bonded via a carbon atom" and C₁₋₆ alkyl (e.g., methyl, ethyl, propyl, isopropyl, butyl, isobutyl, sec-butyl, tert-butyl, pentyl, propyl etc.).

Of the "optionally substituted group bonded via a carbon atom, a nitrogen atom or an oxygen atom" for R^{1"}, examples of the "optionally substituted group bonded via an oxygen atom" include hydroxy optionally substituted by the above-mentioned "optionally substituted group bonded via a carbon atom".

As R^{1"}, a hydrogen atom, a halogen atom or cyano is preferable, and a hydrogen atom or a halogen atom (particularly, a chlorine atom) is particularly preferable.

R^{2"} is a hydrogen atom, or an optionally substituted group bonded via a carbon atom or a sulfur atom.

Of the "optionally substituted group bonded via a carbon atom or a sulfur atom" for R^{2"}, examples of the "optionally substituted group bonded via a carbon atom" include those similar to the "optionally substituted group bonded via a carbon atom" for R^{1"}.

Of the "optionally substituted group bonded via a carbon atom or a sulfur atom" for R^{2"}, examples of the "optionally substituted group bonded via a sulfur atom" include mercapto optionally substituted by the above-mentioned "optionally substituted group bonded via a carbon atom" wherein the sulfur atom may be oxidized.

As R², a hydrogen atom or optionally substituted alkyl is preferable. Of these,
(1) a hydrogen atom, or
(2) C₁₋₆ alkyl optionally substituted by 1 to 3 substituents selected from the group consisting of
   (a) C₃₋₆ cycloalkyl,
   (b) -O-(CH₂)ₙ-OH,
   (c) -NR^{5"}-(CH₂)ₙ-OH,
   (d) -NR^{5"}-CO-(C₁₋₄ alkyl optionally substituted by 1 to 4 substituents selected from hydroxy, halogen atom, cyano, C₁₋₄ alkoxy, amino and di-C₁₋₄ alkylamino),
   (e) -NR^{5"}-CO-(CH₂)ₙ-SO₂-C₁₋₄ alkyl,
   (f) -NR^{5"}-CO-(5- or 6-membered heterocycle optionally substituted by 1 or 2 C₁₋₄ alkyl),
   (g) -NR^{5"}-CO-(CH₂)ₙ-(6-membered heterocycle optionally substituted by C₁₋₄ alkyl),
   (h) -NR^{5"}-CO- (C₃₋₆ cycloalkyl optionally substituted by substituent(s) selected from hydroxy and cyano),
   (i) -NR^{5"}-CO- (C₆₋₁₀ aryl optionally substituted by C₁₋₄ alkyl optionally substituted by 1 to 3 halogen atoms),
   (j) -NR^{5"}-CO-NR^{5"'}-C₃₋₆ cycloalkyl,
   (k) -NR^{5"}-SO₂-C₁₋₄ alkyl,
   (l) C₁₋₄ alkyl-carbonyl,
   (m) (5- or 6-membered heterocycle optionally substituted by 1 or 2 substituents selected from hydroxy and amino)-carbonyl,
   (n) hydroxy,
   (o) a halogen atom, and
   (p) 3- to 6 -membered cyclic amino optionally substituted by 1 to 3 substituents selected from C₁₋₄ alkyl and oxo, wherein n is an integer of 1 to 4, R^{5"} and R^{5'"} are each a hydrogen atom or C₁₋₄ alkyl, -(CH₂)ₙ- is optionally
      substituted by C₁₋₄ alkyl, is preferable, and particularly, methyl or ethyl substituted by hydroxy is preferable.

R^{3"} is a hydrogen atom or an optionally substituted aliphatic hydrocarbon group.

Examples of the "aliphatic hydrocarbon group" of the "an optionally substituted aliphatic hydrocarbon group" for R^{3"} include those similar to the "optionally substituted C₁₋₈ alkyl", "optionally substituted C₂₋₈ alkenyl", "optionally substituted C₂₋₈ alkynyl" and "optionally substituted C₃₋₈ cycloalkyl" exemplified as the "optionally substituted group bonded via a carbon atom" for R^{1"}.

As R^{3"}, a hydrogen atom is preferable.

Ring A" is an optionally substituted benzene ring.
The "benzene ring" of the "optionally substituted benzene ring" for ring A" is optionally substituted by 1 to 5 substituents selected from the group consisting of
(1) C₃₋₁₀ cycloalkyl (e.g., cyclopropyl, cyclohexyl) optionally substituted by 1 to 3 substituents selected from the group consisting of
   (a) halogen;
   (b) hydroxy;
   (c) carboxyl;
   (d) sulfo;
   (e) cyano;
   (f) azido;
   (g) nitro;
   (h) nitroso;
   (i) optionally halogenated C₁₋₄ alkyl (e.g., methyl, ethyl, propyl, isopropyl, butyl, isobutyl, sec-butyl, tert-butyl);
   (j) optionally halogenated C₂₋₄ alkenyl (e.g., ethenyl, 1-propenyl, 2-propenyl, 2-methyl-1-propenyl, 1-butenyl, 2-butenyl, 3-butenyl);
   (k) optionally halogenated C₂₋₄ alkynyl (e.g., ethynyl, 1-propynyl, 2-propynyl, 1-butynyl, 2-butynyl, 3-butynyl);
   (l) C₃₋₇ cycloalkyl (e.g., cyclopropyl, cyclobutyl, cyclopentyl, cyclohexyl, cycloheptyl);
   (m) C₆₋₁₄ aryl (e.g., phenyl, naphthyl, anthryl, phenanthryl, acenaphthyl, biphenylyl);
   (n) C₇₋₁₆ aralkyl (e.g., benzyl, phenethyl, phenylpropyl, naphthylmethyl, biphenylylmethyl);
   (o) formyl;
   (p) optionally halogenated C₁₋₆ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl);
   (q) optionally halogenated C₁₋₆ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
   (r) optionally halogenated C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl);
   (s) carbamoyl;
   (t) carbamoyl mono- or di-substituted by optionally halogenated C₁₋₆ alkyl (e.g., methyl, ethyl, propyl, isopropyl, butyl, isobutyl, sec-butyl, tert-butyl);
   (u) mono- or di-C₆₋₁₄ aryl-carbamoyl (e.g., phenylcarbamoyl, naphthylcarbamoyl, anthrylcarbamoyl, phenanthrylcarbamoyl, acenaphthylcarbamoyl, biphenylylcarbamoyl);
   (v) thiocarbamoyl optionally mono- or di-substituted by optionally halogenated C₁₋₆ alkyl (e.g., methyl, ethyl, propyl, isopropyl, butyl, isobutyl, sec-butyl, tert-butyl);
   (w) ureido optionally mono- or di-substituted by optionally halogenated C₁₋₆ alkyl (e.g., methyl, ethyl, propyl, isopropyl, butyl, isobutyl, sec-butyl, tert-butyl);
   (x) mono- or di-C₆₋₁₄ aryl-ureido (e.g., phenylureido, naphthylureido, anthrylureido, phenanthrylureido, acenaphthylureido, biphenylylureido);
   (y) sulfamoyl optionally mono- or di-substituted by optionally halogenated C₁₋₆ alkyl (e.g., methyl, ethyl, propyl, isopropyl, butyl, isobutyl, sec-butyl, tert-butyl);
   (z) optionally halogenated C₁₋₆ alkoxy (e.g., methoxy, ethoxy, propoxy, isopropoxy, butoxy, tert-butoxy, pentyloxy, hexyloxy);
   (aa) optionally halogenated C₂₋₆ alkenyloxy (e.g., ethenyloxy, 1-propenyloxy, 2-propenyloxy, 2-methyl-1-propenyloxy, 1-butenyloxy, 2-butenyloxy, 3-butenyloxy);
   (bb) C₃₋₁₀ cycloalkyloxy (e.g., cyclopropyloxy, cyclobutyloxy, cyclopentyloxy, cyclohexyloxy, cycloheptyloxy);
   (cc) C₇₋₁₃ aralkyloxy (e.g., benzyloxy, phenethyloxy, phenylpropyloxy, naphthylmethyloxy, biphenylylmethyloxy); (dd) C₆₋₁₄ aryloxy (e.g., phenyloxy, naphthyloxy, anthryloxy, phenanthryloxy, acenaphthyloxy, biphenylyloxy);
   (ee) C₁₋₆ alkyl-carbonyloxy (e.g., acetyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy);
   (ff) C₃₋₁₀ cycloalkyl (e.g., cyclopropyl, cyclobutyl, cyclopentyl, cyclohexyl, cycloheptyl)-C₁₋₆ alkyloxy (e.g., methoxy, ethoxy, propoxy, isopropoxy, butoxy, tert-butoxy, pentyloxy, hexyloxy);
   (gg) C₁₋₆ alkylsulfonyloxy (e.g., methylsulfonyloxy, ethylsulfonyloxy, propylsulfonyloxy, isopropylsulfonyloxy, butylsulfonyloxy, isobutylsulfonyloxy, sec-butylsulfonyloxy, tert-butylsulfonyloxy);
   (hh) mercapto;
   (ii) optionally halogenated C₁₋₆ alkylthio (e.g., methylthio, ethylthio, propylthio, isopropylthio, butylthio, isobutylthio, sec-butylthio, tert-butylthio);
   (jj) C₇₋₁₃ aralkylthio (e.g., benzylthio, phenethylthio, phenylpropylthio, naphthylmethylthio, biphenylylmethylthio);
   (kk) C₆₋₁₄ arylthio (e.g., phenylthio, naphthylthio, anthrylthio, phenanthrylthio, acenaphthylthio, biphenylylthio);
   (11) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl); (mm) oxo;
   (nn) C₁₋₃ alkylenedioxy (e.g., methylenedioxy, ethylenedioxy, propylenedioxy); and
   (oo) hydroxyimino optionally substituted by C₁₋₆ alkyl (e.g., methyl, ethyl, propyl, isopropyl, butyl, isobutyl, sec-butyl, tert-butyl); (hereinafter sometimes to be referred to as substituent group A),
(2) C₆₋₁₄ aryl (e.g., phenyl, naphthyl) optionally substituted by 1 to 3 substituents selected from substituent group A;
(3) a heterocyclic group (the heterocyclic group is similar to the "heterocyclic group" of the "optionally substituted heterocyclic group" exemplified as the "optionally substituted group bonded via a carbon atom" for R^{1"}) optionally substituted by 1 to 3 substituents selected from substituent group A;
(4) amino optionally substituted by 1 or 2 substituents selected from the group consisting of
   (a) C₁₋₆ alkyl (e.g., methyl, ethyl, propyl, isopropyl, butyl, isobutyl, sec-butyl, tert-butyl) optionally substituted by substituent(s) selected from the group consisting of halogen, hydroxy, C₃₋₇ cycloalkyl (e.g., cyclopropyl, cyclobutyl, cyclopentyl, cyclohexyl, cycloheptyl), C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and C₁₋₆ alkoxy (e.g., methoxy, ethoxy, propoxy, isopropoxy, butoxy, tert-butoxy, pentyloxy, hexyloxy);
   (b) optionally halogenated C₂₋₄ alkenyl (e.g., ethenyl, 1-propenyl, 2-propenyl, 2-methyl-1-propenyl, 1-butenyl, 2-butenyl, 3-butenyl);
   (c) optionally halogenated C₂₋₄ alkynyl (e.g., ethynyl, 1-propynyl, 2-propynyl, 1-butynyl, 2-butynyl, 3-butynyl);
   (d) C₃₋₇ cycloalkyl (e.g., cyclopropyl, cyclobutyl, cyclopentyl, cyclohexyl, cycloheptyl);
   (e) C₆₋₁₄ aryl (e.g., phenyl, naphthyl, anthryl, phenanthryl, acenaphthyl, biphenylyl);
   (f) C₇₋₁₆ aralkyl (e.g., benzyl, phenethyl, phenylpropyl, naphthylmethyl, biphenylylmethyl);
   (g) a 4- to 7-membered (preferably 5- or 6-membered) heterocyclic group (e.g., a non-aromatic heterocyclic group such as morpholinyl etc.) containing, as a ring-constituting atom besides carbon atoms, 1 to 4 heteroatoms selected from an oxygen atom, a sulfur atom and a nitrogen atom;
   (h) formyl;
   (i) C₁₋₆ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl) optionally substituted by substituent(s) selected from the group consisting of halogen, hydroxy, C₃₋₇ cycloalkyl (e.g., cyclopropyl, cyclobutyl, cyclopentyl, cyclohexyl, cycloheptyl), C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl) and C₁₋₆ alkoxy (e.g., methoxy, ethoxy, propoxy, isopropoxy, butoxy, tert-butoxy, pentyloxy, hexyloxy);
   (j) C₁₋₆ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
   (k) C₆₋₁₄ aryl-carbonyl (e.g., benzoyl);
   (l) C₇₋₁₃ aralkyl-carbonyl (e.g., benzylcarbonyl, phenethylcarbonyl);
   (m) C₃₋₇ cycloalkyl-carbonyl (e.g., cyclopropylcarbonyl, cyclobutylcarbonyl, cyclopentylcarbonyl, cyclohexylcarbonyl, cycloheptylcarbonyl);
   (o) C₁₋₆ alkyl-carbamoyl (e.g., methylcarbamoyl, ethylcarbamoyl);
   (p) C₆₋₁₄ aryl-carbamoyl (e.g., phenylcarbamoyl, 1-naphthylcarbamoyl, 2-naphthylcarbamoyl);
   (q) C₇₋₁₃ aralkyl-carbamoyl (e.g., benzylcarbamoyl);
   (r) C₁₋₆ alkylsulfonyl (e.g., methylsulfonyl, ethylsulfonyl, isopropylsulfonyl);
   (s) C₆₋₁₄ arylsulfonyl (e.g., benzenesulfonyl, toluenesulfonyl, 1-naphthalenesulfonyl, 2-naphthalenesulfonyl); and
   (t) C₇₋₁₃ aralkylsulfonyl (e.g., benzylsulfonyl) (hereinafter sometimes to be referred to as substituent group B);
(5) amidino;
(6) optionally formylated or halogenated C₁₋₆ alkyl-carbonyl (e.g., methylcarbonyl, ethylcarbonyl, propylcarbonyl, isopropylcarbonyl, butylcarbonyl, isobutylcarbonyl, sec-butylcarbonyl, tert-butylcarbonyl);
(7) optionally halogenated C₁₋₆ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(8) optionally halogenated C₁₋₆ alkylsulfonyl (e.g., methylsulfonyl);
(9) carbamoyl optionally substituted by 1 or 2 substituents selected from substituent group B;
(10) thiocarbamoyl optionally mono- or di-substituted by optionally halogenated C₁₋₆ alkyl (e.g., methyl, ethyl, propyl, isopropyl, butyl, isobutyl, sec-butyl, tert-butyl);
(11) ureido optionally substituted by 1 or 2 substituents selected from substituent group B;
(12) sulfamoyl optionally substituted by 1 or 2 substituents selected from substituent group B;
(13) carboxyl;
(14) hydroxy;
(15) C₁₋₆ alkoxy (e.g., methoxy, ethoxy, propoxy, isopropoxy, butoxy, tert-butoxy, pentyloxy, hexyloxy) optionally substituted by 1 to 3 substituents selected from the group consisting of halogen, carboxyl, C₁₋₆ alkoxy (e.g., methoxy, ethoxy, propoxy, isopropoxy, butoxy, tert-butoxy, pentyloxy, hexyloxy) and C₁₋₆ alkoxy-carbonyl (e.g., methoxycarbonyl, ethoxycarbonyl, propoxycarbonyl, isopropoxycarbonyl, butoxycarbonyl, tert-butoxycarbonyl, pentyloxycarbonyl, hexyloxycarbonyl);
(16) optionally halogenated C₂₋₆ alkenyloxy (e.g., ethenyloxy);
(17) C₃₋₁₀ cycloalkyloxy (e.g., cyclohexyloxy);
(18) C₇₋₁₃ aralkyloxy (e.g., benzyloxy);
(19) C₆₋₁₄ aryloxy (e.g., phenyloxy, naphthyloxy);
(20) C₁₋₆ alkyl-carbonyloxy (e.g., acetyloxy, tert-butylcarbonyloxy);
(21) mercapto;
(22) optionally halogenated C₁₋₆ alkylthio (e.g., methylthio, ethylthio);
(23) C₇₋₁₃ aralkylthio (e.g., benzylthio);
(24) C₆₋₁₄ arylthio (e.g., phenylthio, naphthylthio);
(25) sulfo;
(26) cyano;
(27) azido;
(28) nitro;
(29) nitroso;
(30) a halogen atom;
(31) C₁₋₆ alkylsulfinyl (e.g., methylsulfinyl);
(32) oxo;
(33) C₃₋₁₀ cycloalkyl-C₁₋₆ alkyloxy (e.g., cyclopropylmethyloxy);
(34) C₁₋₃ alkylenedioxy (e.g., methylenedioxy, ethylenedioxy);
(35) hydroxyimino optionally substituted by C₁₋₆ alkyl;
(36) C₁₋₁₀ alkyl (e.g., methyl, ethyl, propyl, isopropyl, butyl, isobutyl, sec-butyl, tert-butyl) optionally substituted by 1 to 3 substituents selected from the above-mentioned (1) - (35);
(37) C₂₋₁₀ alkenyl (e.g., ethenyl, 1-propenyl) optionally substituted by 1 to 3 substituents selected from the above-mentioned (1) - (35), and
(38) C₇₋₁₃ aralkyl (e.g., benzyl) optionally substituted by 1 to 3 substituents selected from the above-mentioned (1) - (35) (hereinafter sometimes to be referred to as substituent group V). When the number of the substituents is not less than 2, the respective substituents may be the same or different.

As ring A^{"}, a benzene ring optionally substituted by 1 or 2 substituents selected from the group consisting of (1) a halogen atom and (2) C₁₋₄ alkyl is preferable. Particularly, a benzene ring optionally substituted by 1 or 2 substituents selected from the group consisting of a halogen atom and methyl is preferable. Especially, a benzene ring optionally substituted by one substituent selected from the group consisting of a halogen atom and methyl is preferable.

Ring B^{"} is
(i) an optionally substituted fused ring, or
(ii) a pyridine ring having optionally substituted carbamoyl (said pyridine ring is optionally further substituted).

The "optionally substituted fused ring" for ring B^{"} is, for example, an "optionally substituted fused homocyclic ring" or an "optionally substituted fused heterocycle".
The "fused homocyclic ring" of the "optionally substituted fused homocyclic ring" is, for example, a ring
wherein two or more, the same or different rings selected from benzene, C₃₋₈ cycloalkane (e.g., cyclopropane, cyclobutane, cyclopentane, cyclohexane, cycloheptane, cyclooctane), C₃₋₈ cycloalkene (e.g., cyclopropene, cyclobutene, cyclopentene, cyclohexene, cycloheptene, cyclooctene), C₄₋₈ cycloalkadiene (e.g., cyclobutadiene, cyclopentadiene, cyclohexadiene, cycloheptadiene, cyclooctadiene), C₇₋₈ cycloalkatriene (e.g., cycloheptatriene, cyclooctatriene) and cyclooctatetraene are fused. Specifically, naphthalene, dihydronaphthalene, tetrahydronaphthalene, hexahydronaphthalene, decahydronaphthalene, pentalene, indene, indane, azulene, heptalene and the like can be mentioned.
The "fused heterocycle" of the "optionally substituted fused heterocycle" is, for example, a fused aromatic heterocycle such as quinoline, isoquinoline, quinazoline, quinoxaline, benzofuran, benzothiophene, benzooxazole, benzoisoxazole, benzothiazole, benzoimidazole, benzotriazole, indole, indazole, pyrrolopyridine, pyrrolopyrimidine, pyrrolopyrazine, imidazopyridine, imidazopyrazine, imidazopyridazine, pyrazolopyridine, pyrazolothiophene, pyrazolotriazine, triazolopyridine and the like; or a fused non-aromatic heterocycle such as dihydroindole, dihydroisoindole, dihydrobenzofuran, dihydrobenzothiophene, dihydrobenzodioxine, dihydrobenzodioxepine, tetrahydrobenzofuran, tetrahydrobenzothiophene, chromene, dihydroquinoline, tetrahydroquinoline, dihydroisoquinoline, tetrahydroisoquinoline, dihydrophthalazine, benzooxazoline, benzisoxazoline, benzothiazoline, benzimidazoline, benzotriazoline, indazoline, dihydropyrrolopyridine and the like.

The "fused ring" of the "optionally substituted fused ring" for ring B^{"} optionally have one or more (preferably 1 to 5, more preferably 1 to 3) substituents at substitutable positions. Examples of the substituent include substituents selected from substituent group V and C₂₋₄ alkylene (e.g., ethylene, propylene, trimethylene, tetramethylene). The C₂₋₄ alkylene may be bonded to a single carbon atom of ring B^{"} to form a spiro ring. When the number of substituents is two or more, the respective substituents may be the same or different.

The "optionally substituted carbamoyl" of the "pyridine ring having optionally substituted carbamoyl" for ring B^{"} is, for example, carbamoyl optionally mono- or di-substituted by a group similar to the "optionally substituted group bonded via carbon atom" exemplified for R^{1"}.
The "pyridine ring having optionally substituted carbamoyl" for ring B^{"} is optionally further substituted, and the substituent that is optionally further present is, for example, a substituent selected from substituent group V.

As ring B^{"},
(1) a fused ring optionally substituted by substituent selected from the group consisting of
   (a) halogen atom,
   (b) cyano,
   (c) C₁₋₆ alkyl optionally substituted by halogen atom or C₃₋₆ cycloalkyl,
   (d) oxo,
   (e) C₂₋₄ alkylene,
   (f) hydroxy,
   (g) carbamoyl,
   (h) C₁₋₆ alkyl-carbamoyl, and
   (i) C₁₋₆ alkoxy-carbonyl, or
(2) a pyridine ring having carbamoyl optionally substituted by C₁₋₆ alkyl (said pyridine ring is optionally further substituted by C₁₋₆ alkyl) is preferable.

Particularly,
(1) a fused homocyclic ring (e.g., indane, naphthalene) optionally having, as substituent, 1 or 2 substituents selected from the group consisting of (a) C₁₋₆ alkyl optionally substituted by halogen atom, (b) hydroxy and (c) oxo;
(2) a fused heterocycle (e.g., quinoline, isoquinoline, quinazoline, quinoxaline, benzooxazoline, benzisoxazoline, benzothiazoline, benzimidazoline, benzotriazoline, indole, indazole, pyrrolopyridine, dihydropyrrolopyridine, benzoxazole, benzimidazole, benzothiazole, benzisoxazole, benzisothiazole, pyrrolopyrimidine, imidazopyridazine, indazoline, pyrrolopyrazine, imidazopyridine, imidazopyrazine, pyrazolopyridine, pyrazolothiophene, pyrazolotriazine, dihydroindole, dihydroisoindole, dihydroquinoline, tetrahydroquinoline, dihydroisoquinoline, tetrahydroisoquinoline, dihydrophthalazine, dihydrobenzoxazole, benzothiophene, benzofuran, dihydrobenzothiophene, dihydrobenzofuran and the like) optionally having, as substituent, 1 to 4 substituents selected from the group consisting of (a) halogen atom, (b) cyano, (c) C₁₋₆ alkyl optionally substituted by halogen atom or C₃₋₆ cycloalkyl, (d) oxo, (e) C₂₋₄ alkylene, (f) carbamoyl, (g) C₁₋₆ alkyl-carbamoyl, and (h) C₁₋₆ alkoxy-carbonyl; or
(3) a pyridine ring having carbamoyl optionally substituted by C₁₋₈ alkyl (said pyridine ring is optionally further substituted by C₁₋₆ alkyl) ; is preferable.

Especially,
(1) indole optionally having one C₁₋₄ alkyl;
(2) pyrrolopyrimidine optionally having one C₁₋₄ alkyl;
(3) imidazopyridine;
(4) dihydroindole optionally having 1 or 2 substituents selected from the group consisting of
   (a) C₁₋₄ alkyl optionally substituted by C₃₋₆ cycloalkyl, (b) halogen atom, (c) C₂₋₄ alkylene and (d) oxo;
(5) dihydroisoindole optionally having 1 to 4 substituents selected from the group consisting of
   (a) C₁₋₄ alkyl, (b) halogen atom and (c) oxo;
(6) dihydrobenzoxazole optionally having 1 or 2 substituents selected from the group consisting of C₁₋₄ alkyl and oxo;
(7) pyrrolopyridine;
(8) indane optionally having 1 or 2 substituents selected from
   (a) C₁₋₆ alkyl optionally substituted by 1 to 3 halogen atoms,
   (b) hydroxy and (c) oxo;
(9) benzothiophene;
(10) indazole optionally having one substituent selected from the group consisting of halogen atom and C₁₋₄ alkyl;
(11) dihydrobenzofuran;
(12) quinoline optionally having one substituent selected from the group consisting of
   (a) halogen atom and (b) C₁₋₆ alkyl optionally substituted by 1 to 3 halogen atoms;
(13) benzooxazole;
(14) benzofuran optionally having one substituent selected from the group consisting of
   (a) cyano and (b) carbamoyl;
(15) benzoisoxazole optionally having one C₁₋₄ alkyl;
(16) pyrazolopyridine optionally having one substituent selected from the group consisting of
   (a) C₁₋₆ alkoxy-carbonyl and (b) C₁₋₆ alkyl-carbamoyl;
(17) benzoimidazole optionally having 1 or 2 C₁₋₄ alkyl;
(18) triazolopyridine;
(19) naphthalene; or
(20) pyridine substituted by C₁₋₈ alkyl-carbamoyl and optionally further substituted by C₁₋₆ alkyl; is preferable.

R^{1"} and R^{2"} are optionally bonded to each other to form an optionally substituted ring structure. Examples of the "ring structure" include a saturated or unsaturated (preferably saturated) 4- to 8-membered (preferably 5- to 7-membered) heterocycle.

Examples of the "ring structure" of the "optionally substituted ring structure" formed by R^{1"} and R^{2"} bonded to each other include

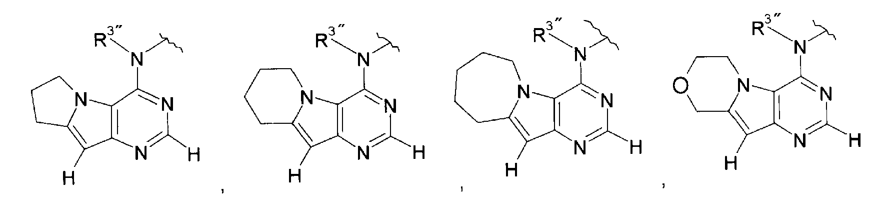

wherein each symbol is as defined above, and the like.

R^{2"} and R^{3"} are optionally bonded to each other to form an optionally substituted ring structure. Examples of the "ring structure" include a saturated or unsaturated (preferably saturated) 4- to 8-membered (preferably 5- to 7-membered) heterocycle.

Examples of the "ring structure" of the "optionally substituted ring structure" formed by R^{2"} and R^{3"} bonded to each other include

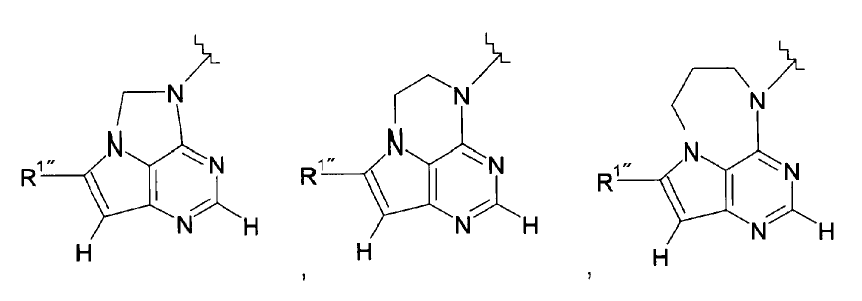

wherein each symbol is as defined above, and the like.

The "ring structure" of the "optionally substituted ring structure" formed by R^{1"} and R^{2"}, or R^{2"} and R^{3"} bonded respectively, optionally has 1 to 5 (preferably 1 to 3, more preferably 1 or 2), the same or different substituents at any substitutable positions. Examples of the substituent include substituents selected from substituent group V. When the number of the substituents is not less than 2, the respective substituents may be the same or different.

Examples of the "ring structure" of the "optionally substituted ring structure" formed by R^{3"} bonded to the carbon atom on the adjacent benzene ring (ring A") include a saturated or unsaturated (preferably saturated) 4- to 8-membered (preferably 5- or 6-membered) nitrogen-containing heterocycle.

Specifically, the moiety of

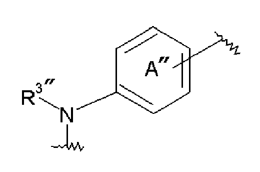

wherein each symbol is as defined above, is, for example,

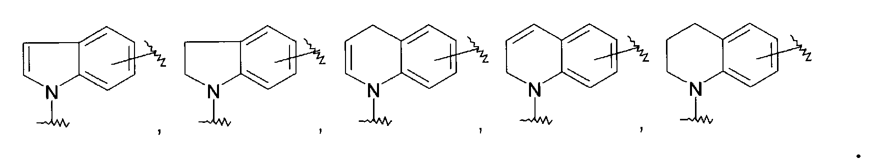

The "ring structure" optionally has 1 to 5 (preferably 1 to 3, more preferably 1 or 2), the same or different substituents at any substitutable positions. Examples of the substituent include substituents selected from substituent group V. When the number of the substituents is not less than 2, the respective substituents may be the same or different.

Preferable compounds of compound (I") are as follows.

### [Compound A]

Compound (I") wherein
R^{1"} is a hydrogen atom, a halogen atom or cyano;
R^{2"} is a hydrogen atom or optionally substituted alkyl;
R^{3"} is a hydrogen atom; ring A^{"} is a benzene ring optionally substituted by 1 or 2 substituents selected from the group consisting of (1) a halogen atom and (2) C₁₋₄ alkyl; and
ring B is
(1) a fused ring optionally substituted by substituent selected from the group consisting of
   (a) a halogen atom,
   (b) cyano,
   (c) C₁₋₆ alkyl optionally substituted by halogen atom or C₃₋₆ cycloalkyl,
   (d) oxo,
   (e) C₂₋₄ alkylene,
   (f) hydroxy,
   (g) carbamoyl,
   (h) C₁₋₆ alkyl-carbamoyl, and
   (i) C₁₋₆ alkoxy-carbonyl, or
(2) a pyridine ring having carbamoyl optionally substituted by C₁₋₆ alkyl (said pyridine ring is optionally further substituted by C₁₋₆ alkyl).

### [Compound B]

Compound (I") wherein
R^{1"} is a hydrogen atom, a halogen atom or cyano;
R^{2"} is
(1) a hydrogen atom, or
(2) C₁₋₆ alkyl optionally substituted by 1 to 3 substituents selected from the group consisting of
   (a) C₃₋₆ cycloalkyl,
   (b) -O-(CH₂)ₙ-OH,
   (c) -NR^{5"}-(CH₂)ₙ-OH,
   (d) -NR^{5"}-CO- (C₁₋₄ alkyl optionally substituted 1 to 4 substituent selected from hydroxy, halogen atom, cyano, C₁₋₄ alkoxy, amino and di-C₁₋₄ alkylamino),
   (e) -NR^{5"}-CO-(CH₂)ₙ-SO₂-C₁₋₄ alkyl,
   (f) -NR^{5"}-CO- (5- or 6-membered heterocycle optionally substituted by 1 or 2 C₁₋₄ alkyl),
   (g) -NR^{5"}-CO-(CH₂)ₙ- (6-membered heterocycle optionally substituted by C₁₋₄ alkyl),
   (h) -NR^{5"}-CO- (C₃₋₆ cycloalkyl optionally substituted by substituent(s) selected from hydroxy and cyano),
   (i) -NR^{5"}-CO- (C₆₋₁₀ aryl optionally substituted by C₁₋₄ alkyl optionally substituted by 1 to 3 halogen atoms),
   (j) -NR^{5"}-CO-NR^{5"'}-C3-6 cycloalkyl,
   (k) -NR^{5"}-SO₂-C₁₋₄ alkyl,
   (l) C₁₋₄ alkyl-carbonyl,
   (m) (5- or 6-membered heterocycle optionally substituted by 1 or 2 substituents selected from hydroxy and amino)-carbonyl,
   (n) hydroxy,
   (o) halogen atom, and
   (p) 3- to 6-membered cyclic amino optionally substituted by 1 to 3 substituents selected from C₁₋₄ alkyl and oxo,
      wherein n is an integer of 1 to 4, R^{5"} and R^{5"'} are each a hydrogen atom or C₁₋₄ alkyl, -(CH₂)ₙ- is optionally substituted by C₁₋₄ alkyl);
R^{3"} is a hydrogen atom; ring A^{"} is a benzene ring optionally substituted by 1 or 2 substituents selected from the group consisting of (1) a halogen atom and (2) C₁₋₄ alkyl; and
ring B is
(1) a fused homocyclic ring optionally having, as substituent, 1 or 2 substituents selected from the group consisting of (a) C₁₋₆ alkyl optionally substituted by halogen atom, (b) hydroxy and (c) oxo;
(2) fused heterocycle optionally having, as substituent, 1 to 4 substituents selected from the group consisting of (a) a halogen atom, (b) cyano, (c) C₁₋₆ alkyl optionally substituted by halogen atom or C₃₋₆ cycloalkyl, (d) oxo, (e) C₂₋₄ alkylene, (f) carbamoyl, (g) C₁₋₆ alkyl-carbamoyl, and (h) C₁₋₆ alkoxy-carbonyl; or
(3) a pyridine ring having carbamoyl optionally substituted by C₁₋₈ alkyl (said pyridine ring is optionally further substituted by C₁₋₆ alkyl).

### [Compound C]

Compound (I") wherein
R^{1"} is a hydrogen atom, a halogen atom or cyano;
R^{2"} is
(1) a hydrogen atom, or
(2) C₁₋₆ alkyl optionally substituted by 1 to 3 substituents selected from the group consisting of
   (a) C₃₋₆ cycloalkyl,
   (b) -O-(CH₂)ₙ-OH,
   (c) -NR^{5"}-(CH₂)ₙ-OH,
   (d) -NR^{5"}-CO- (C₁₋₄ alkyl optionally substituted by 1 to 4 substituent selected from hydroxy, halogen atom, cyano, C₁₋₄ alkoxy, amino and di-C₁₋₄ alkylamino),
   (e) -NR^{5"}-CO-(CH₂)ₙ-SO₂-C₁₋₄ alkyl,
   (f) -NR^{5"}-CO- (5- or 6-membered heterocycle optionally substituted by 1 or 2 C₁₋₄ alkyl),
   (g) -NR^{5"}-CO-(CH₂)ₙ- (6-membered heterocycle optionally substituted by C₁₋₄ alkyl),
   (h) -NR^{5"}-CO- (C₃₋₆ cycloalkyl optionally substituted by substituent(s) selected from hydroxy and cyano),
   (i) -NR^{5"}-CO- (C₆₋₁₀ aryl optionally substituted by C₁₋₄ alkyl optionally substituted by 1 to 3 halogen atoms),
   (j) -NR^{5"}-CO-NR5^{"'}-C₃₋₆ cycloalkyl,
   (k) -NR^{5"}-SO₂-C₁₋₄ alkyl,
   (l) C₁₋₄ alkyl-carbonyl,
   (m) (5- or 6-membered heterocycle optionally substituted by 1 or 2 substituents selected from hydroxy and amino)-carbonyl,
   (n) hydroxy,
   (o) halogen atom, and
   (p) 3- to 6-membered cyclic amino optionally substituted by 1 to 3 substituents selected from C₁₋₄ alkyl and oxo, wherein n is an integer of 1 to 4, R⁵ and R^{5'} are each a hydrogen atom or C₁₋₄ alkyl, -(CH₂)ₙ- is optionally substituted by C₁₋₄ alkyl;
R^{3"} is a hydrogen atom;
ring A^{"} is a benzene ring optionally substituted by 1 or 2 substituents selected from the group consisting of (1) a halogen atom and (2) C₁₋₄ alkyl; and
ring B^{"} is
(1) indole optionally having one C₁₋₄ alkyl;
(2) pyrrolopyrimidine optionally having one C₁₋₄ alkyl;
(3) imidazopyridine;
(4) dihydroindole optionally having 1 or 2 substituents selected from the group consisting of (a) C₁₋₄ alkyl optionally substituted by C₃₋₆ cycloalkyl, (b) a halogen atom, (c) C₂₋₄ alkylene and (d) oxo;
(5) dihydroisoindole optionally having 1 to 4 substituents selected from the group consisting of (a) C₁₋₄ alkyl, (b) a halogen atom and (c) oxo;
(6) dihydrobenzoxazole optionally having 1 or 2 substituents selected from the group consisting of C₁₋₄ alkyl and oxo;
(7) pyrrolopyridine;
(8) indane optionally having 1 or 2 substituents selected from
   (a) C₁₋₆ alkyl optionally substituted by 1 to 3 halogen atoms,
   (b) hydroxy and (c) oxo;
(9) benzothiophene;
(10) indazole optionally having one substituent selected from the group consisting of a halogen atom and C₁₋₄ alkyl;
(11) dihydrobenzofuran;
(12) quinoline optionally having one substituent selected from the group consisting of (a) a halogen atom and (b) C₁₋₆ alkyl optionally substituted by 1 to 3 halogen atoms;
(13) benzooxazole;
(14) benzofuran optionally having one substituent selected from the group consisting of (a) cyano and (b) carbamoyl;
(15) benzoisoxazole optionally having one C₁₋₄ alkyl;
(16) pyrazolopyridine optionally having one substituent selected from the group consisting of (a) C₁₋₆ alkoxy-carbonyl and (b) C₁₋₆ alkyl-carbamoyl;
(17) benzoimidazole optionally having 1 or 2 C₁₋₄ alkyl;
(18) triazolopyridine;
(19) naphthalene; or
(20) pyridine substituted by C₁₋₈ alkyl-carbamoyl and optionally further substituted by C₁₋₆ alkyl.

As the salts of the compound (I"), for example, metal salt, ammonium salt, salts with organic base, salts with inorganic acid, salts with organic acid, salts with basic or acidic amino acid and the like can be mentioned.

As preferable examples of the metal salt, for example, alkali metal salts such as sodium salt, potassium salt and the like; alkaline earth metal salts such as calcium salt, magnesium salt, barium salt and the like; aluminum salt and the like can be mentioned.

As preferable examples of the salts with organic base, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like can be mentioned.

As preferable examples of salts with inorganic acid, for example, salts with hydrochloric acid, hydrobromic acid, nitric acid, sulfuric acid, phosphoric acid and the like can be mentioned.

As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned.

As preferable examples of the salts with basic amino acid, for example, salts with arginine, lysine, ornithine and the like can be mentioned.

As preferable examples of the salts with acidic amino acid, for example, salts with aspartic acid, glutamic acid and the like can be mentioned.

Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like can be mentioned.

Among the above-mentioned compounds (I) - (I"), a HER2 inhibitor most preferable for the prophylaxis or treatment of a HER2 inhibitor (trastuzumab and the like)-resistant cancer is N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide (compound A).

Compound (I) and a salt thereof can be produced according to the method described in W02005/118588.
Compound (I') and a salt thereof can be produced according to the method described in W02007/064045.
Compound (I") and a salt thereof can be produced according to the method described in W02007/073879.

When compound (I) - (I") has isomers such as optical isomer, stereoisomer, positional isomer, rotational isomer and the like, and any isomers and mixtures are encompassed in the compound (I) - (I"). For example, when compound (I) - (I") has an optical isomer, an optical isomer separated from a racemate is also encompassed in the compound (I) - (I"). These isomers can be obtained as independent products by a synthesis means or a separation means (concentration, solvent extraction, column chromatography, recrystallization and the like) known *per se.*

Compounds (I) - (I") may be a crystal, and both a single crystal and crystal mixtures are encompassed in the compound (I) - (I"). Crystals can be produced by crystallization according to crystallization methods known *per se.*

Compounds (I) - (I") may be a solvate (e.g., hydrate etc.) or a non-solvate, both of which are encompassed in the compound (I) - (I").

A compound labeled with an isotope (e.g., ³H, ¹⁴C, ³⁵S, ¹²⁵I and the like) is also encompassed in the compound (I) - (I") .

A prodrug of the compound (I) - (I") or a salt thereof (hereinafter referred to as compound (I) - (I")) means a compound which is converted to the compound (I) - (I") with a reaction due to an enzyme, an gastric acid, etc. under the physiological condition in the living body, that is, a compound which is converted to the compound (I) - (I") with oxidation, reduction, hydrolysis, etc. according to an enzyme; a compound which is converted to the compound (I) - (I") by hydrolysis etc. due to gastric acid, etc. A prodrug for compound (I) - (I") may be a compound obtained by subjecting an amino group in compound (I) - (I") to an acylation, alkylation or phosphorylation (e.g., a compound obtained by subjecting an amino group in compound (I) - (I") to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) - (I") to an acylation, alkylation, phosphorylation or boration (e.g., a compound obtained by subjecting an hydroxy group in compound (I) - (I") to an acetylation, palmitoylation, propanoylation, pivaloylation, succinylation, fumarylation, alanylation, dimethylaminomethylcarbonylation, etc.); a compound obtained by subjecting a carboxyl group in compound (I) - (I") to an esterification or amidation (e.g., a compound obtained by subjecting a carboxyl group in compound (I) - (I") to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Any of these compounds can be produced from compound (I) - (I") by a method known *per se.*

A prodrug of compound (I) - (I") may also be converted to compound (I) - (I") under physiological conditions, such as those described in IYAKUHIN no KAIHATSU (Development of Pharmaceuticals), Vol. 7, Design of Molecules, p.163-198, Published by HIROKAWA SHOTEN (1990).

The "combination drug comprising (1) a HER2 inhibitor and (2) one or more medicaments selected from a cofilin inhibitor, a PAK1 inhibitor, a LIMK inhibitor, a RHO inhibitor, a ROCK1 inhibitor and a ROCK2 inhibitor" of the present invention means a concomitant or combination agent of two or more medicaments.

The "combination drug comprising (1) a HER2 inhibitor and (2) one or more medicaments selected from a cofilin inhibitor, a PAK1 inhibitor, a LIMK inhibitor, a RHO inhibitor, a ROCK1 inhibitor and a ROCK2 inhibitor" of the present invention includes both a single preparation comprising (1) a HER2 inhibitor and (2) one or more medicaments selected from a cofilin inhibitor, a PAK1 inhibitor, a LIMK inhibitor, a Rho inhibitor, a ROCK1 inhibitor and a ROCK2 inhibitor, and separate preparations each comprising (1) a HER2 inhibitor or (2) one or more medicaments selected from a cofilin inhibitor, a PAK1 inhibitor, a LIMK inhibitor, a Rho inhibitor, a ROCK1 inhibitor and a ROCK2 inhibitor. These are generically abbreviated as the combination drug of the present invention.
The combination drug of the present invention can be formulated into a preparation according to a method similar to that for the aforementioned agent for preventing or treating cancer of the present invention by directly using (1) a HER2 inhibitor and (2) one or more medicaments selected from a cofilin inhibitor, a PAK1 inhibitor, a LIMK inhibitor, a Rho inhibitor, a ROCK1 inhibitor and a ROCK2 inhibitor or by mixing them with a pharmaceutically acceptable carrier and the like separately or simultaneously. The daily dose of the combination drug of the present invention varies depending on the severity of the symptom, age, sex, body weight and sensitivity difference of the subject, period and interval of administration, and properties, dosage form and kind of the pharmaceutical composition, kind of effective ingredient, and the like, and is not particularly restricted. The dose of each of (1) a HER2 inhibitor and (2) one or more medicaments selected from a cofilin inhibitor, a PAK1 inhibitor, a LIMK inhibitor, a Rho inhibitor, a ROCK1 inhibitor and a ROCK2 inhibitor is any as long as the side effects thereof are not problematic, and is not particularly limited. For oral administration, the dose is generally about 0.005 - 100 mg, preferably about 0.05 - 50 mg, more preferably about 0.2 - 30 mg, per 1 kg of a mammal, which is normally administered in one to 3 portions a day.
For administration of the combination drug of the present invention, (1) a HER2 inhibitor and (2) one or more medicaments selected from a cofilin inhibitor, a PAK1 inhibitor, a LIMK inhibitor, a Rho inhibitor, a ROCK1 inhibitor and a ROCK2 inhibitor may be administered simultaneously, or (1) a HER2 inhibitor may be administered earlier and then (2) one or more medicaments selected from a cofilin inhibitor, a PAK1 inhibitor, a LIMK inhibitor, a Rho inhibitor, a ROCK1 inhibitor and a ROCK2 inhibitor may be administered, or (1) one or more medicaments selected from a cofilin inhibitor, a PAK1 inhibitor, a LIMK inhibitor, a Rho inhibitor, a ROCK1 inhibitor and a ROCK2 inhibitor may be administered earlier and then (2) a HER2 inhibitor may be administered. In administration in a staggered manner, the time difference varies depending on the active ingredient to be administered, dosage form and administration method. For example, when a HER2 inhibitor is to be administered earlier, a method including administering one or more medicaments selected from a cofilin inhibitor, a PAK1 inhibitor, a LIMK inhibitor, a Rho inhibitor, a ROCK1 inhibitor and a ROCK2 inhibitor within one min - 3 days, preferably 10 min - 1 day, more preferably 15 min - 1 hr, after administration of the HER2 inhibitor can be employed. When one or more medicaments selected from a cofilin inhibitor, a PAK1 inhibitor, a LIMK inhibitor, a Rho inhibitor, a ROCK1 inhibitor and a ROCK2 inhibitor is/are to be administered earlier, a method including administering a HER2 inhibitor within one min - 1 day, preferably 10 min - 6 hr, more preferably 15 min - 1 hr, after administration of one or more medicaments selected from a cofilin inhibitor, a PAK1 inhibitor, a LIMK inhibitor, a Rho inhibitor, a ROCK1 inhibitor and a ROCK2 inhibitor can be employed.
In the "combination drug of the present invention comprising (1) a HER2 inhibitor and (2) one or more medicaments selected from a cofilin inhibitor, a PAK1 inhibitor, a LIMK inhibitor, a RHO inhibitor, a ROCK1 inhibitor and a ROCK2 inhibitor", the content of each of the HER2 inhibitor and one or more medicaments selected from a cofilin inhibitor, a PAK1 inhibitor, a LIMK inhibitor, a Rho inhibitor, a ROCK1 inhibitor and a ROCK2 inhibitor varies depending on the form of the preparation. It is generally about 0.01 - 90 wt%, preferably about 0.1 - 50 wt%, more preferably about 0.5 - 20 wt%, relative to the whole preparation.
The content of the carrier in the combination drug is generally about 0 - 99.8 wt%, preferably about 10 - 99.8 wt%, more preferably about 10 - 90 wt%, relative to the whole preparation.
In the combination drug of the present invention separately comprising (1) a HER2 inhibitor and (2) one or more medicaments selected from a cofilin inhibitor, a PAK1 inhibitor, a LIMK inhibitor, a Rho inhibitor, a ROCK1 inhibitor and a ROCK2 inhibitor, the pharmaceutical composition containing a HER2 inhibitor can be produced and used in the same manner as in the agent for preventing or treating cancer of the present invention.

The present invention also provides a method of examining the sensitivity of a HER2-expressing cancer to a HER2 inhibitor, comprising measuring the expression or activation state of one or more selected from cofilin, PAK1, LIMK, RHO, ROCK1 and ROCK2 in a sample collected from an animal having the cancer. The sample is, for example, a cancer tissue. As a control, an animal-derived sample known to be highly sensitive to a HER2 inhibitor can be used. The expression and activation states of cofilin, PAK1, LIMK, Rho, ROCK1 and ROCK2 can be measured by a method similar to the screening method of the present invention mentioned above. By comparison of the measurement values of test animal and control animal, when the expression or activation state of one or more selected from cofilin, PAK1, LIMK, Rho, ROCK1 and ROCK2 shows a significant increase in the test animal than in the control animal, the test animal can be judged to highly possibly show decreased sensitivity to the HER2 inhibitor.

The present invention also provides a method of treating cancer, which comprises using HER2 inhibitor and one or more medicaments selected from a cofilin inhibitor, a PAK1 inhibitor, a LIMK inhibitor, a Rho inhibitor, a ROCK1 inhibitor and a ROCK2 inhibitor in combination for an animal judged to highly possibly show decreased sensitivity to a HER2 inhibitor by the above-mentioned examination method. The detail of the combined use of a HER2 inhibitor and one or more medicaments selected from a cofilin inhibitor, a PAK1 inhibitor, a LIMK inhibitor, a Rho inhibitor, a ROCK1 inhibitor and a ROCK2 inhibitor is as described above.

A HER2 inhibitor comprising compounds (I) - (I") (particularly compound A) (hereinafter sometimes to be referred to as "the HER2 inhibitor of the present invention") or a salt thereof or a prodrug thereof is useful as a therapeutic agent that suppresses growth of a trastuzumab-resistant cancer expressing HER2 and/or EGFR kinase, since it inhibits HER2 kinase and/or EGFR kinase. The HER2 inhibitor of the present invention can be used for the prophylaxis or treatment of a trastuzumab-resistant cancer not only when it is combined with one or more medicaments selected from a cofilin inhibitor, a PAK1 inhibitor, a LIMK inhibitor, a Rho inhibitor, a ROCK1 inhibitor and a ROCK2 inhibitor, but also when it is used singly.

In particular, of the HER2 inhibitors of the present invention, a trastuzumab-resistant cancer can be prevented or treated extremely effectively by using N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide (compound A) alone (see the below-mentioned Example 7).

Lapatinib can also prevent or treat a trastuzumab-resistant cancer not only when it is combined with one or more medicaments selected from a cofilin inhibitor, a PAK1 inhibitor, a LIMK inhibitor, a Rho inhibitor, a ROCK1 inhibitor and a ROCK2 inhibitor, but also when it is used singly, as in the case of the above-mentioned HER2 inhibitor of the present invention (see the below-mentioned Example 8).

The HER2 inhibitor of the present invention and lapatinib are useful as medicaments since they show low toxicity (e.g., acute toxicity, chronic toxicity, genetic toxicity, reproductive toxicity, cardiotoxicity, drug interaction, carcinogenicity and the like), high water solubility, and superior stability, in vivo kinetics (absorbability, distribution, metabolism, excretion and the like) and efficacy expression.

The HER2 inhibitor of the present invention or Lapatinib can prevent or treat HER2 positive cancers including various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colon cancer (e.g., gastrointestinal stromal tumor etc.), rectal cancer (e.g., gastrointestinal stromal tumor etc.), colorectal cancer (e.g., familial colorectal cancer, hereditary nonpolyposis colorectal cancer, gastrointestinal stromal tumor etc.), small intestinal cancer (e.g., non-Hodgkin lymphoma, gastrointestinal stromal tumor, etc.), esophagus cancer, duodenal cancer, cancer of the tongue, pharyngeal cancer (e.g., nasopharyngeal carcinoma, oropharyngeal cancer, hypopharyngeal cancer etc.), salivary gland cancer, brain tumor (e.g., pineal astrocytoma, pilocytic astrocytoma, diffuse astrocytoma, anaplastic astrocytoma etc.), schwannoma, liver cancer (e.g., primary liver cancer, extrahepatic bile duct cancer etc.), kidney cancer (e.g., renal cell carcinoma, transitional cell cancers of renal pelvis and ureter etc.), biliary tract cancer, endometrial carcinoma, carcinoma of the uterine cervix, ovary cancer (e.g., ovarian epithelial cancer, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), Hemangioma, malignant lymphoma, malignant melanoma, thyroid cancer (e.g., medullary thyroid carcinoma etc.), parathyroid cancer, nasal cavity cancer, paranasal sinus cancer, bone tumor (e.g., osteosarcoma, Ewing's tumor, uterus sarcoma, soft tissue sarcoma etc.), vascular fibroma, retinoblastoma, penile cancer, testis tumor, solid cancer in childhood (e.g., Wilms' tumor, childhood kidney tumor, etc.), Kaposi's sarcoma, Kaposi's sarcoma related to AIDS, maxillary tumor, fibrous histiocytoma, leiomyosarcoma, rhabdomyosarcoma, leukemia (e.g., acute myelocytic leukemia, acute lymphoblastic leukemia etc.) etc.), and the like. The HER2 inhibitor of the present invention or lapatinib is particularly useful for the prophylaxis or treatment of breast cancer, prostate cancer, gastric cancer, lung cancer, colon cancer, rectal cancer, colorectal cancer, esophagus cancer, pharyngeal cancer, ovary cancer, urinary bladder cancer and the like.

The HER2 inhibitor of the present invention or lapatinib can prevent or treat these cancers even by administration without combination with one or more medicaments selected from a cofilin inhibitor, a PAK1 inhibitor, a LIMK inhibitor, a Rho inhibitor, a ROCK1 inhibitor and a ROCK2 inhibitor (namely, as a single agent/alone).

The HER2 inhibitor of the present invention or lapatinib can be directly used as it is as a medicament or as a pharmaceutical composition containing a pharmaceutically acceptable carrier known *per se* etc. for a mammal (e.g., human, horse, bovine, dog, cat, rat, mouse, rabbit, swine, monkey etc.). A pharmaceutical composition to be used for administration may contain the HER2 inhibitor of the present invention or lapatinib, and a pharmacologically acceptable carrier, diluent or excipient.

The HER2 inhibitor of the present invention or lapatinib can be safely administered orally or parenterally (e.g., topical, rectal, intravenous administrations etc.) as a single agent, or a pharmaceutical composition containing a pharmacologically acceptable carrier according to a conventional method (e.g., a method described in the Japanese Pharmacopoeia etc.), such as tablet (including sugar-coated tablet, film-coated tablet), powder, granule, capsule, liquid, emulsion, suspension, injection, suppository, sustained release preparation, plaster and the like.

As a medicament for mammals such as humans, the HER2 inhibitor of the present invention or lapatinib can be generally administered orally in the form of, for example, tablets, capsules (including soft capsules and microcapsules), powders, granules and the like, or parenterally in the form of injections, suppositories, pellets and the like. Examples of the "parenteral administration route" include intravenous, intramuscular, subcutaneous, intra-tissue, intranasal, intradermal, instillation, intracerebral, intrarectal, intravaginal, intraperitoneal and intratumoral administrations, as well as administration to the vicinity and the like of tumor, or directly to the lesion.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. The injection can be prepared by, for example, dissolving, suspending or emulsifying the above-mentioned HER2 inhibitor of the present invention or lapatinib in a sterile aqueous or oily solution normally used for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with solubilizers such as benzyl benzoate, benzyl alcohol. The injectable preparation prepared is preferably filled in an appropriate ampoule. A suppository used for rectal administration may also be prepared by mixing the above-mentioned HER2 inhibitor of the present invention or lapatinib in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a commonly known method, and may contain a carrier, diluent or filler normally used in the field of pharmaceutical production. As the carrier or filler for tablets, for example, lactose, starch, sucrose, and magnesium stearate are used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned.
While the content of the HER2 inhibitor of the present invention or Lapatinib varies depending on the form of the preparation, it is generally about 0.01 - 100 wt%, preferably about 0.1 - 50 wt%, more preferably about 0.5 - 20 wt%, of the whole preparation.

The HER2 inhibitor of the present invention or lapatinib can be administered orally or parenterally as they are, or in the form of a solid agent such as powder, fine granules, granules, tablet, capsule and the like or a liquid such as injection and the like by mixing with an appropriate pharmaceutically acceptable carrier, such as excipient (e.g., starch, lactose, sucrose, calcium carbonate, calcium phosphate and the like), binder (e.g., starch, gum arabic, carboxymethylcellulose, hydroxypropylcellulose, crystalline cellulose, alginic acid, gelatin, polyvinylpyrrolidone and the like), lubricant (for example, stearic acid, magnesium stearate, calcium stearate, talc and the like), disintegrant (e.g., calcium carboxymethylcellulose, talc and the like), diluent (e.g., water for injection, saline and the like), additive (stabilizer, preservative, colorant, flavor, solubilizer, emulsifier, buffer agent, isotonicity agent and the like) as necessary and the like by a conventional method.
The content of the carrier in the preparation is generally about 0 - 99.9 wt%, preferably about 10 - 99.9 wt%, more preferably about 10 - 90 wt%, of the whole preparation.

The dose of the HER2 inhibitor of the present invention or Lapatinib varies depending on the kind thereof, level of symptoms, age, sex, body weight and sensitivity difference of the subject of administration, timing of administration, administration route, administration intervals, properties, dosage form and kind of the pharmaceutical composition, and the like. Examples are shown in the following.

While the dose of the HER2 inhibitor of the present invention (i.e., compounds (I) - (I")) varies depending on the administration route, symptom and the like, for example, for oral administration to a patient (body weight 40 - 80 kg) with breast cancer or prostate cancer as an anti-cancer agent, the dose is, for example, 0.5 - 300 mg/kg body weight/day, preferably 0.5 - 100 mg/kg body weight/day, more preferably 1 - 50 mg/kg body weight/day, further preferably 1 - 25 mg/kg body weight/day, which can be administered once or in 2 or 3 portions a day.

Particularly when compound A is used from among the HER2 inhibitors of the present invention, the dose varies depending on the administration route, symptom and the like, for example, for oral administration to a patient (body weight 40 - 80 kg) with breast cancer or prostate cancer as an anti-cancer agent, the dose is, for example, 0.5 - 300 mg/kg body weight/day, preferably 1 - 250 mg/kg body weight/day, more preferably 10 - 200 mg/kg body weight/day, which can be administered once or in 2 or 3 portions a day.

While the dose of lapatinib varies depending on the administration route, symptom and the like, for example, for oral administration to a patient (body weight 40 - 80 kg) with breast cancer or prostate cancer as an anti-cancer agent, the dose is, for example, 1000 - 1500 mg/day, preferably 1200 - 1300 mg/day, generally 1250 mg/day, which can be administered once or in 2 or 3 portions a day. Generally, the amount is administered once a day.

In the case of other parenteral administrations and oral administration, a dose based thereon can be administered. When the symptom is particularly severe, the dosage may be increased depending on the symptom.

For administration of a sustained-release preparation, the dose of the HER2 inhibitor of the present invention or Lapatinib varies depending on the kind and content thereof, dosage form, duration of sustained drug release, animal as administration subject and administration object. For example, in the case of parenteral administration, about 0.1 to about 100 mg of the HER2 inhibitor of the present invention or Lapatinib only needs to be released from the administered preparation in one week.

As long as an unpreferable interaction is not caused by blending the HER2 inhibitor of the present invention or lapatinib, a pharmaceutical composition may contain the HER2 inhibitor of the present invention or Lapatinib along with other active ingredients, for example, the following hormonal therapeutic agent, anti-cancer agent (e.g., chemotherapeutic agent, immunotherapeutic agent, or medicament that inhibits the action of cell growth factor and cell growth factor receptor etc.) and the like.

(1) Administration of an effective amount of the HER2 inhibitor of the present invention or lapatinib and (2) a combination of 1 to 3 selected from the group consisting of (i) administering an effective amount of other anticancer agents, (ii) administering an effective amount of hormonal therapeutic agents and (iii) non-drug therapy can prevent and/or treat cancer more effectively. As the non-drug therapy, for example, surgery, radiotherapy, gene therapy, thermotherapy, cryotherapy, laser cauterization and the like are exemplified and two or more of these may be combined.

For example, the HER2 inhibitor of the present invention or lapatinib can be administered simultaneously with other hormonal therapeutic agents, anticancer agents (e.g., chemotherapeutic agents, immunotherapeutic agents, or medicaments inhibiting the action of cell growth factors or cell growth factor receptors) (hereafter, these are referred to as a concomitant drug).

The HER2 inhibitor of the present invention or Lapatinib as a single agent shows a superior anticancer action on a trastuzumab-resistant cancer. Such effect can be enhanced by using it in combination with one or more of the concomitant drug(s) mentioned above (multi-agent co-administration).

Examples of the "hormonal therapeutic agents" include fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogens (e.g., tamoxifen citrate, toremifene citrate, and the like), ER down-regulator (e.g., fulvestrant, and the like), human menopausal gonadotrophin, follicle stimulating hormone, pill preparations, mepitiostane, testrolactone, aminoglutethimide, LH-RH agonists (e.g., goserelin acetate, buserelin, leuprorelin, and the like), droloxifene, epitiostanol, ethinylestradiol sulfonate, aromatase inhibitors (e.g., fadrozole hydrochloride, anastrozole, retrozole, exemestane, vorozole, formestane, and the like), anti-androgens (e.g., flutamide, bicartamide, nilutamide, and the like), 5α-reductase inhibitors (e.g., finasteride, dutasteride, epristeride, and the like), adrenocorticohormone drugs (e.g., dexamethasone, prednisolone, betamethasone, triamcinolone, and the like), androgen synthesis inhibitors (e.g., abiraterone, and the like), retinoid and drugs that retard retinoid metabolism (e.g., liarozole, and the like), etc. and LH-RH agonists (e.g., goserelin acetate, buserelin, leuprorelin etc.) are preferable.

As examples of said "chemotherapeutic agents", there may be mentioned alkylating agents, antimetabolites, anticancer antibiotics, plant-derived anticancer agents, and the like.

Examples of the "alkylating agents" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and the like.

Examples of the "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, and the like.

Examples of the "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, and the like.

Examples of the "plant-derived anticancer agents" include etoposide, etoposide phosphate, vinblastine sulfate, vincristine sulfate, vindesine sulfate, teniposide, paclitaxel, docetaxel, vinorelbine, and the like.

Examples of said "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, *Corynebacterium parvum,* levamisole, polysaccharide K, procodazole, and the like.

As the "growth factor" in said "medicaments inhibiting the action of cell growth factors or receptors", there may be mentioned any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin (HER2 ligand), and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., acidic FGF, basic FGF, KGF (keratinocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), and the like], and the like.

Examples of said "growth factor receptors" include any receptors capable of binding to the aforementioned cell growth factors, including EGF receptor, heregulin receptor (HER2), insulin receptor, IGF receptor, FGF receptor-1 or FGF receptor-2, and the like.

Examples of said "medicament that inhibits the action of cell growth factor" include trastuzumab (Herceptin (trade mark); HER2 antibody), imatinib mesylate, ZD1839 or cetuximab, antibody against VEGF (e.g., bevacizumab), antibody against VEGF receptor, gefitinib, erlotinib and the like.

In addition to the aforementioned drugs, L-asparaginase, aceglatone, procarbazine hydrochloride, protoporphyrin-cobalt complex salt, mercuric hematoporphyrin-sodium, topoisomerase I inhibitors (e.g., irinotecan, topotecan, and the like), topoisomerase II inhibitors (e.g., sobuzoxane, and the like), differentiation inducers (e.g., retinoid, vitamin D, and the like), angiogenesis inhibitors (e.g., thalidomide, SU11248, and the like), α-blockers (e.g., tamsulosin hydrochloride, naftopidil, urapidil, alfuzosin, terazosin, prazosin, silodosin, and the like) serine/threonine kinase inhibitor, endothelin receptor antagonist (e.g., atrasentan, and the like), proteasome inhibitor (e.g., bortezomib, and the like), Hsp 90 inhibitor (e.g., 17-AAG, and the like), spironolactone, minoxidil, 11α-hydroxyprogesterone, bone resorption inhibiting/metastasis suppressing agent (e.g., zoledronic acid, alendronic acid, pamidronic acid, etidronic acid, ibandronic acid, clodronic acid) and the like can be used.

While the HER2 inhibitor of the present invention or Lapatinib can be concomitantly used with trastuzumab, it is most preferably used in place of trastuzumab against cancer identified to be resistant to trastuzumab by the method of examining the sensitivity of HER2 expression cancer.

The present invention is explained in more detail in the following by referring Examples, which are mere examples and do not at all limit the scope of the present invention.

### Examples

### Example 1

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. LIMK1 gene was knocked down by the following RNAi method. Three kinds of Sthealth RNAis (LIMK1 Stealth Select 3 RNAi, Invitrogen, HSS140837:HSS140836:HSS141043) corresponding to LIMK1 gene and Lipofectamin RNAiMAX (Invtrogen, 13778-150) were mixed with Opti-MEM(R) I Reduced-Serum Medium (Invitrogen, 31985-070) to 36.7 nM x3 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 70% in the high sensitive cell line and about 30% in the low sensitive cell line by the addition of trastuzumab (3 µg/mL) as compared to without addition of trastuzumab. In contrast, with knockdown of LIMK1 by LIMK1 siRNA, the growth was suppressed by about 80% in the low sensitive cell line, and the growth was completely suppressed in the high sensitive cell line. Therefrom it was revealed that inhibition of LIMK1 enhances the sensitivity to trastuzumab.

### Example 2

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. PAK1 gene was knocked down by the following RNAi method. Two kinds of Sthealth RNAis (PAK1 Validated Stealth RNAi DuoPac, Invitrogen, 45-1676) corresponding to PAK1 gene and Lipofectamin RNAiMAX (Invtrogen, 13778-150) were mixed with Opti-MEM(R) I Reduced-Serum Medium (Invitrogen, 31985-070) to 55 nM x2 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 70% in the high sensitive cell line and about 30% in the low sensitive cell line by the addition of trastuzumab (3 µg/mL) as compared to without addition of trastuzumab. In contrast, with knockdown of PAK1 by PAK1 siRNA, the growth was suppressed by about 50% in the low sensitive cell line, and the growth was completely suppressed in the high sensitive cell line. Therefrom it was revealed that inhibition of PAK1 enhances the sensitivity to trastuzumab.

### Example 3

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. Cofilin1 gene was knocked down by the following RNAi method. Three kinds of Sthealth RNAis (CFL Stealth Select 3 RNAi, Invitrogen, HSS141559:HSS141560:HSS141561) corresponding to cofilin1 gene and Lipofectamin RNAiMAX (Invtrogen, 13778-150) were mixed with Opti-MEM(R) I Reduced-Serum Medium (Invitrogen, 31985-070) to 36.7 nM x3 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 70% in the high sensitive cell line and about 30% in the low sensitive cell line by the addition of trastuzumab (3 µg/mL) as compared to without addition of trastuzumab. In contrast, with knockdown of cofilin1 by cofilin1 siRNA, the growth was suppressed by about 80% in the high sensitive cell line and by about 50% in the low sensitive cell line. Therefrom it was revealed that inhibition of cofilin1 enhances the sensitivity to trastuzumab.

### Example 4

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. The gene was knocked down by the following RNAi method. Three kinds of Sthealth RNAis (RHO Stealth Select 3 RNAi, Invitrogen, HSS140837:HSS140836:HSS141043) corresponding to Rho gene and Lipofectamin RNAiMAX (Invtrogen, 13778-150) were mixed with Opti-MEM(R) I Reduced-Serum Medium (Invitrogen, 31985-070) to 36.7 nM x3 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 40% in the high sensitive cell line and about 10% in the low sensitive cell line by the addition of trastuzumab (3 µg/mL) as compared to without addition of trastuzumab. In contrast, with knockdown of Rho by Rho siRNA, the growth was suppressed by about 30% in the low sensitive cell line, and by about 70% in the high sensitive cell line. Therefrom it was revealed that inhibition of Rho enhances the sensitivity to the HER2 inhibitor.

### Example 5

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. ROCK1 gene was knocked down by the following RNAi method. Two kinds of Sthealth RNAis (PAK1 Validated Stealth RNAi DuoPac, Invitrogen, 45-1676) corresponding to ROCK1 gene and Lipofectamin RNAiMAX (Invtrogen, 13778-150) were mixed with Opti-MEM(R) I Reduced-Serum Medium (Invitrogen, 31985-070) to 55 nM x2 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 40% in the high sensitive cell line and about 10% in the low sensitive cell line by the addition of trastuzumab (3 µg/mL) as compared to without addition of trastuzumab. In contrast, with knockdown of ROCK1 by ROCK1 siRNA, the growth was suppressed by about 30% in the low sensitive cell line, and by about 75% in the high sensitive cell line. Therefrom it was revealed that inhibition of ROCK1 enhances the sensitivity to the HER2 inhibitor.

### Example 6

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. ROCK2 gene was knocked down by the following RNAi method. Three kinds of Sthealth RNAis (CFL1 Stealth Select 3 RNAi,
Invitrogen, HSS141559:HSS141560:HSS141561) corresponding to ROCK2 gene and Lipofectamin RNAiMAX (Invtrogen, 13778-150) were mixed with Opti-MEM(R) I Reduced-Serum Medium (Invitrogen, 31985-070) to 36.7 nM x3 (total 110 nM) and 11 µL/mL, respectively. After standing for 20 min at room temperature, the Sthealth RNAis were mixed at 1:11 liquid volume with respective BT-474 cell suspensions adjusted to 22,000 cells/mL in the culture medium (final concentration; Stealth RNAis total 10 nM, Lipofectamin RNAiMAX 1 µL/mL, 20,000 cells/mL). As a control, similar operation was performed using Stealth^{™} RNAi Negative Control Duplexes Complete Kit (Invitrogen, 12935-100). Respective BT-474 cells mixed with Stealth RNAis were seeded in a 96 well plate (Nunc) at 3000 cells/150 µL/well and cultured 2-overnights, and trastuzumab (Rosh) was added at 50 µL/well to respective final concentrations. After culture for 5 days at 37°C, 5% CO₂, the number of viable cells was counted.

When treated with control siRNA, the growth was suppressed by about 40% in the high sensitive cell line and about 10% in the low sensitive cell line by the addition of trastuzumab (3 µg/mL) as compared to without addition of trastuzumab. In contrast, with knockdown of ROCK2 by ROCK2 siRNA, the growth was suppressed by about 50% in the low sensitive cell line, and the growth was completely suppressed in the high sensitive cell line. Therefrom it was revealed that inhibition of ROCK2 enhances the sensitivity to the HER2 inhibitor.

### Example 7

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. Respective BT-474 cells were seeded in a 96 well plate (Nunc) at 3000 cells/150 µL/well and cultured 2-overnights, and trastuzumab (Rosh) or compound A were added by 50 µL/well to respective final concentrations. After culture for 5 days at 37°C, 5% CO₂, the number of viable cells was counted.

The growth was suppressed by about 50% in the high sensitive cell line and about 10% in the low sensitive cell line by the addition of trastuzumab (3 µg/mL) as compared to without addition of trastuzumab. In contrast, by the addition of compound A, a similar level of concentration-dependent growth suppression was observed in both the low sensitive cell line and the high sensitive cell line, and by the addition of compound A (1 µmol/L), complete suppression of growth was observed in both the low sensitive cell line and the high sensitive cell line.

### Example 8

Used were BT474 cells (American Type Culture Collection, HTB-20, J Natl Cancer Inst 61: 967-978 (1978)), which are highly sensitive to trastuzumab, and trastuzumab low sensitive BT-474 cells which were obtained by culturing the BT474 cells highly sensitive to trastuzumab in RPMI complete medium containing 5 µg/mL trastuzumab for 3 months or longer. Respective BT-474 cells were seeded in a 96 well plate (Nunc) at 3000 cells/150 µL/well and cultured 2-overnights, and trastuzumab (Rosh) or lapatinib were added by 50 µL/well to respective final concentrations. After culture for 5 days at 37°C, 5% CO₂, the number of viable cells was counted.

The growth was suppressed by about 50% in the high sensitive cell line and about 10% in the low sensitive cell line by the addition of trastuzumab (3 µg/mL) as compared to without addition of trastuzumab. In contrast, by the addition of lapatinib, a similar level of concentration-dependent growth suppression was observed in both the low sensitive cell line and the high sensitive cell line, and by the addition of lapatinib (1 µmol/L), complete suppression of growth was observed in both the low sensitive cell line and the high sensitive cell line.

### Industrial Applicability

The agent for preventing or treating cancer of the present invention, which comprises one or more medicaments selected from a cofilin inhibitor, a PAK1 inhibitor, a LIMK inhibitor, a Rho inhibitor, a ROCK1 inhibitor and a ROCK2 inhibitor, can be used effectively for the prophylaxis or treatment of cancer not only by a single use but also by combining with a conventional HER2 inhibitor.

This application is based on a patent application No. 2007-161769 filed in Japan, the contents of which are incorporated in full herein by this reference.

## Claims

1. An agent for preventing or treating a trastuzumab-resistant cancer, comprising one or more medicaments selected from a cofilin inhibitor, a PAK1 inhibitor, a LIMK inhibitor, a RHO inhibitor, a ROCK1 inhibitor and a ROCK2 inhibitor.

2. The agent according to claim 1, comprising a cofilin inhibitor.

3. The agent according to claim 1, comprising a PAK1 inhibitor.

4. The agent according to claim 1, comprising a LIMK inhibitor.

5. The agent according to claim 1, comprising a RHO inhibitor.

6. The agent according to claim 1, comprising a ROCK1 inhibitor.

7. The agent according to claim 1, comprising a ROCK2 inhibitor.

8. A combination drug comprising (1) a HER2 inhibitor and (2) one or more medicaments selected from a cofilin inhibitor, a PAK1 inhibitor, a LIMK inhibitor, a RHO inhibitor, a ROCK1 inhibitor and a ROCK2 inhibitor.

9. The drug according to claim 8, wherein the HER2 inhibitor is trastuzumab or lapatinib.

10. The drug according to claim 8, wherein the HER2 inhibitor is a compound represented by the formula 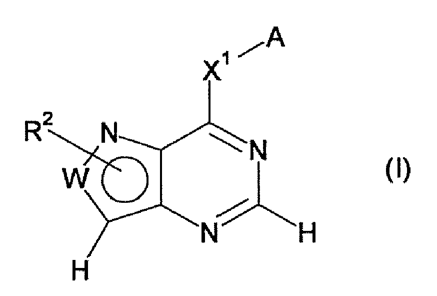 wherein W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or hetero atom on an aryl group or heteroaryl group for A to form an optionally substituted ring structure,
Y¹ is a single bond or C₁₋₄ alkylene or -O-(C₁₋₄ alkylene)-, each of which is optionally substituted,
R¹ is a hydrogen atom, or an optionally substituted group bonded via a carbon atom, a nitrogen atom or an oxygen atom, and R² is a hydrogen atom, or an optionally substituted group bonded via a carbon atom or a sulfur atom, or
R¹ and R², or R² and R³ are optionally bonded to each other to form an optionally substituted ring structure, except a compound represented by the formula 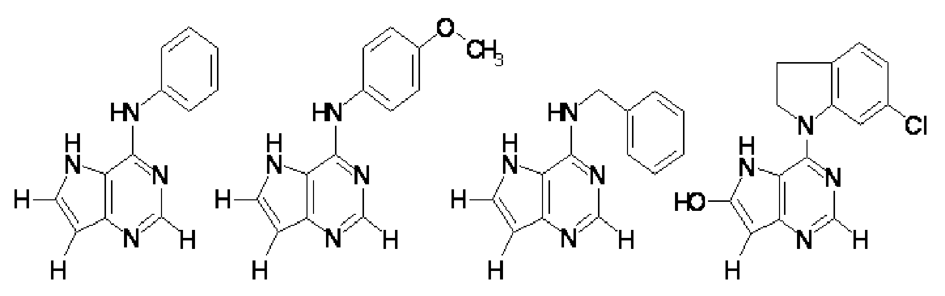 or a salt thereof.

11. The drug according to claim 8, wherein the HER2 inhibitor is a compound represented by the formula 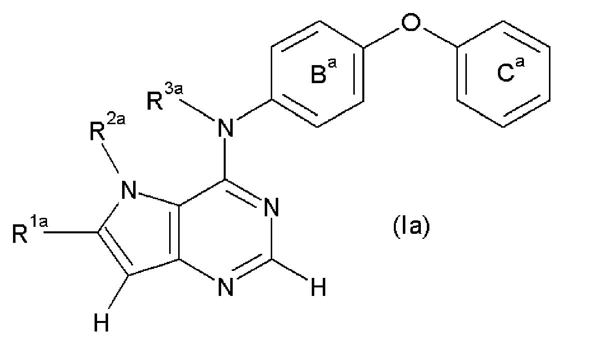 wherein
R^{1a} is a hydrogen atom, or an optionally substituted group bonded via a carbon atom, a nitrogen atom or an oxygen atom, R^{2a} is an optionally substituted group bonded via a carbon atom or a sulfur atom, or
R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or R^{3a} is optionally bonded via a carbon atom of the adjacent phenyl group to form an optionally substituted ring structure,
B^{a} is an optionally substituted benzene ring, and C^{a} is an optionally substituted C₆₋₁₈ aryl group, or a salt thereof.

12. The drug according to claim 8, wherein the HER2 inhibitor is a compound represented by the formula 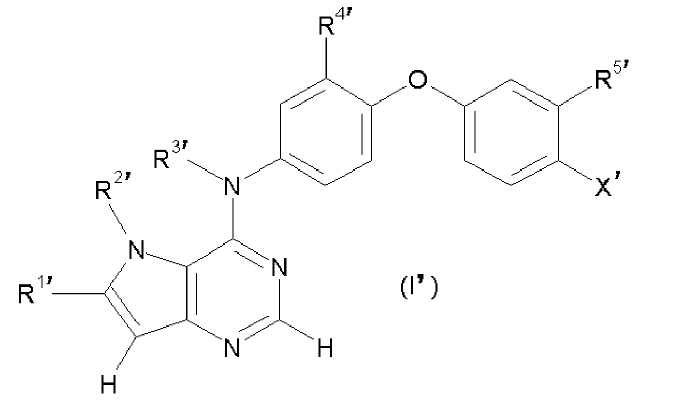 wherein
R^{1'} is a hydrogen atom,
R^{2'} is a C₁₋₆ alkyl group substituted by a group represented by -NR^{6'}-CO-(CH₂)ₙ-SO₂- (optionally halogenated C₁₋₄ alkyl) wherein n is an integer of 1 to 4, R^{6'} is a hydrogen atom or a C₁₋₄ alkyl group, wherein -(CH₂)ₙ- is optionally substituted by C₁₋₄ alkyl, R^{3'} is a hydrogen atom or a C₁₋₆ alkyl group,
R^{4'} is a halogen atom or a C₁₋₆ alkyl group,
R^{5'} is a halogen atom or a C₁₋₆ alkyl group, and
X' is a hydrogen atom or a halogen atom, except N-[2-(4-{[3-chloro-4-(3-chlorophenoxy)phenyl]amino}-5H-pyrrolo[3,2-d]pyrimidin-5-yl)ethyl]-2-(methylsulfonyl)acetamide, or a salt thereof.

13. The drug according to claim 8, wherein the HER2 inhibitor is a compound represented by the formula 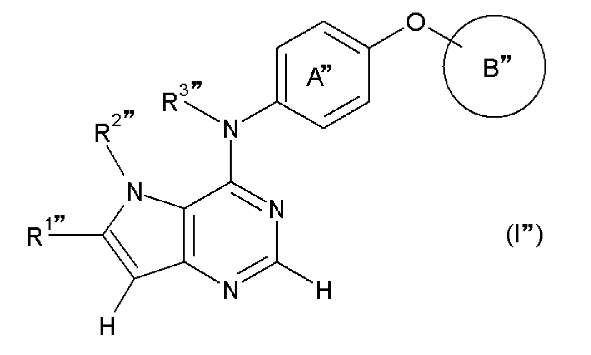 wherein
R^{1"} is a hydrogen atom, a halogen atom, or an optionally substituted group bonded via a carbon atom, a nitrogen atom or an oxygen atom,
R^{2"} is a hydrogen atom, or an optionally substituted group bonded via a carbon atom or a sulfur atom,
or R^{1"} and R^{2"}, or R^{2"} and R^{3"} are optionally bonded to each other to form an optionally substituted ring structure;
R^{3"} is a hydrogen atom or an optionally substituted aliphatic hydrocarbon group, or
R^{3"} is optionally bonded to a carbon atom of ring A" to form an optionally substituted ring structure;
ring A" is an optionally substituted benzene ring;
ring B" is
(i) an optionally substituted fused ring, or
(ii) a pyridine ring having optionally substituted carbamoyl wherein the pyridine ring is optionally further substituted, or a salt thereof.

14. The drug according to claim 8, wherein the HER2 inhibitor is N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof.

15. The drug according to claim 8, which is an agent for preventing or treating HER2-expressing cancer.

16. A method of preventing or treating trastuzumab-resistant cancer, which comprises inhibiting one or more selected from cofilin, PAK1, LIMK, RHO, ROCK1 and ROCK2.

17. The method according to claim 16, which comprises administering an effective amount of one or more medicaments selected from a cofilin inhibitor, a PAK1 inhibitor, a LIMK inhibitor, a RHO inhibitor, a ROCK1 inhibitor and a ROCK2 inhibitor to a mammal.

18. Use of one or more medicaments selected from a cofilin inhibitor, a PAK1 inhibitor, a LIMK inhibitor, a RHO inhibitor, a ROCK1 inhibitor and a ROCK2 inhibitor for the production of an agent for preventing or treating a trastuzumab-resistant cancer.

19. A method of examining the sensitivity of a HER2-expressing cancer to a HER2 inhibitor, comprising measuring the expression or activation state of one or more selected from cofilin, PAK1, LIMK, RHO, ROCK1 and ROCK2 in a sample collected from an animal having the cancer.

20. A method of treating a cancer in an animal judged to be low sensitive to a HER2 inhibitor by the method according to claim 19, which comprises administering an effective amount of each of (1) a HER2 inhibitor, and (2) one or more medicaments selected from a cofilin inhibitor, a PAK1 inhibitor, a LIMK inhibitor, a RHO inhibitor, a ROCK1 inhibitor and a ROCK2 inhibitor to the animal.

21. A method of screening for a drug for the prophylaxis or treatment of a trastuzumab-resistant cancer, comprising measuring and comparing the expression, activation state or activity of one or more selected from cofilin, PAK1, LIMK, RHO, ROCK1 and ROCK2 in a cell in the presence or absence of a test compound.

22. An agent for preventing or treating a trastuzumab-resistant cancer, comprising N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof.

23. A method of treating a trastuzumab-resistance cancer in a mammal, comprising administering an effective amount of N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide or a salt thereof to the animal.
